# EUROPEAN PATENT SPECIFICATION

(11) **EP 0 287 982 B1**
(45) Date of publication and mention of the grant of the patent: **07.12.1994**
(21) Application number: 88106121.2
(22) Date of filing: 18.04.1988
(51) Int. Cl.: C07D 401/12, C07D 401/14, C07D 405/14, C07D 409/14, A61K 31/44

(54) **N-(Pyridinyl)-1H-indol-1-amines, a process for their preparation and their use as medicaments**
N-(Pyridinyl)-1H-indol-1-amine, Verfahren zu deren Herstellung und ihre Verwendung als Arzneimittel
N-(pyridinyl)-1H-indol-1-amines, leur procédé de préparation et leur utilisation comme médicaments

(30) Priority: 24.04.1987 US 42079
(43) Date of publication of application: 26.10.1988
(73) Proprietor: HOECHST-ROUSSEL PHARMACEUTICALS INCORPORATED, Somerville New Jersey 08876 (US)
(72) Inventor: Effland, Richard Charles, Bridgewater, N.J. 08807 (US); Klein, Joseph Thomas, Bridgewater, N.J. 08807 (US)
(74) Representative: Losert, Wolfgang Dr.

(56) References cited:
- EP-A- 0 226 099
- DE-A- 1 670 184
- DE-A- 2 512 702
- US-A- 3 242 185
- US-A- 4 260 767
- CHEMICAL ABSTRACTS vo. 104, no. 5, 3 February1986, page 541, column 2, abstract no. 34004k, Columbus, Ohio, US; & JP- A - 60142955 (FUJI PHOTO FILM CO LTD) 29.07.1985

## Description

The present invention relates to novel compounds of the formula 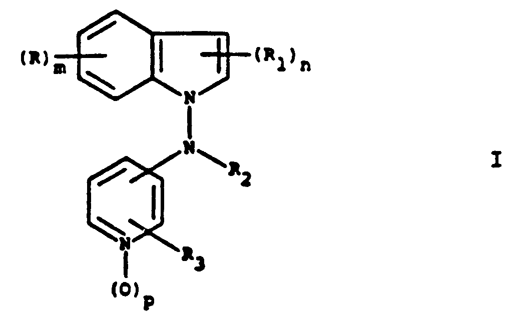
where m is 1 or 2; n is 1 or 2; P is 0̸ or 1; each R is independently hydrogen, halogen, C₁-C₆-alkyl, C₁-C₆-alkoxy, hydroxy, nitro, amino, C₁-C₆-alkylamino, C₁-C₆-alkylcarbonylamino, cyano, formyl, C₁-C₆-alkoxycarbonyl, C₁-C₆-alkylthio or C₁-C₆alkoxycarbonyl-C₁-C₆-alkylthio; each R₁ is independently hydrogen, C₁-C₆-alkyl, C₂-C₆-alkenyl, formyl, C₁-C₆-alkylcarbonyl, phenyl-C₁-C₆-alkylcarbonyl, phenylcarbonyl, halogen, phenyl-C₂-C₆-alkenyl, phenyl-C₁-C₆-alkyl, heteroaryl-C₂-C₆-alkenyl, heteroaryl-C₁-C₆-alkyl, cyano-C₂-C₆-alkenyl, cyano-C₁-C₆-alkyl, methoxy-C₂-C₆-alkenyl, methoxy-C₁-C₆-alkyl, C₁-C₆-alkoxycarbonyl-C₂-C₆-alkenyl, C₁-C₆-alkoxycarbonyl-C₁-C₆-alkyl, cycloalkyl-C₂-C₆-alkenyl or C₃-C₇-cycloalkyl-C₁-C₆-alkyl, the term phenyl signifying a phenyl group which may have 1-3 substituents each of which being independently C₁-C₆-alkyl, C₁-C₆-alkoxy, halogen, CF₃, NO₂ or CN, and the term heteroaryl signifying a group of the formula 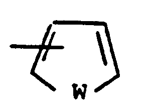
where W is O, S, NH or CH=N, cyano, -CH(OH)R₄, -C(OH)R₄R₅ or -CH₂OR₅, R₄ being hydrogen, C₁-C₆-alkyl, phenyl-C₁-C₆-alkyl or phenyl and R₅ being C₁-C₆-alkyl, phenyl-C₁-C₆-alkyl or phenyl; each R₂ is independently hydrogen, C₁-C₆-alkyl, C₂-C₆-alkenyl, C₂-C₆-alkynyl, C₁-C₆-alkoxycarbonyl-C₁-C₆-alkyl, C₁-C₆-alkylaminocarbonyl-C₁-C₆-alkyl, aminocarbonyl-C₁-C₆-alkyl, phenyl-C₁-C₆-alkyl, C₁-C₆-alkoxycarbonyl, phenyl-C₁-C₆-alkoxycarbonyl, phenyloxycarbonyl, C₁-C₆-alkylaminocarbonyl, phenyl-C₁-C₆-alkylaminocarbonyl, phenylaminocarbonyl, C₁-C₂₀-alkanoyl, phenyl-C₁-C₆-alkanoyl, phenylcarbonyl, C₂-C₂₀-alkenoyl, C₂-C₂₀-alkynoyl or -R₆-NR'R'' where R₆ is C₁-C₆-alkylene, C₂-C₆-alkenylene or C₂-C₆-alkynylene and R' and R'' are each independently C₁-C₆-alkyl or alternatively the group -NR'R'' as a whole is 1-pyrrolidinyl, 1-piperidinyl, 4-morpholinyl, 4-C₁-C₆-alkyl-1-piperazinyl or 4-phenyl-1-piperzinyl, the term phenyl signifying a phenyl group which may be substituted as indicated above, or each R₂ is independently phenyl, nitrophenyl, cyanophenyl, trifluoromethyl, aminophenyl or C₁-C₆-alkanoylaminophenyl, wherein the phenyl group is unsubstituted,and R₃ is hydrogen, nitro, amino halogen, C₁-C₆-alkanoylamino, phenyl-C₁-C₆-alkanoylamino, phenylcarbonylamino, alkylamino, phenyl-C₁-C₆-alkylamino or C₁-C₆-alkyl, the term phenyl signifying a phenyl group which may be substituted as indicated above; which compounds are useful for enhancing memory and also as analgesic and antidepressant agents; and pharmaceutical compositions comprising an effective amount of such a compound.

DE-A- 25 12 702 discloses substituted 1-amino-3-phenyl-indoles having antidepressant activity, in which the amino group is substituted by aliphatic or aromatic amines, but not by pyridine derivatives.

Throughout the specification and the appended claims, a given chemical formula or name shall encompass all stereo, optical, and geometrical isomers thereof where such isomers exist, as well as pharmaceutically acceptable acid addition salts thereof and solvates thereof such as for instance hydrates.

The following general rules of terminology shall apply throughout the specification and the appended claims.

Unless otherwise stated or indicated, the term C₁-C₆-alkyl denotes a straight or branched alkyl group having from 1 to 6 carbon atoms. Examples of said C₁-C₆-alkyl include methyl, ethyl, n-propyl, iso-propyl, n-butyl, iso-butyl, sec-butyl, t-butyl and straight- and branched-chain pentyl and hexyl.

Unless otherwise stated or indicated, the term C₁-C₆-alkoxy denotes a straight or branched alkoxy group having from 1 to 6 carbon atoms. Examples of said loweralkoxy include methoxy, ethoxy, n-propoxy, iso-propoxy, n-butoxy, iso-butoxy, sec-butoxy, t-butoxy and straight- and branched-chain pentoxy and hexoxy.

Unless otherwise stated or indicated, the term halogen shall mean fluorine, chlorine, bromine or iodine.

Unless otherwise stated or indicated, the term alkyl shall mean a saturated hydrocarbon group of 1 to 20̸ carbon atoms, the term alkenyl shall mean a hydrocarbon group of 2-20̸ carbon atoms having one or more carbon-carbon double bonds, and the term alkynyl shall mean a hydrocarbon group of 2-20̸ carbon atoms having one or more carbon-carbon triple bonds.

The term C₁-C₆-alkanoic acid shall mean a carboxylic acid in which the carboxyl group is attached to hydrogen or an alkyl group of from 1 to 5 carbon atoms.

The term C₁-C₆-alkanoyl shall mean a group obtained by removing a hydroxy group from the carboxyl group of a C₁-C₆-alkanoic acid, and thus it includes for instance formyl, and acetyl.

The terms alkanoyl, alkenoyl and alkynoyl shall mean groups obtained by removing a hydroxy group from the carboxyl group of alkanoic acid, alkenoic acid and alkynoic acid, respectively. Thus, for instance, linoleyl group derived from linoleic acid is an example of the term alkenoyl as defined above.

The term acyl shall mean C₁-C₆-alkanoyl or phenyl-C₁-C₆-alkanoyl wherein the phenyl may be substituted as defined above.

The compounds of formula (I) of this invention can be synthesized by following or combining one or more of the steps described below, not necessarily in the order presented. For the sake of simplification, the description of synthetic schemes is presented below for compounds in which m=n=1, but it will be apparent that other compounds in which m and/or n is 2 can be prepared by utilizing the synthetic schemes and making necessary modifications. Throughout the description of the synthetic steps, the definitions of R, R₁ through R₆, R', R'', m, n and p are as given above unless otherwise stated or indicated, and other nomenclatures appearing below shall have the same meanings defined in their respective first appearances unless otherwise stated or indicated.

### STEP A

A compound of formula (II) where R₇ is H, halogen, C₁-C₆-alkyl, C₁-C₆-alkoxy, nitro, cyano, formyl, C₁-C₆-alkylthio or C₁-C₆-alkoxycarbonyl-C₁-C₆-alkylthio and R₈ is H, C₁-C₆-alkyl, halogen or cyano and R₉ is H or C₁-C₆-alkyl is reacted with a compound of formula (III) where X is chlorine or fluorine and R₁₀ is H, NO₂, halogen or C₁-C₆-alkyl to afford a compound of formula (IV). 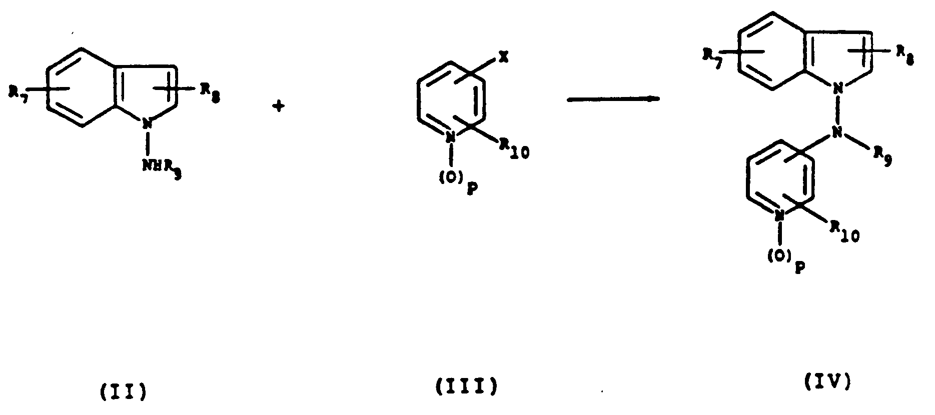
Said reaction is typically conducted in an ethereal solvent such as bis(2-methoxyethyl)ether, diethyl ether, dimethoxy ether, dioxane or tetrahydrofuran or polar aprotic solvent such as dimethylformamide, dimethylacetamide, hexamethylphosphoramide or dimethylsulfoxide or protic solvent such as ethanol or isopropanol at a temperature of between 20̸°C and 150̸°C.

### STEP B

A compound of formula IVa obtained from STEP A is treated with a strong base such as sodium hydride or potassium hydride in a suitable solvent such as polar aprotic solvent including dimethylformamide, dimethylsulfoxide and ethereal solvents or aromatic hydrocarbon at a temperature of between -10̸° and 50̸°, preferably 0̸-25° to form the anion of IVa, which is reacted with a chloride compound of the formula R₁₁-Cl, where R₁₁ is C₁-C₆-alkyl, C₁-C₆-alkoxycarbonyl-C₁-C₆-alkyl, C₂-C₆-alkenyl, C₂-C₆-alkynyl, C₁-C₆-alkoxycarbonyl, phenyl-C₁-C₆-alkyl, phenyl-C₁-C₆-alkoxycarbonyl or phenyloxycarbonyl wherein the phenyl may be substituted as indicated above, at a temperature of between -10°C and 80°C, preferably between 0°C and 25° to obtain a compound of formula V.

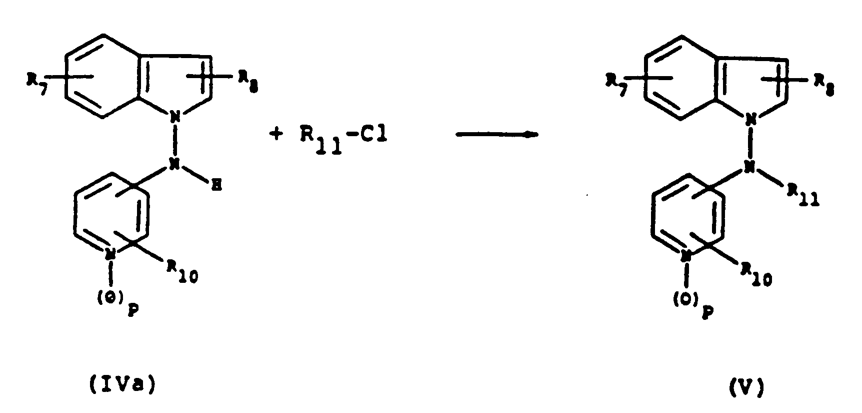

### STEP C

The anion of compound IVa prepared as in STEP B is reacted with fluoro-nitrobenzene, cyano-fluorobenzene or fluoro-trifluoromethylbenzene of the formula 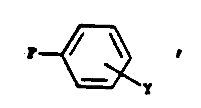
where Y is nitro, cyano or trifluoromethyl to afford a compound of formula VI below. Said reaction is conducted in substantially the same manner as in STEP B.

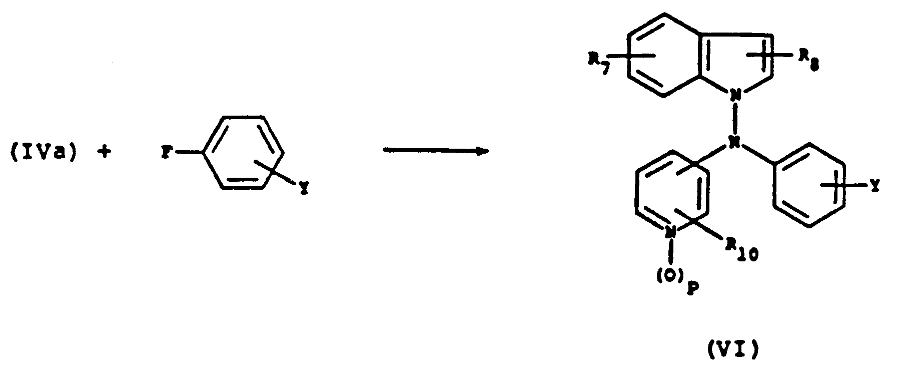

### STEP D

Compound IVa is reacted with a C₁-C₆-alkyl isocyanate, phenyl-C₁-C₆-alkyl isocyanate or phenyl isocyanate of the formula R₁₂NCO where R₁₂is C₁-C₆-alkyl, phenyl-C₁-C₆-alkyl or phenyl wherein the phenyl may be substituted as indicated above, to afford a compound of formula VII. 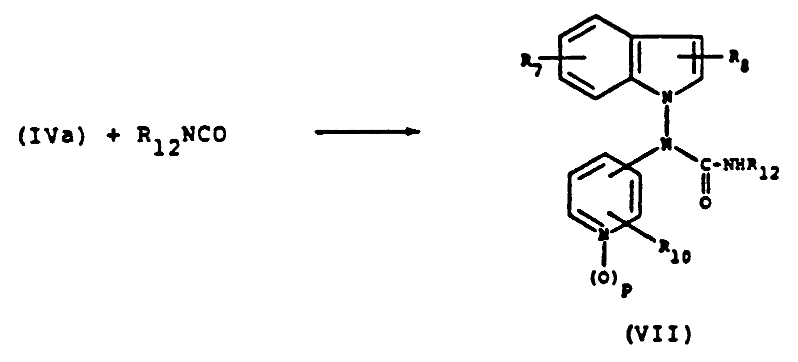
Said reaction is typically conducted in a suitable solvent such as aromatic hydrocarbon including benzene, toluene and the like, halogenated hydrocarbon or ethereal solvent at a temperature of 0̸-80̸°, preferably 30̸-60̸°C.

### STEP E

Compound IVa is reacted with an alkanonyl chloride, phenyl-C₁-C₆-alkanoyl chloride, phenylcarbonyl chloride, alkenoyl chloride or alkynoyl chloride of formula (VIII) where R₁₃ is alkyl, alkenyl, alkynyl, phenyl-C₁-C₆-alkyl or phenyl wherein the phenyl may be substituted as indicated above, to afford a compound of formula (IX). Said reaction is typically conducted in substantially the same manner as in STEP D. 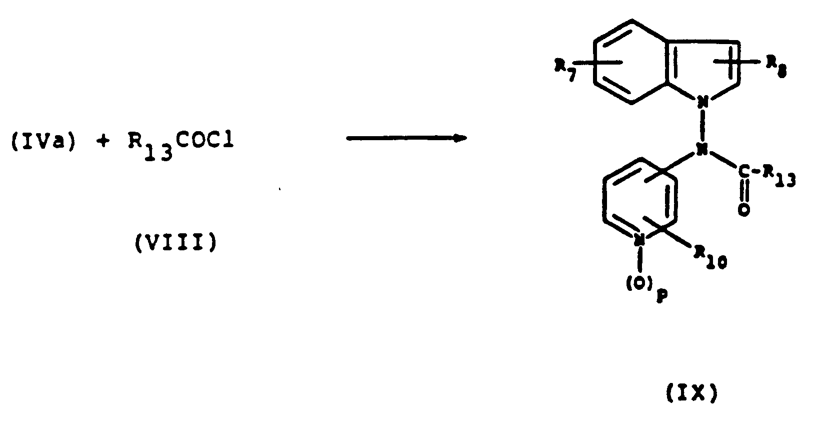
Where the compound R₁₃COCl is not commercially available, it is prepared from the corresponding carboxylic acid R₁₃COOH and thionyl chloride in a suitable solvent, for instance, in benzene at the reflux temperature.

### STEP F

As an alternative to STEP A or B, a compound of formula (IVb) where R₁₄ is C₁-C₆-alkyl can be prepared by reacting compound IVa with a strong base such as sodium hydride or potassium hydride and then reacting the product with a di-C₁-C₆-alkyl sulfate of the formula (R₁₄)₂SO₄. Said two steps are conducted under substantially the same conditions as used in STEP B.

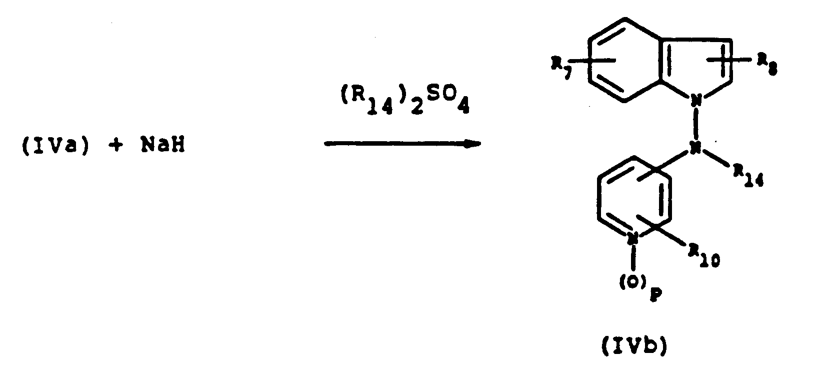

### STEP G

A compound of formula Va obtained in STEP B is subjected to Mannich reaction with formaldehyde and a secondary amine of the formula HNR'R'', where R' and R'' are independently C₁-C₆-alkyl or -NR'R'' taken together is 1-pyrrolidinyl, 1-piperidinyl, 4-morpholinyl, 4-C₁-C₆-alkyl-1-piperazinyl or 4-phenyl-1-piperazinyl wherein the phenyl may be substituted as indicated above, to afford a compound of formula X. 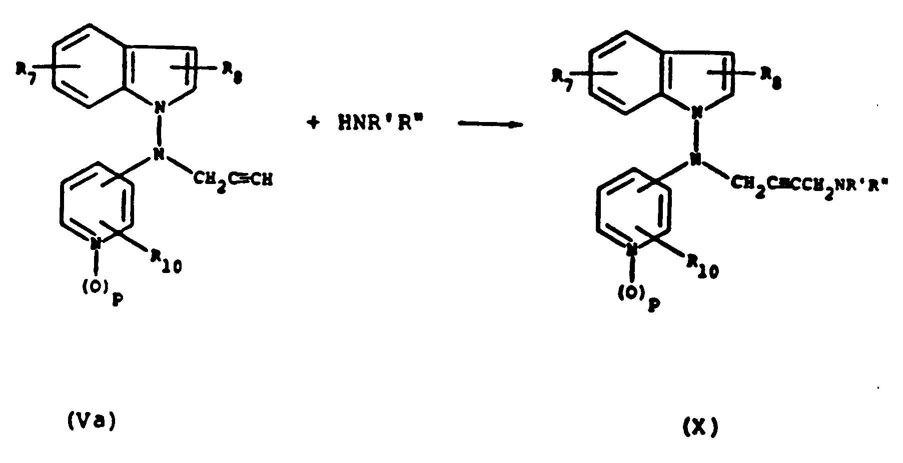
The above reaction can be conducted under conditions usually used in the art for carrying out Mannich reactions. Typically, it is conducted by preparing a mixture of compound Va, paraformaldehyde, HNR'R'', cuprous chloride (used as a catalyst) and a suitable medium including ethereal solvents such as dioxane, and heating the mixture at 25-10̸0̸°.

### STEP H

Compound X is catalytically hydrogenated to afford a compound of formula XI or XII by making a suitable selection of reaction conditions in a manner known to the art.

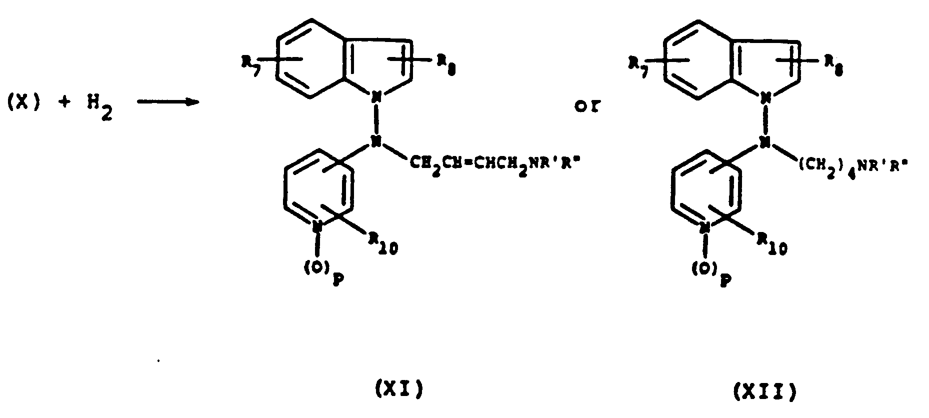

### STEP I

As an alternative to the foregoing steps, a compound of formula XIV where R₁₅ is C₁-C₆-alkyl, C₁-C₆-alkoxycarbonyl-C₁-C₆-alkyl, alkanoyl, C₁-C₆-alkoxycarbonyl, phenyl-C₁-C₆-alkoxycarbonyl, phenyl-C₁-C₆-alkyl, phenyl, phenyloxycarbonyl, phenyl-C₁-C₆-alkanoyl or phenylcarbonyl wherein the phenyl may be substituted as indicated above, can be prepared by reacting a compound of formula XIII with a suitable chlorinating agent such as sulfuryl chloride (SO₂Cl₂) in a manner known in the art.

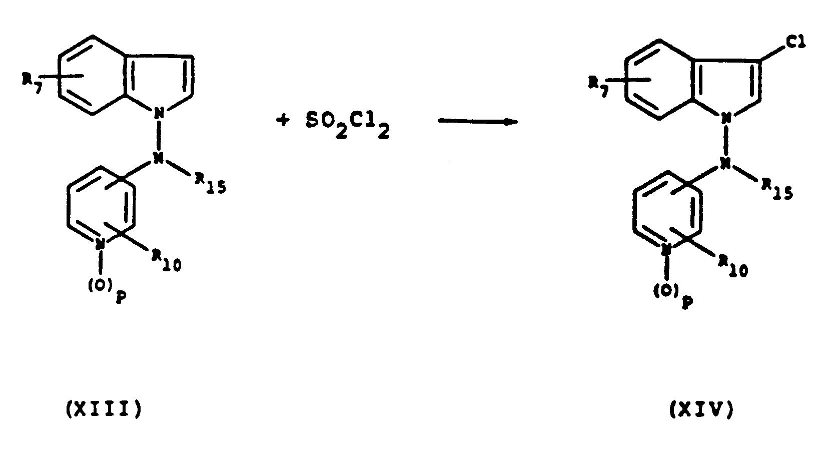

### STEP J

A compound of formula (XV) where R₁₆ is C₁-C₆-alkyl, C₁-C₆-alkoxycarbonyl, alkanoyl, alkenoyl, alkynoyl, phenyl-C₁-C₆-alkanoyl or phenylcarbonyl wherein the phenyl may be substituted as indicated above, and R₁₇ is H, NO₂, halogen or C₁-C₆-alkyl which is prepared by use of one or more of the reaction steps described in this specification, is reacted with phosphorus oxychloride and dimethylformamide to afford a compound of formula (XVI). 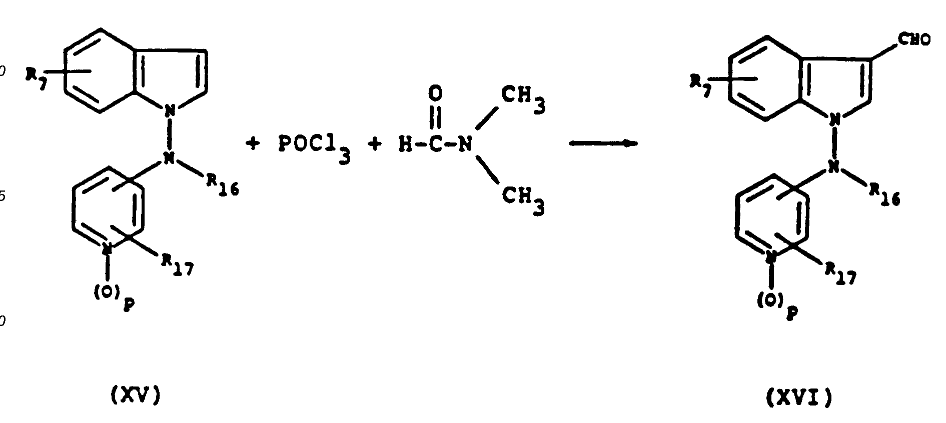
Said reaction can be conducted under conditions usually used for carrying out Vilsmeier reactions. Typically, it is conducted in a suitable solvent such as halogenated hydrocarbon at a temperature of 20̸-10̸0̸°.

Where the positional isomer of compound XVI in which the formyl group is at the 2-position of the indole ring is desired, compound XV is reacted with secondary butyllithium and the resultant lithio compound reacted with N-formyl-N-methyl-aniline in a manner known in the art.

### STEP K

Compound XV is reacted with a C₁-C₆-alkanoyl chloride, phenyl-C₁-C₆-alkanoyl chloride or phenylcarbonyl chloride wherein the phenyl may be substituted as indicated above, of formula R₁₂COCl in the presence of zinc chloride to afford a compound of formula (XVII). Said reaction is typically conducted in a suitable solvent such as halogenated hydrocarbon at a temperature of 20̸-10̸0̸°C. 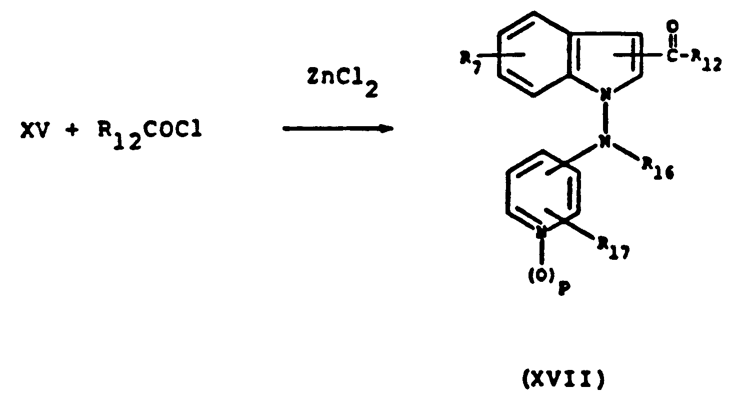
Where the positional isomer of compound XVII in which the group -C(O)R₁₂ is at the 2-position of the indole ring is desired, compound XV is reacted with secondary butyllithium and the resultant lithio compound reacted with R₁₂COCl in a manner known to the art.

### STEP L

A compound of formula XVIII below where R₁₈ is H, halogen or C₁-C₆-alkyl is reduced to a compound of formula XIX below with NaBH₄, LiAlH₄ or borane complexes. 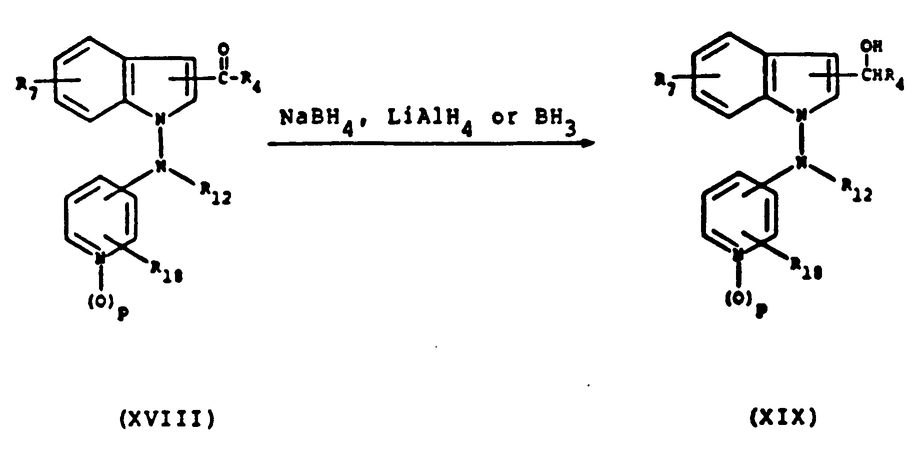
When NaBH₄ is used, said reduction is conducted typically in a C₁-C₆-aliphatic alcohol such as isopropanol or ethanol or C₁-C₆-alkanoic acid at a temperature of 0̸-80̸°. After the reaction, water is added to the reaction mixture. When LiAlH₄ is used, said reduction is conducted typically in an ethereal solvent such as tetrahydrofuran or ether at a temperature of 0̸-80̸°. When borane complexes are used, the reaction temperature is typically 0̸-80̸°C.

### STEP M

Compound XVIII is reacted with a Grignard reagent of the formula R₅MgBr and the product is thereafter hydrolyzed to afford a compound of formula XX below.

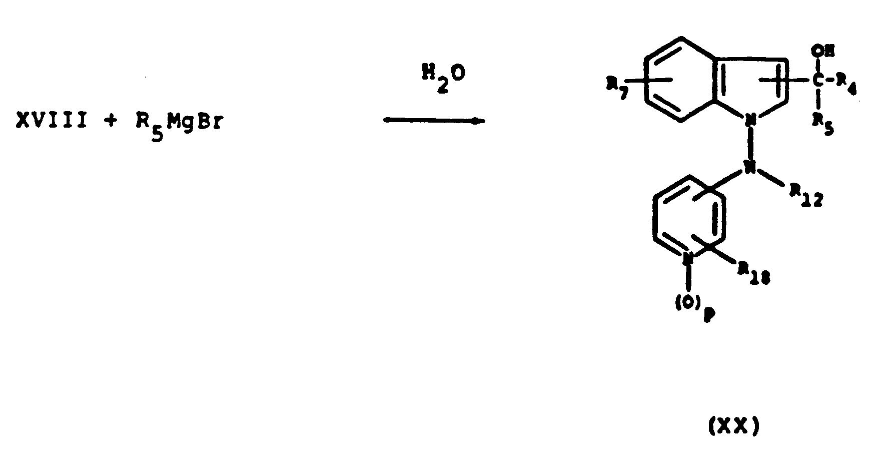

### STEP N

A compound of formula XXI which is prepared by use of one or more of the reaction steps described in this specification is catalytically hydrogenated with hydrogen gas and a suitable catalyst such as palladium on carbon to afford a compound of formula (XXII). 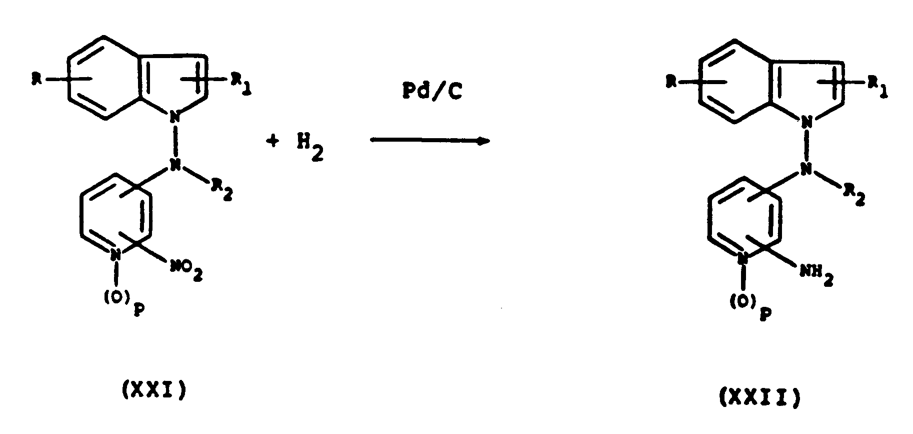
Said catalytic hydrogenation is typically conducted in a suitable solvent such as C₁-C₆-alkanol or C₁-C₆-alkyl ester or C₁-C₆-alkanoic acid at a temperature of 20-50°C.

### STEP O

As an alternative to the foregoing steps, compound IVa can be prepared by hydrolyzing a carbamate compound of formula Vb. (Needless to say, the purpose of this STEP is not to reverse aforementioned STEP B in order to regain the starting compound of STEP B. This STEP can be useful, for instance, for the purpose of converting R₈ in formula IVa from hydrogen to 3-chloro. Thus, for this purpose, one can first convert the amino hydrogen in formula IVa to ethoxycarbonyl by use of STEP B or similar to STEP E and then introduce a chlorine atom into the 3-position of the indole ring by use of STEP I and thereafter hydrolyze the resultant product by use of this STEP, instead of conducting the chlorination reaction directly with compound IVa. Similarly, this STEP can also be useful for introducing the group -COR₁₂ into the indole ring according to STEP K above or the group -CHO according to STEP J when R₂ is hydrogen. 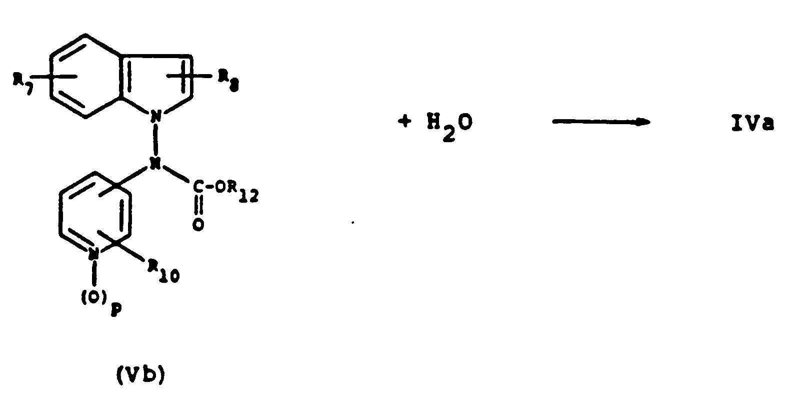
Said hydrolysis is conducted typically by stirring a mixture comprising compound Vb, an alkali such as NaOH and a suitable medium such as ethanol plus water at a temperature of 70̸-10̸0̸°C.

### STEP P

Compound XXII is reacted with phenyl formate to afford a compound of formula (XXIII) 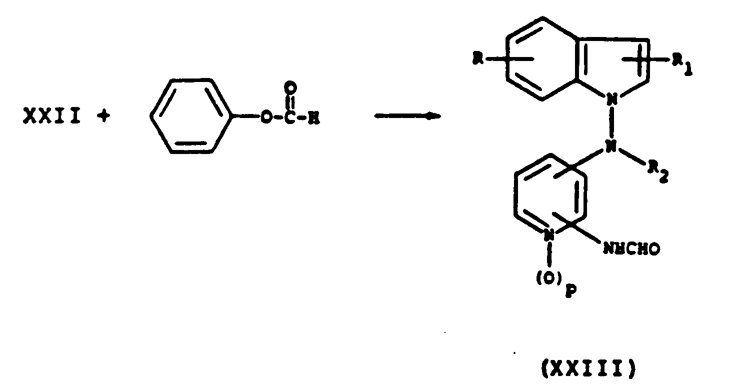
Typically said reaction is conducted by stirring a solution of compound XXII in excess phenyl formate at a temperature of 20̸-50̸°C. The same reaction can also be conducted with C₁-C₆-alkyl formate under substantially the same conditions.

### STEP Q

Compound XXII is reacted with an acyl chloride of the formula R₁₂COCl or acid anhydride of the formula R₁₂CO-O-COR₁₂ to afford a compound of formula XXIV. 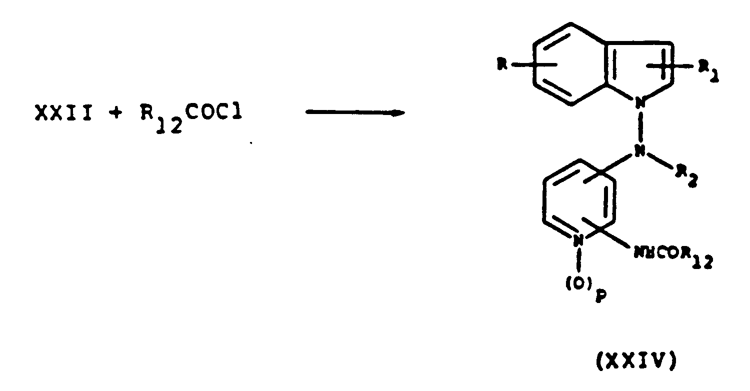
Said reaction is conducted under substantially the same conditions as used in STEP E.

### STEP R

As an alternative to the foregoing steps, a compound of formula XXV where R₁ is H or C₁-C₆-alkyl, and R₂ is C₁-C₆-alkyl, phenyl, nitrophenyl, or trifluoromethylphenyl, wherein the phenyl group is unsubstituted, or phenyl-C₁-C₆-alkyl, wherein the phenyl may be substituted as indicated above, can be prepared by reacting a compound of formula (IVc) with a C₁-C₆-alkyl lithium of the formula R₁₉Li where R₁₉ is C₁-C₆-alkyl. 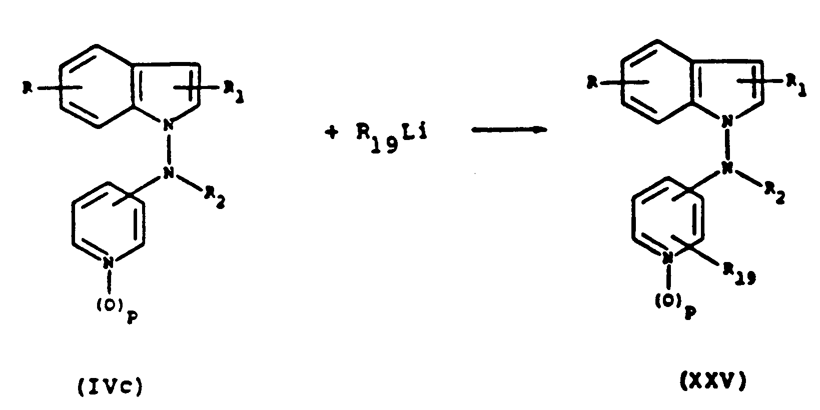
Said reaction is usually conducted in a suitable solvent such as ethereal solvent, preferably tetrahydrofuran at a temperature of between -10̸° and 50̸°C.

### STEP S

A compound of formula XIXa below is reacted with a strong base such as sodium hydride or potassium hydride and the resultant alkoxide anion is reacted with a halide of the formula R₅X to afford an ether of formula XXVI below. Said two-step procedure is conducted in substantially the same manner as in STEP B above.

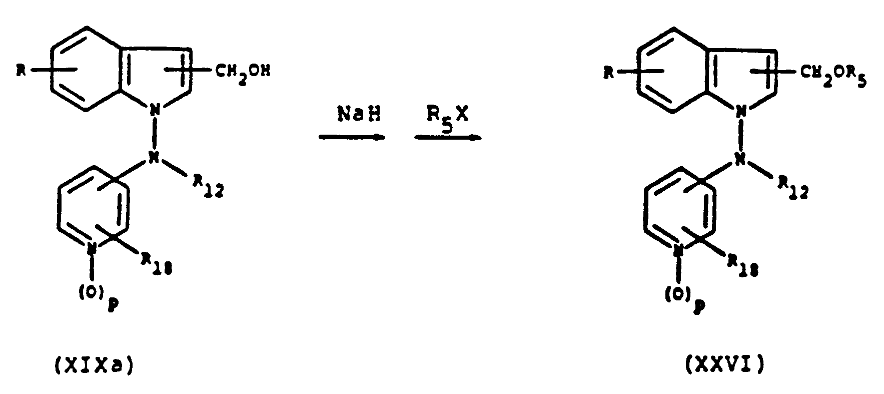

### STEP T

Compound XVI is subjected to Wittig reaction with an ylide of the formula (C₆H₅)₃P=CR_{20̸}R₂₁ to afford a compound of formula XXVII where R_{20̸} and R₂₁ are each independently hydrogen, C₁-C₆-alkyl, phenyl, phenyl-C₁-C₆-alkyl wherein the phenyl group may be substituted as indicated above, heteroaryl or heteroaryl-C₁-C₆-alkyl wherein the term heteroaryl has the meaning as indicated above, cyano, methoxy, C₁-C₆-alkoxycarbonyl or together form a cycloalkylidene. 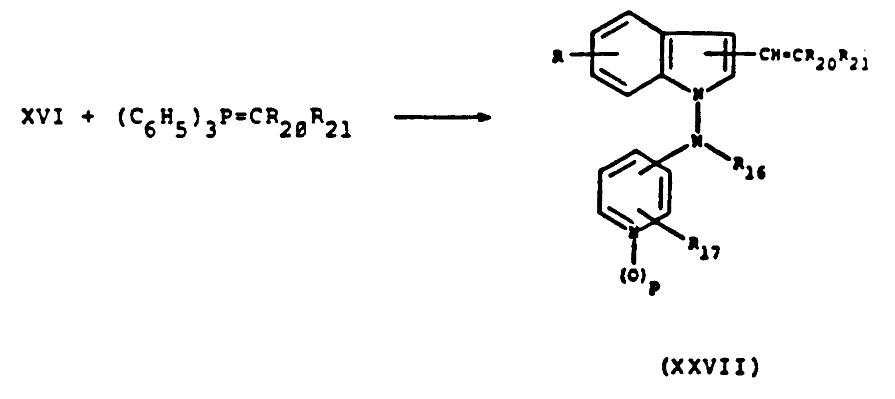
The above reaction can be conducted under conditions usually used for carrying out Wittig reactions. Thus, the ylide is prepared in a routine manner by first preparing a phosphonium salt from a bromide of the formula BrCHR_{20̸}R₂₁ and triphenylphosphine and thereafter reacting the phosphonium salt with a suitable base such as sodium hydride, potassium tert-butoxide or n-butyllithium in a suitable solvent such as anhydrous ethereal solvent. Thereafter a solution of compound XVI in a suitable solvent such as anhydrous ether is added to the freshly prepared ylide solution and the mixture is stirred at a temperature of between -10̸°C and 80̸°C.

It will be apparent that by making a suitable selection of the groups R_{20̸} and R₂₁ and/or conducting Wittig reaction more than once if necessary, one can obtain compounds of formula XXVIII where the group R₂₂ is C₂-C₆-alkenyl, phenyl-C₂-C₆-alkenyl wherein the phenyl may be substituted as indicated above, heteroaryl-C₂-C₆-alkenyl wherein the term heteroaryl has the meaning as indicated above, cyano-C₂-C₆-alkenyl, methoxy-C₂-C₆-alkenyl, C₁-C₆-alkoxycarbonyl-C₂-C₆-alkenyl, or C₃-C₇-cycloalkyl-C₂-C₆-alkenyl.

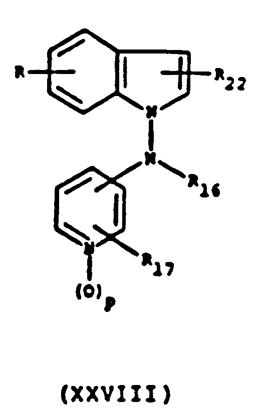

### Step U

Compound XXVIII is catalytically hydrogenated in a suitable manner known to the art afford a compound of formula XXIX where R₂₃ is C₁-C₆-alkyl, phenyl-C₁-C₆-alkyl wherein the phenyl group may be substituted as indicated above, heteroaryl-C₁-C₆-alkyl wherein the term heteroaryl has the meaning as indicated above, cyano-C₁-C₆-alkyl, methoxy-C₁-C₆-alkyl, C₁-C₆-alkoxycarbonyl-C₁-C₆-alkyl or C₃-C₇-cycloalkyl-C₁-C₆-alkyl.

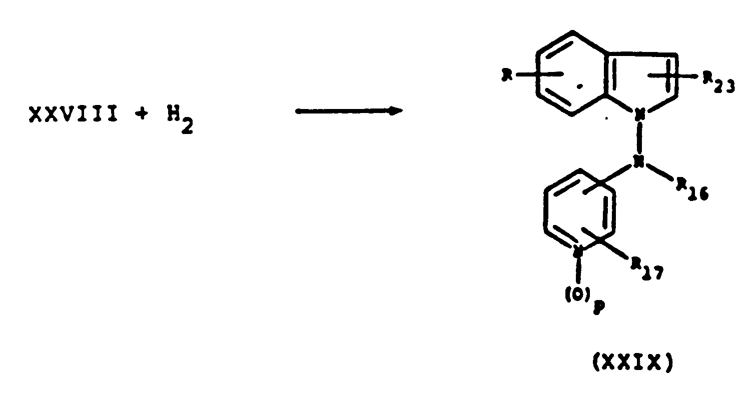

### STEP V

Substantially the same hydrogenation technique as described in STEP N or U can be used to hydrogenate a compound of formula XXX below to afford a compound of formula XXXI below.

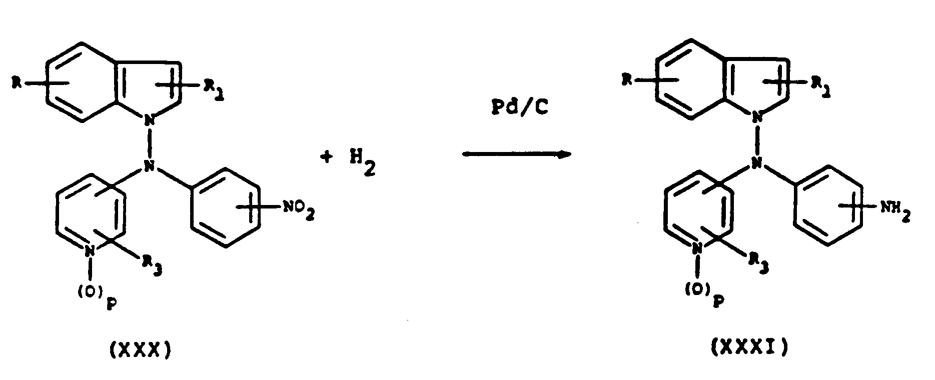

### STEP W

Substantially the same reaction technique as described in STEP P can be used to convert compound XXXI to a compound of formula XXXII below.

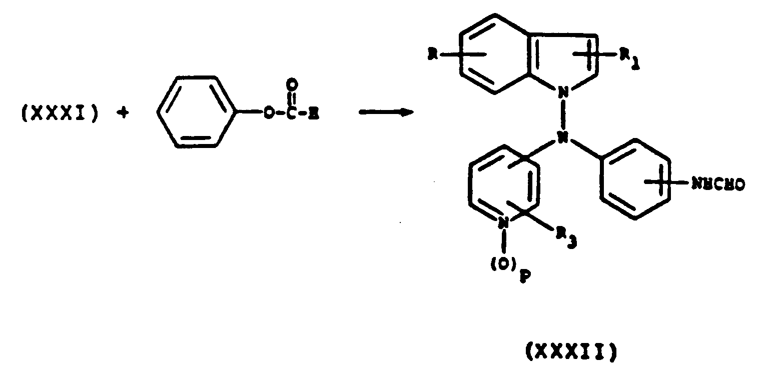

### STEP X

Substantially the same reaction technique as described in STEP Q can be used to convert compound XXXI to a compound of formula XXXIII below.

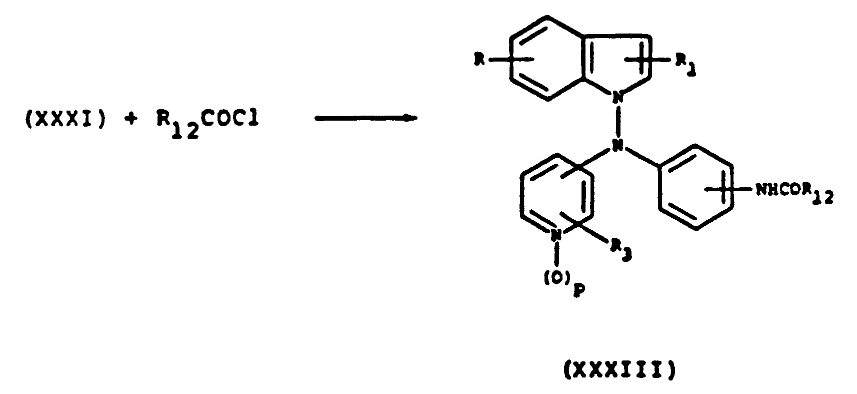

### STEP Y

As an alternative to the foregoing steps, introduction of a cyano group into the 2- or 3-position of the indole ring can be accomplished by using, as a starting compound, the formyl compound of formula XXXIV and converting the formyl group into a cyano group. For this purpose, compound XXXIV is first reacted with hydroxylamine in a routine manner to obtain the corresponding oxime and thereafter the oxime is reacted with benzenesulfonyl chloride to obtain a nitrile compound of formula XXXV. The second step is typically conducted in a suitable solvent such as ethereal solvent by warming the reaction mixture at 60̸-10̸0̸°C.

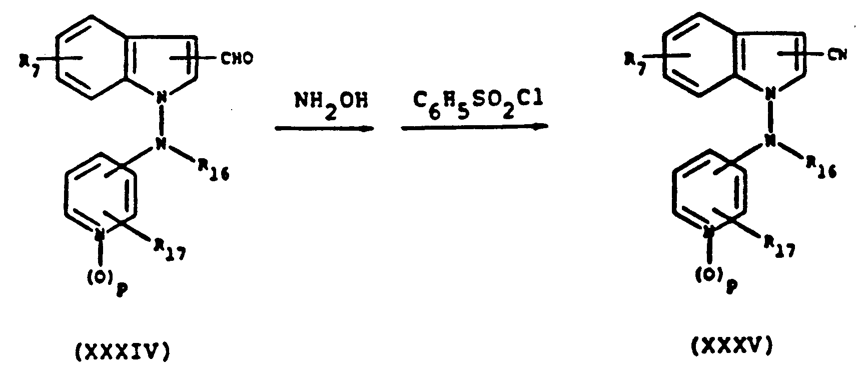

### Step Z

For the purpose of preparing a compound of formula I where R₂ is aminocarbonyl-C₁-C₆-alkyl, a compound of formula I where R₂ is C₁-C₆-alkoxycarbonyl-C₁-C₆-alkyl (preferably ethorycarbonyl-C₁-C₆-alkyl) is reacted with ammonia in a manner known to the art.

The compounds of formula I of the invention are useful in the treatment of various memory dysfunctions characterized by decreased cholinergic function, such as Alzheimer's disease.

This utility is demonstrated by the ability of these compounds to restore cholinergically deficient memory in the Dark Avoidance Assay, where they are generally active over a broader dose range than heretofore known compounds, a distinct therapeutic advantage.

### Dark Avoidance Assay

In this assay mice are tested for their ability to remember an unpleasant stimulus for a period of 24 hours. A mouse is placed in a chamber that contains a dark compartment; a strong incandescent light drives it to the dark compartment, where an electric shock is administered through metal plates on the floor. The animal is removed form the testing apparatus and tested again, 24 hours later, for the ability to remember the electric shock.

If scopolamine, an anticholinergic that is known to cause memory impairment, is administered before an animal's initial exposure to the test chamber, the animal re-enters the dark compartment shortly after being placed in the test chamber 24 hours later. This effect of scopolamine is countered by an active test compound, resulting in a greater interval before re-entry into the dark compartment.

The results for active compound are expressed as the percent of a group of animals in which the effect of scopolamine is countered, as manifested by an increased interval between being placed in the test chamber and re-entering the dark compartment.

The results of some of the compounds of this invention are presented in Table 1 along with the result of physostigmine.

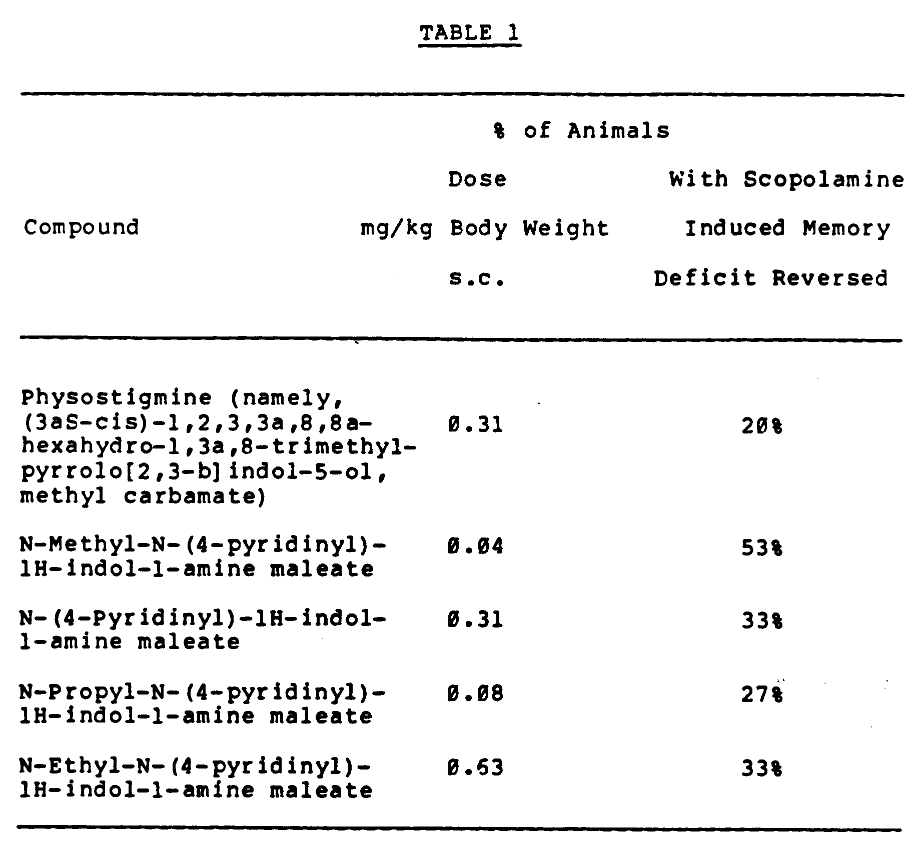

Additionally, some of the compounds of this invention exhibit antidepressant activities, which activities being particularly helpful for patients suffering from Alzheimer's disease. The antidepressant activities were evaluated in this invention on the basis of prevention of Tetrabenazine-induced ptosis in mice. The test method and results are described below.

### Prevention Of Tetrabenazine-Induced Ptosis In Mice

Tetrabenazine (TBZ) induces behavioral depression with concomitant ptosis in mice similar to reserpine. Antidepressant compounds, both monoamineoxidase inhibitors and tricyclics, are known to prevent or antagonize these effects and the degree of antagonism correlates with clinical efficacy. The prevention of TBZ-induced ptosis in mice is used as a preliminary screen for possible antidepressant activity. The method used in this invention is as follows:
Male mice weighing 20̸ to 30̸ grams are used in test groups of five subjects. All compounds are dissolved or suspended with a suitable surfactant in distilled water and administered in volumes of 10̸ ml/kg of body weight. TBZ solution is made from the methanesulfonate salt and the concentration is adjusted to enable administration of 60̸ mg/kg of base by intraperitoneal (i.p.) injection.

The pretreatment time is measured from the time of dosing to observation. Therefore, when a 30̸-minute pretreat is utilized, drug and TBZ are given simultaneously. A control group received solvent and TBZ at intervals indentical to drug group. For a primary screen, the drug is administered i.p. and a group size of five is utilized. Eight animals/group are used for a dose range.

Thirty minutes after TBZ, the subjects are placed in individual plastic containers (10̸.5 x 8 x 6 inches) in the presence of white noise and one minute after the transfer, they are scored for ptosis on the following scale: Eyes closed = 4, eyes 3/4 closed = 3, eyes 1/2 closed = 2, eyes 1/4 closed = 1, eyes open = 0̸. The total score for each group of five in a primary screen will, therefore, be from 0̸ to 20̸ and these scores are used as indications of drug activity.

The vehicle control group score is used as a determinant of the validity of each test. If the control score is less than 17, the results are discarded and the test repeated. The calculation of percent inhibition of ptosis is: 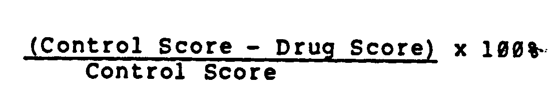
For ED_{50̸} estimation, four or five doses are administered in order to bracket the estimated value and only vehicle control scores of 27 to 32 are accepted to assure the accuracy of the ED_{50̸} estimation.

Linear regression analysis is used to estimate ED_{50̸} values and 95% confidence intervals.

The results of some of the compounds of this invention are shown in Table 2 along with a result for desipramine (prior art compound).

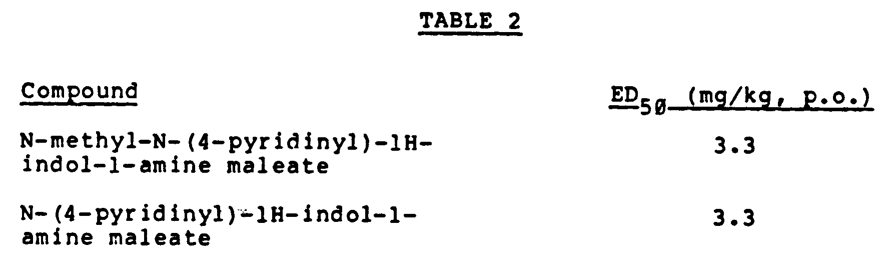

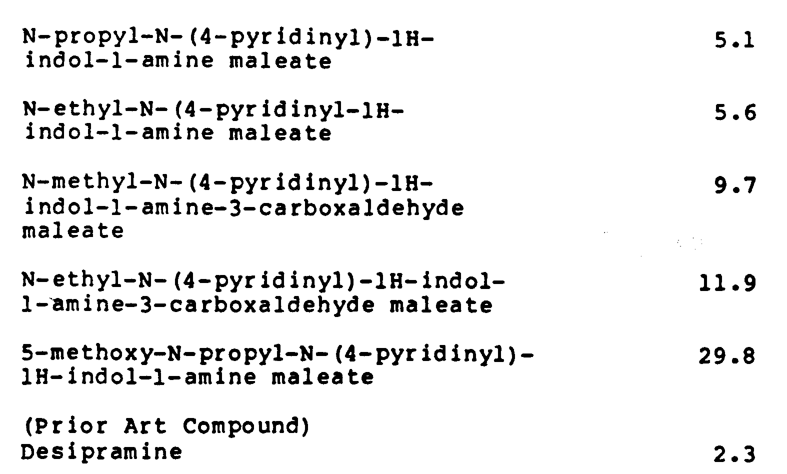

Compounds I of the present invention are also useful as analgesic agents due to their ability to alleviate pain in mammals. The activity of the compounds is demonstrated in the 2-phenyl-1,4-benzoquinone-induced writhing (PQW) test in mice, a standard assay for analgesia [Proc. Soc. Exptl. Biol. Med., 95, 729 (1957)] and in modified Haffner's analgesia.

The latter assay is used to evaluate analgesic activity by measuring drug-induced changes in the sensitivity of mice to pressure stress by placing an artery clip (2 1/2 inches long) on their tail. The procedure used is a modification of the test developed by Haffner, Dtsch. Med. Wschr. 55, 731 (1929), and it is described below.

### METHOD:

Male mice (Charles River CD-1) from 18-30̸ grams are used for the test. An artery clip is applied to the root of the tail of a mouse (approximately 1/2 inch from the body) to induce pain. The animals quickly responds to this noxious stimuli by biting the clip or the location of the clip. This reaction time, the interval between stimulus onset and response, is recorded in 1/10̸ second increments by a stop watch.

For at time response, the screening dose (25 mg/kg) is administered subcutaneously 10̸ ml/kg) to the animal receiving food and water ad libitum before testing. Animals receiving the compound orally are fasted 18-24 hours before drug administration. Drug to be tested is prepared with distilled water and if insoluble, one drop of a surfactant is added.

Twenty-eight animals (seven/group) are administered the drug 15, 30̸, 45 and 60̸ minutes prior to testing.

The cut-off time (CO) is determined by taking the (x̅) average + 3 standard (SD) deviation of the combined response latencies of the control mice in all time periods.

$\text{CO =} \overline{\text{x}} \text{+ 3 SD (seconds)}$

Any reaction time, in subsequent drug tests, which is greater than the CO (for the same time period) therefore exceeds 99% of a noraml Gaussian distribution and is called "positive response" indicative of analgesic activity. A time response indicates the period of greatest analgesic effect after dosing. The ED_{50̸} is determined at the peak time of drug activity. A minimum of three dose groups are used. ED_{50̸}'s are calculated using computer analysis.

The results of some of the compounds of this invention are shown in Table 3 along with those of a prior art compound.

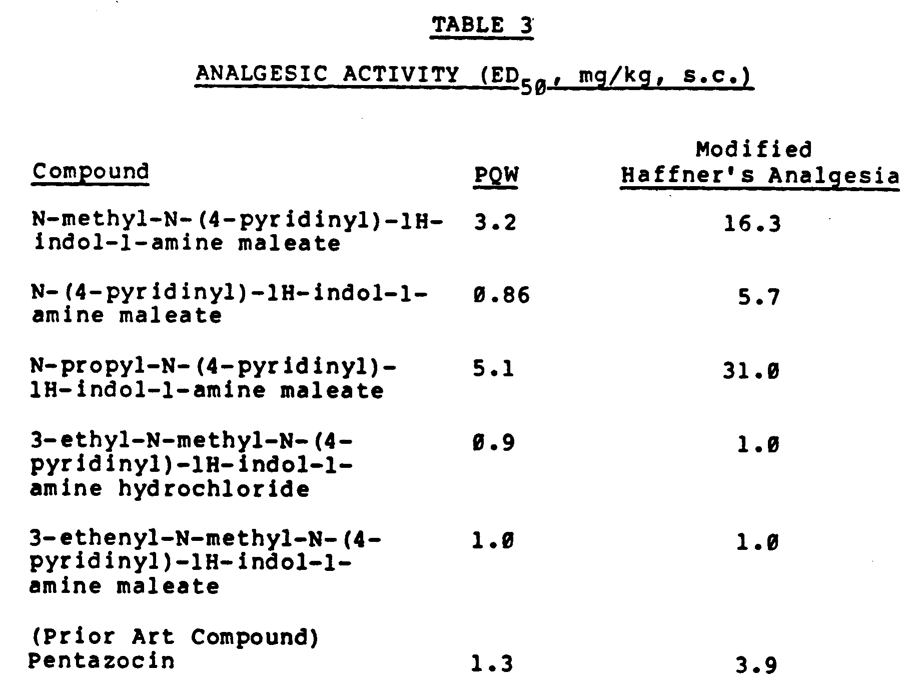

Effective quantities of the compounds of the invention may be administered to a patient by any of the various methods, for example, orally as in capsules or tablets, parenterally in the form of sterile solutions or suspensions, and in some cases intravenously in the form of sterile solutions. The free base final products, while effective themselves, may be formulated and administered in the form of their pharmaceutically acceptable acid addition salts for purposes of stability, convenience of crystallization, increased solubility and the like.

Acids useful for preparing the pharmaceutically acceptable acid addition salts of the invention include inorganic acids such as hydrochloric, hydrobromic, sulfuric, nitric, phosphoric and perchloric acids, as well as organic acids such as tartaric, citric, acetic, succinic, salicylic, maleic, fumaric and oxalic acids.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0̸.5% of active compound, but may be varied depending upon the particular form and may conveniently be between 4% to about 70̸% of the weight of the unit. The amount of active compound in such compositions is such that a suitable dosage will be obtained. Preferred compositions and preparations according to the present invention are prepared so that an oral dosage unit form contains between 1.0̸-30̸0̸ milligrams of active compound.

The tablets, pills, capsules, troches and the like may also contain the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweetening agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes, coloring and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0̸.1% of active compound, but may be varied between 0̸.5 and about 30̸% of the weight thereof. The amount of active compound in such compositions is such that a suitable dosage will be obtained. Preferred compositions and preparations according to the present invention are prepared so that a parenteral dosage unit contains between 0̸.5 to 10̸0̸ milligrams of active compound. The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparations can be enclosed in disposable syringes or multiple dose vials made of glass or plastic.

Examples of the compounds of this invention include:
N-(4-pyridinyl)-1H-indol-1-amine;
N-methyl-N-(4-pyridinyl)-1H-indol-1-amine;
N-ethyl-N-(4-pyridinyl)-1H-indol-1-amine;
N-propyl-N-(4-pyridinyl)-1H-indol-1-amine;
5-methoxy-N-propyl-N-(4-pyridinyl)-1H-indol-1-amine;
N-methyl-N-(4-pyridinyl)-1H-indol-1-amine-3-carboxaldehyde;
N-ethyl-N-(4-pyridinyl)-1H-indol-1-amine-3-carboxaldehyde;
N-(4-nitro-3-pyridinyl)-1H-indol-1-amine-N¹-oxide;
N-(4-amino-3-pyridinyl)-1H-indol-1-amine-N¹-oxide;
3-ethenyl-N-methyl-N-(4-pyridinyl)-1H-indol-1-amine;
3-ethyl-N-methyl-N-(4-pyridinyl)-1H-indol-1-amine;
5-chloro-N-(4-pyridinyl)-1H-indol-1-amine;
5-chloro-N-propyl-N-(4-pyridinyl)-1H-indol-1-amine;
5-bromo-N-(4-pyridinyl)-1H-indol-1-amine;
5-bromo-N-methyl-N-(4-pyridinyl)-1H-indol-1-amine; and
5-bromo-N-propyl-N-(4-pyridinyl)-1H-indol-1-amine.

### EXAMPLE 1

### N-(4-Pyridinyl)-1H-indol-1-amine maleate

A solution of 1H-indol-1-amine (30̸ g), 4-chlorpyridine hydrochloride (34 g) and pyridine (18 g) in 250̸ ml of isopropanol was stirred at 85° for 1.5 hours, and thereafter cooled, stirred with ice-water, basified with sodium carbonate and extracted with dichloromethane. The organic extract was washed with water and saturated sodium chloride solution, was dried over anhydrous magnesium sulfate, filtered and concentrated to a dark oil. This oil was purified by flash chromatography (silica, ethyl acetate) and thereafter by column chromatography (alumina, ether) to give 24 g of oil. A 3.6 g sample was purified by high performance liquid chromatography (HPLC hereafter) (silica, ethyl acetate) to give 3.5 g of oil. This oil was converted to the maleate salt and recrystallized twice from methanol/ether to give 3.8 g of needles, d 145-146°.

| ANALYSIS: | | | |
|---|---|---|---|
| Calculated for C₁₃H₁₁N₃·C₄H₄O₄: | 62.76%C | 4.65%H | 12.92%N |
| Found: | 62.62%C | 4.81%H | 12.73%N |

### EXAMPLE 2

### N-Methyl-N-(4-pyridinyl)-1H-indol-1-amine maleate

A solution of N-(4-pyridinyl)-1H-indol-1-amine (7.4 g) in 30̸ ml of dimethylformamide was added to an ice-cooled suspension of NaH (1.6 g of 60̸% NaH dispersion in mineral oil was washed with hexanes, the liquid portion was decanted and the residual solid was dispersed in 10̸ ml of dimethylformamide). After anion formation, a solution of dimethylsulfate (5 g) in 10̸ ml of dimethylformamide was added. After one hour of stirring at ambient temperature, the reaction mixture was stirred with ice-water and extracted with ether. The organic extract was washed with water and saturated sodium chloride solution, was dried over anhydrous magnesium sulfate, filtered and concentrated to 8 g of oil. This oil was purified by flash chromatography (silica, ethyl acetete), column chromatography (alumina, ether) and HPLC (silica, ethyl acetate) to give 2.9 g of oil. This oil was converted to the maleate salt and was recrystallized twice from methanol/ether to give 2.1 g of crystals, mp 10̸3-10̸4°.

| ANALYSIS: | | | |
|---|---|---|---|
| Calculated for C₁₄H₁₃N₃·C₄H₄O₄: | 63.70̸%C | 5.0̸5%H | 12.39%N |
| Found: | 63.36%C | 4.93%H | 12.39%N |

### EXAMPLE 3

### N-Ethyl-N-(4-pyridinyl)-1H-indol-1-amine maleate

To an ice-cooled suspension of sodium hydride (1.7 g of 60̸% NaH dispersion in mineral oil was washed with hexanes, the liquid was decanted and the residual solid was dispersed in 5 ml of dimethylformamide) was slowly added a solution of N-(4-pyridinyl)-1H-indol-1-amine (7.6 g) in 25 ml of dimethylformamide. After anion formation, a solution of diethyl sulfate (6.4 g) in 10̸ ml of dimethylformamide was slowly added. After one hour, the mixture was stirred with ice-water and extracted with ethyl acetate. The organic extract was washed with water and saturated sodium chloride solution, was dried over anhydrous magnesium sulfate, filtered and concentrated to 11 g of oil. This oil was purified by flash chromatography (silica, ethyl acetate) to give 6.2 of oil. This oil was purified by column chromatography (alumina, ether) to give 6 g of oil. A 3 g sample was converted to the maleate salt and recrystallized from ethanol/ether and thereafter from methanol/ether to give 2.7 g of crystals, mp 119-120̸°.

| ANALYSIS: | | | |
|---|---|---|---|
| Calculated for C₁₅H₁₅N₃·C₄H₄O₄: | 64.58%C | 5.42%H | 11.89%N |
| Found: | 64.27%C | 5.49%H | 12.11%N |

### EXAMPLE 4

### N-Propyl-N-(4-pyridinyl)-1H-indol-1-amine maleate

A solution of N-(4-pyridinyl)-1H-indol-1-amine (6 g) in 25 ml of dimethylformamide was slowly added to an ice-cooled suspension of NaH (1.3 g of 60̸% NaH dispersion in mineral oil was washed with hexanes, the liquid was decanted and the residual solid was dispersed in 5 ml of dimethylformamide). After anion formation, a solution of 1-bromopropane (4 g) in 5 ml of dimethylformamide was added. After one hour of stirring at ambient temperature, the reaction mixture was stirred with ice-water and extracted with dichloromethane. The organic extract was washed with water and saturated sodium chloride solution, was dried over anhydrous magnesium sulfate, filtered and concentrated to 8 g of oil. This oil was purified by HPLC (silica, ethyl acetate) and thereafter by column chromatography (alumina, ether) to give 6.4 g oil. This oil was converted to the maleate salt and recrystallized from methanol/ether to give 6.8 g of crystals, mp 115-116°.

| ANALYSIS: | | | |
|---|---|---|---|
| Calculated for C₁₆H₁₇N₃·C₄H₄O₄: | 65.38%C | 5.76%H | 11.44%N |
| Found: | 65.26%C | 5.71%H | 11.34%N |

### EXAMPLE 5

### 5-Methoxy-N-propyl-N-(4-pyridinyl)-1H-indol-1-amine maleate

To an ice-cooled suspension of sodium hydride (0̸.5 g of 60̸% NaH dispersion in mineral oil was wahsed with hexanes, the liquid was decanted and the residual solid was dispersed in 5 ml of dimethylformamide)was slowly added a solution of 5-methoxy-N-(4-pyridinyl)-1H-indol-1-amine (2.3 g) in 20̸ ml of dimethylformamide. After anion formation, a solution of 1-bromopropane (1.4 g) in 5 ml of dimethylformamide was added. After one hour of stirring, the reaction mixture was stirred with ice-water and extracted with dichloromethane. The organic extract was washed with water and saturated sodium chloride solution, was dried over anhydrous magnesium sulfate, filtered and concentrated to 2.3 g of oil. This oil was purified by flash chromatography (silica, ethyl acetate) to give 2.1 g of oil. This oil was converted to the maleate salt in ethanol/ether to give 2.0̸ g of crystals, mp 138-139°C.

| ANALYSIS: | | | |
|---|---|---|---|
| Calculated for C₁₇H₁₉N₃O·C₄H₄O₄: | 63.46%C | 5.83%H | 10̸.58%N |
| Found: | 63.26%C | 5.77%H | 10̸.47%N |

### EXAMPLE 6

### N-Methyl-N-(4-pyridinyl)-1H-indol-1-amine-3-carboxaldehyde maleate

To ice-cooled dimethylformamide (4 g) was slowly added phosphorous oxychloride (7 g). After complex formation, a solution of N-methyl-N-(4-pyridinyl)-1H-indol-1-amine (5 g) in 50̸ ml of dichloroethane was added. After one hour of stirring at 85°C, the reaction mixture was cooled, hydrolyzed with a solution of sodium acetate (5 g) in 25 ml of water, again cooled, basified with sodium carbonate and extracted with dichloromethane. The organic extract was washed with water and saturated sodium chloride solution, was dried over anhydrous magnesium sulfate, fitlered and concentrated to 6 g of oil. This oil was purified by flash chromatography (silica, ethyl acetate) to give 4.6 of oil. This oil was converted to the maleate salt and recrystallized from ethanol/ether and thereafter from methanol/ether to give 2.6 g of crystals, d 162-163°.

| ANALYSIS: | | | |
|---|---|---|---|
| Calculated for C₁₅H₁₃N₃O·C₄H₄O₄: | 62.12%C | 4.66%H | 11.44%N |
| Found: | 61.71%C | 4.62%H | 11.14%N |

### EXAMPLE 7

### N-Ethyl-N-(4-pyridinyl)-1H-indol-1-amine-3-carboxaldehyde maleate

To ice-cooled dimethylformamide (2.2. g) was slowly added phosphorous oxychloride (4.5 g). After complex formation, a solution of N-ethyl-N-(4-pyridinyl)-1H-indol-1-amine (3.5 g) in 50̸ ml of dichloroethane was added. The mixture was stirred at 80̸° for one hour and thereafter hydrolyzed with a solution of sodium acetate (5 g) in 25 ml of water, cooled, basified with sodium carbonate and extracted with dichloromethane. The organic extract was washed with water and saturated sodium chloride solution, was dried over anhydrous magnesium sulfate, filtered and concentrated to 5 g of oil. This oil was purified by flash chromatography (silica, ethyl acetate) to give 3.5 g of oil. This oil was converted to the maleate salt and recrystallized from ethanol/ether and thereafter from methanol/ether to give 3 g of solid, d 170̸-171°.

| ANALYSIS: | | | |
|---|---|---|---|
| Calculated for C₁₆H₁₅N₃O·C₄H₄O₄: | 62.98%C | 5.0̸2%H | 11.0̸2%N |
| Found: | 62.97%C | 5.0̸8%H | 11.0̸6%N |

### EXAMPLE 8

### N-(4-Nitro-3-pyridinyl)-1H-indol-1-amine N¹-oxide

A solution of 3-fluoro-4-nitro-pyridine-N-oxide (5 g) and 1H-indol-1-amine (4.2 g) in 25 ml of ethanol was stirred four hours at reflux. The solution was cooled and the product collected and dried to give 4 g of solid, d 195°. This material was recrystallized from ethanol/ether to give 2.2 g of solid, d 20̸0̸-20̸1°.

| ANALYSIS: | | | |
|---|---|---|---|
| Calculated for C₁₃H_{10̸}N₄O₃: | 57.77%C | 3.73%H | 20̸.74%N |
| Found: | 57.56%C | 3.74%H | 20̸.53%N |

### EXAMPLE 9

### N-(4-Amino-3-pyridinyl)-1H-indol-1-amine N¹-oxide

A mixture of N-(4-nitro-3-pyridinyl)-1H-indol-1-amine N¹-oxide (5 g) and 0̸.5 g of platinum oxide in 250̸ ml of ethanol was hydrogenated at 50̸ psi (pounds per square inch) for four hours. The mixture was filtered and the filtered liquid was concentrated to a dark residue which was purified by flash chromatography (silica, 10̸% methanol in dichloromethane) to give 2.5 g of solid, d 235°. This material was recrystallized from methanol/ether to give 2.1 g of crystals, d 235°.

| ANALYSIS: | | | |
|---|---|---|---|
| Calculated for C₁₃H₁₂N₄O: | 64.98%C | 5.0̸3%H | 23.32%N |
| Found: | 64.74%C | 5.12%H | 23.0̸4%N |

### EXAMPLE 10̸

### 3-Ethenyl-N-methyl-N-(4-pyridinyl)-1H-indol-1-amine maleate

To an ice-cooled suspension of methyltriphenylphosphonium bromide (13 g) in 10̸0̸ ml of anhydrous ether was added potassium t-butoxide (4 g). After phosphorane formation, a solution of N-ethyl-N-(4-pyridinyl)-1H-indol-1-amine-3-carboxaldehyde (7.5 g) in 50̸ ml of ether and 50̸ ml of tetrahydrofuran was added. After one hour of stirring, the reaction mixture was stirred with water and extracted with ether. The organic extract was washed with water and saturated sodium chloride solution, was dried over anhydrous magnesium sulfate, filtered and concentrated to 20̸ g of oil. This oil was purified by flash chromatography (silica, ethyl acetate) to give 7 g of oil. A 3.5 g sample was converted to the maleate salt in ethanol and recrystallized from methanol/ether to give 3 g of crystals, mp 153-154°.

| ANALYSIS: | | | |
|---|---|---|---|
| Calculated for C₁₆H₁₅N₃·C₄H₄O₄: | 65.74%C | 5.24%H | 11.50̸%N |
| Found: | 65.94%C | 5.39%H | 11.45%N |

### EXAMPLE 11

### 3-Ethyl-N-methyl-N-(4-pyridinyl)-1H-indol-1-amine hydrochloride

A solution of 3-ethenyl-N-methyl-N-(4-pyridinyl)-1H-indol-1-amine (5 g) in 250̸ ml of ethanol containing 0̸.5 g of platinum oxide was hydrogenated at 50̸ psi for one hour. The mixture was filtered and the filtered liquid was concentrated to 5 g of oil. This oil was purified by flash chromatography (silica, ethyl acetate) to give 3.5 g of oil. This oil was converted to the hydrochloride salt in ethanol/ether and recrystallized from methanol/ether to give 3.0̸ g of crystals, d 262°.

| ANALYSIS: | | | |
|---|---|---|---|
| Calculated for C₁₆H₁₇N₃·HCl: | 66.77%C | 6.30̸%H | 14.60̸%N |
| Found: | 66.87%C | 6.33%H | 14.57%N |

### EXAMPLE 12

### 5-Chloro-N-(4-pyridinyl)-1H-indol-1-amine maleate

A solution of 5-chloro-1H-indol-1-amine (9 g), 4-chloropyridine hydrochloride (12 g) and pyridine (6.4 g) in 10̸0̸ ml of isopropanol was stirred at reflux for one hour, cooled and stirred with ice-water, and the mixture was basified with sodium carbonate, extracted with dichloromethane and filtered. The organic extract was washed with water and saturated sodium chloride, was dried over anhydrous magnesium sulfate, filtered and concentrated to a dark oil. This oil was purified by flash chromatography (silica, ethyl acetate) to give 6.2 g of oil. This oil was converted to the maleate salt in methanol-ether to give 7 g of crystals, mp 148-150̸°C. A 2.6 g sample was recrystallized from methanol-ether to give 2.4 g of crystals,
d 150̸-152°.

| ANALYSIS: | | | |
|---|---|---|---|
| Calculated for C₁₃H_{10̸}ClN₃·C₄H₄O₄: | 56.75%C | 3.92%H | 11.68%N |
| Found: | 56.71%C | 4.0̸0̸%H | 11.62%N |

### EXAMPLE 13

### 5-Chloro-N-propyl-N-(4-pyridinyl)-1H-indol-1-amine maleate

A solution of 5-chloro-N-(4-pyridinyl)-1H-indol-1-amine (3.3 g) in 15 ml of dimethylformamide was slowly added to an ice-cooled suspension of sodium hydride (0̸.65 g of 60̸% oil dispersion was washed with hexanes) in 5 ml of dimethylformamide. After anion formation a solution of 1-bromopropane (2 g) in 5 ml of dimethylformamide was added. After one hour the reaction mixture was stirred with ice-water and extracted with dichloromethane. The organic extract was washed with water and saturated sodium chloride, was dried over anhydrous magnesium sulfate, filtered and concentrated to 5 g of oil. This oil was purified by flash chromatography (silica, ethyl acetate) to give 3.1 g of oil. This oil was converted to the maleate salt in ethanol-ether and thereafter recrystallized from methanol-ether to give 3.4 g of crystals, mp 130̸°.

| ANALYSIS: | | | |
|---|---|---|---|
| Calculated for C₁₆H₁₆ClN₃·C₄H₄O₄: | 59.77%C | 5.0̸2%H | 10̸.46%N |
| Found: | 59.97%C | 5.13%H | 10̸.35%N |

### EXAMPLE 14

### 5-Bromo-N-(4-pyridinyl)-1H-indol-1-amine maleate

A solution of 5-bromo-1H-indol-1-amine (13 g), 4-chloropyridine hydrochloride (14 g) and pyridine (7.2 g) in 10̸0̸ ml of isopropanol was stirred at reflux for one hour, cooled and stirred with ice-water, and thereafter the mixture was basified with sodium carbonate, extracted with dichloromethane and filtered. The organic extract was washed with water and saturated sodium chloride, was dried over anhydrous magnesium sulfate, filtered and concentrated to a dark oil. This oil was purified by flash chromatography (silica, ethyl acetate) to give 11 g of oil. This oil was converted to the maleate salt in ethanol-ether to give 13 g of solid, d 155-157°. A three gram sample was recrystallized from methanol-ether to give 2.8 g of crystals, d 161-162°.

| ANALYSIS: | | | |
|---|---|---|---|
| Calculated for C₁₃H_{10̸}BrN₃·C₄H₄O₄: | 50̸.51%C | 3.49%H | 10̸.40̸%N |
| Found: | 50̸.46%C | 3.56%H | 10̸.40̸%N |

### EXAMPLE 15

### 5-Bromo-N-methyl-N-(4-pyridinyl)-1H-indol-1-amine maleate

A solution of 5-bromo-N-(4-pyridinyl)-1H-indol-1-amine (2.7 g) in 20̸ ml of dimethylformamide was slowly added to an ice-cooled suspension of sodium hydride (0̸.45 g of 60̸% oil dispersion was washed with hexanes) in 5 ml of dimethylformamide. After anion formation a solution of dimethylsulfate (1.4 g) in 5 ml of dimethylformamide was added. After one hour the reaction mixture was stirred with ice-water and extracted with dichloromethane. The organic extract was washed with water and saturated sodium chloride, was dried over anhydrous magnesium sulfate, filtered and concentrated to 2 g of oil. This oil was purified by flash chromatography (silica, ethyl acetate) to give 1.4 g of oil. This oil was converted to the maleate salt in ethanol-ether to give 1.2 g of crystals, mp 110̸-111°.

| ANALYSIS: | | | |
|---|---|---|---|
| Calculated for C₁₄H₁₂BrN₃·C₄H₄O₄: | 51.69%C | 3.86%H | 10̸.0̸5%N |
| Found: | 51.55%C | 3.89%H | 10̸.14%N |

### EXAMPLE 16

### 5-Bromo-N-propyl-N-(4-pyridinyl)-1H-indol-1-amine maleate

A solution of 5-bromo-N-(4-pyridinyl)-1H-indol-1-amine (4.9 g) in 25 ml of dimethylformamide was slowly added to an ice-cooled suspension of sodium hydride (0̸.8 g of 60̸% oil dispersion was washed with hexanes) in 5 ml of dimethylformamide. After anion formation a solution of 1-bromopropane (2.5 g) in 5 ml of dimethylformamide was added. After one hour the reaction mixture was stirred with ice-water and extracted with dichloromethane. The organic extract was washed with water and saturated sodium chloride, was dried over anhydrous magnesium sulfate, filtered and concentrated to 5 g of oil. This oil was purified by flash chromatography (silica, ethyl acetate) to give 4.5 g of oil. This oil was converted to the maleate salt in ethanol-ether to give 5.4 g of solid, d 150-152°. This solid was recrystallized from methanol-ether to give 4.8 g of crystals,
d 157-158°.

| ANALYSIS: | | | |
|---|---|---|---|
| Calculated for C₁₆H₁₆BrN₃·C₄H₄O₄: | 53.82%C | 4.52%H | 9.42%N |
| Found: | 53.63%C | 4.62%H | 9.40%N |

### EXAMPLE 17

### 5-Nitro-N-(4-pyridinyl)-1H-indol-1-amine hydrochloride

A solution of 5-nitro-1H-indol-1-amine (4.5 g) and 4-chloropyridine hydrochloride (4.5 g) in 175 ml of isopropanol was stirred at reflux for two hours, another equivalent of 4-chlorpyridine hydrochloride was added and the mixture was refluxed for two additional hours. The reaction mixture was then cooled, stirred with water, basified with sodium carbonate and extracted with ethyl acetate. The organic extract was washed with water and saturated sodium chloride solution, was dried (MgSO₄), filtered and concentrated to 9 g of dark oil. This oil was purified by flash chromatography (silica, ethyl acetate) to give 3.8 g of light brown solid, m.p. 183-184°. This material was converted to the hydrochloride salt and recrystallized twice from methanol/ether to give 3.5 g of orange needles, d 300-302°.

| ANALYSIS: | | | |
|---|---|---|---|
| Calculated for C₁₃H₁₀N₄O₂·HCl: | 55.71%C | 3.81%H | 19.28%N |
| Found: | 53.55%C | 3.77%H | 19.17%N |

### EXAMPLE 18

### 5-Methyl-5-nitro-N-(4-Pyridinyl)-1H-indol-1-amine maleate

A solution of 5-nitro-N-(4-pyridinyl)-1H-indol-1-amine (6 g) in 20 ml of dimethylformamide was slowly added to an ice-cooled NaH suspension prepared by washing 1.2 g of 60 % NaH suspension in oil with hexanes and suspending the residue in 5 ml dimethylformamide. After the anion formation a solution of dimethyl sulfate (3.7 g) in 10 ml of dimethylformamide was added. After one hour the reaction mixture was stirred with water and extracted with ethyl acetate. The organic extract was washed with water and saturated sodium chloride solution, was dried (MgSO₄), filtered and concentrated to 6 g of dark oil. This was purified by flash chromatography (silica, ethyl acetate) to give 2.7 g of orange solid, m.p. 149-150°. This was converted to the maleate salt and recrystallized twice from methanol/ether to give 2.7 g of orange crystals,
d 174-175°.

| ANALYSIS: | | | |
|---|---|---|---|
| Calculated for C₁₄H₁₂N₄O₂:C₄H₄O₄: | 56.25%C | 4.20%H | 14.58%N |
| Found: | 56.14%C | 4.27%H | 14.46%N |

### EXAMPLE 19

### 3-Methyl-N-(4-pyridinyl)-1H-indol-1-amine oxalate

To 200 ml of isopropanol were added 4-chloropyridine hydrochloride (7.5 g) and 3-methyl-1H-indole-1-amine (7.6 g). The mixture was stirred at 90°C for six hours, and thereafter poured into 400 ml of ice water, and stirred for five minutes. The pH was adjusted to 10 with Na₂Co₃ solution and then extracted with ethyl acetate. The organic layer was washed with water and dried (saturated NaCl, anhydrous MgSO₄).

After filtration, the solvent was evaporated to obtain 8.4 g of thick brown oil, which was eluted on a silica gel column with ethyl acetate via HPLC. The desired fractions were combined and concentrated to 7.4 g of brown oil. A 2.3 g sample of this oil was dissolved in 50 ml of ethanol, and the pH adjusted to 1 with an ethanolic solution of oxalic acid, and the solution was diluted with ether. The resultant white precipitate was collected and dried to give 4.0 g, d 130-135°C. This material was recrystallized from ethanol/ether (1:1) to give 3,8 g,
d 137°C.

| ANALYSIS: | | | |
|---|---|---|---|
| Calculated for C₁₄H₁₃N₃·C₂H₂O₄: | 61.33%C | 4.83%H | 13.41%N |
| Found: | 61.41%C | 4.96%H | 13.28%N |

### EXAMPLE 20

### 3-Methyl-N-propyl-N-(4-pyridinyl)-1H-indol-1-amine maleate

To a cold NaH suspension prepared by washing 0.8 g of 60 % NaH suspension in oil with hexanes and suspending the residue in 15 ml of dry DMF was added a solution of 3-methyl-N-(4-pyridinyl)-1H-indol-1-amine (4.0 g) in 25 ml of dry DMF in ten minutes.

The mixture was stirred at ambient temperature for thirty minutes, poured into 200 ml of ice water, stirred for five minutes, and then extracted with ethyl acetate. The organic layer was washed with water and dried (saturated NaCl, anhydrous MgSO₄).

After filtration, the solvent was evaporated to give 5 g of brown oil, which was eluted on a silica gel column with ethyl acetate via HPLC. The desired fractions were combined and concentrated to 2.6 g of brown oil.

This oil was dissolved in ether, the pH was adjusted to 1 with ethereal maleic acid, and the resultant white precipitate collected and dried to give, 4.0 g, d.: 148°C. This material was recrystallized from methanol/ether (1:10) to give 3.5 g of white crystals, m.p. 148-149°C.

| ANALYSIS: | | | |
|---|---|---|---|
| Calculated for C₁₇H₁₉N₃·C₄H₄O₄: | 66.13%C | 6.08%H | 11.02%N |
| Found: | 66.15%C | 6.02%H | 11.00%N |

### EXAMPLE 21

### N-(3-Fluoro-4-pyridinyl)-3-methyl-1H-indol-1-amine

To a 200 ml of isopropanol were added 4-chloro-3-fluoropyridine hydrochloride (10 g) and 3-methyl-1H-indol amine (5.9 g). The mixture was stirred at 90°C for four hours, cooled and poured into 500 ml of ice water. The pH was adjusted to 10 with Na₂CO₃ solution, and the mixture was extracted with ethyl acetate. The organic layer was washed with water and dried (saturated NaCl, anhydrous MgSO₄).

After filtration, the solvent was evaporated to give about 10 g of dark oil, which was eluted on a silica gel column first with dichloromethane, and then with ether/petroleum ether (1:1) via "flash chromatography". The desired fractions were combined and concentrated to a yellow solid, 6,2 g, m.p. 45°C. A sample of this material was recrystallized from isopropyl ether/hexanes (1:1) to give a yellow solid, m.p. 141-142°C.

| ANALYSIS: | | | |
|---|---|---|---|
| Calculated for C₁₄H₁₂FN₃: | 69.69%C | 5.02%H | 17.42%N |
| Found: | 69.52%C | 5.01%H | 17.57%N |

### EXAMPLE 22

### N-(3-Fluoro-4-pyridinyl)-N-propyl-3-methyl-1H-indol-1-amine hydrochloride

To a NaH suspension prepared by washing 0.5 g of 60 % NaH suspension in oil with hexanes and suspending the residue in 10 ml DMF, was added a solution of N-(3-fluoro-4-pyridinyl)-3-methyl-1H-indol-1-amine (3.0 g) in 20 ml of DMF at ice-bath temperature in ten minutes. The mixture was stirred for an additional five minutes, and thereafter a solution of propyl bromide (1.2 ml) in 10 ml of DMF was added in five minutes.

The mixture was stirred at ambient temperature for thirty minutes, poured into 10 ml of ice-water, and then extracted with ethyl acetate. The organic layer was collected, washed with water and dried (saturated NaCl, anhydrous MgSO₄).

After filtration, the solvent was evaporated to give 4 g of brown oil, which was eluted on a silica column with 20 % ethyl acetate/DCM via HPLC. The desired fractions were combined and concentrated to a thick yellow oil, 3.4 g.

The oil was dissolved in ether, the pH adjusted to 1 with ethereal-HCl, and the resultant white precipitate collected and dried to give 3.4 g. This material was recrystallized from ethanol/ether (1:20) to give 2.7 g of white crystals,
d 193°.

| ANALYSIS: | | | |
|---|---|---|---|
| Calculated for C₁₇H₁₈FN₃:HCl: | 63.84%C | 5.99%H | 13.14%N |
| Found: | 64.11%C | 6.01%H | 13.20%N |

### EXAMPLE 23

### N-(2-Propen-1-yl)-N-(4-pyridinyl)-1H-indol-1-amine maleate

A solution of N-(4-pyridinyl)-1H-indol-1-amine (8 g) in 40 ml of dimethylformamide was added to an ice-cooled NaH suspension prepared by washing 2 g of 60 % NaH suspension in oil with hexanes and suspending the residue in 10 ml of dimethylformamide. After the anion formation a solution of allyl bromide (6 g) in 10 ml of dimethylformamide was added. After one hour the reaction mixture was stirred with ice-water and extracted with ethyl acetate. The organic extract was washed with water and saturated sodium chloride, dried (MgSO₄), filtered and concentrated to an oil. This oil was purified first by flash chromatography (silica, ethyl acetate) and then by column chromatography (alumina, ether) to give 5 g of oil. This oil was converted to the maleate salt in methanol/water to give 6 g of pale yellow solid, m.p. 105-107°. This is combined with 1.8 g product from a previous reaction and recrystallized from methanol/ether to give 5 g of pale yellow crystals, m.p. 111-112°.

| ANALYSIS: | | | |
|---|---|---|---|
| Calculated for C₁₆H₁₅N₃·C₄H₄O₄: | 65.74%C | 5.24%H | 11.50%N |
| Found: | 65.80%C | 5.24%H | 11.51%N |

### EXAMPLE 24

### N-(3-Fluoro-4-pyridinyl)-N-propyl-1H-indol-1-amine hydrochloride

To an NaH suspension prepared by washing 0.6 g of 60 % NaH suspension in oil with hexanes and suspending the residue in 10 ml of cold DMF, was added a solution of N-(3-fluoro-4-pyridinyl)-1H-indol-1-amine in 25 ml of DMF.

The mixture was stirred at 5°C for ten minutes, and thereafter a solution of bromopropane (1.4 ml) in 10 ml of DMF was added. The mixture was stirred at ambient temperature for thirty minutes, poured into 200 ml of ice water, stirred for five minutes, and extracted with ethyl acetate. The organic layer was washed with water and dried (saturated NaCl, anhydrous MgSO₄).

After filtration, the solvent was evaporated to give 3.2 g of brown oil, which was eluted on a silica gel column with 10 % ethyl acetate/ DCM via HPLC. The desired fractions were combined and concentrated to 2.4 g of brown oil, which was dissolved in 40 ml of absolute ethanol. The pH was adjusted to 1 with ethereal-HCl, and the solution was diluted with 400 ml of ether. The resultant off-white precipitate was collected and dried to give 2.1 g, m.p. 198-200°C dec.

| ANALYSIS: | | | |
|---|---|---|---|
| Calculated for C₁₆H₁₆FN₃·HCl: | 62.85%C | 5.60%H | 13.74%N |
| Found: | 62.80%C | 5.60%H | 13.66%N |

## Claims (Claims for the following Contracting State(s): AT, BE, CH, DE, FR, GB, IT, LI, LU, NL, SE)

1. A compound having the formula 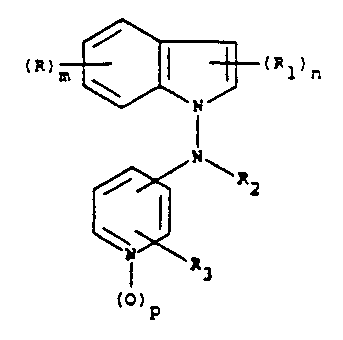 where m is 1 or 2; n is 1 or 2; P is 0̸ or 1; each R is independently hydrogen, halogen, C₁-C₆-alkyl, C₁-C₆-alkoxy, hydroxy, nitro, amino, C₁-C₆-alkylamino, C₁-C₆-alkylcarbonylamino, cyano, formyl, C₁-C₆-alkoxycarbonyl, C₁-C₆-alkylthio or C₁-C₆-alkoxycarbonyl-C₁-C₆-alkylthio; each R₁ is independently hydrogen, C₁-C₆-alkyl, C₂-C₆-alkenyl, formyl, C₁-C₆-alkylcarbonyl, phenyl-C₁-C₆-alkylcarbonyl, phenylcarbonyl, halogen, phenyl-C₂-C₆-alkenyl, phenyl-C₁-C₆-alkyl, heteroaryl-C₂-C₆-alkenyl, heteroaryl-C₁-C₆-alkyl, cyano-C₂-C₆-alkenyl, cyano-C₁-C₆-alkyl, methoxy-C₂-C₆-alkenyl, methoxy-C₁-C₆-alkyl, C₁-C₆-alkoxycarbonyl-C₂-C₆-alkenyl, C₁-C₆-alkoxycarbonyl-C₁-C₆-alkyl, C₃-C₇-cycloalkyl-C₂-C₆-alkenyl or C₃-C₇-cycloalkyl-C₁-C₆-alkyl, the term phenyl signifying a phenyl group which may have 1-3 substituents each of which being independently C₁-C₆-alkyl, C₁-C₆-alkoxy, halogen, CF₃, NO₂ or CN, and the term heteroaryl signifying a group
of the formula 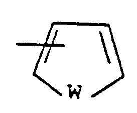 where W is O, S, NH or CH=N, cyano, -CH(OH)R₄, -C(OH)R₄R₅ or -CH₂OR₅, R₄ being hydrogen, C₁-C₆-alkyl, phenyl-C₁-C₆-alkyl or phenyl and R₅ being C₁-C₆-alkyl, phenyl-C₁-C₆-alkyl or phenyl;
each R₂ is independently hydrogen, C₁-C₆-alkyl, C₂-C₆-alkenyl, C₂-C₆-alkynyl, C₁-C₆-alkoxycarbonyl-C₁-C₆-alkyl, C₁-C₆-alkylaminocarbonyl-C₁-C₆-alkyl, aminocarbonyl-C₁-C₆-alkyl, phenyl-C₁-C₆-alkyl, C₁-C₆-alkoxycarbonyl, phenyl-C₁-C₆-alkoxycarbonyl, phenyloxycarbonyl, C₁-C₆-alkylaminocarbonyl, phenyl-C₁-C₆-alkylaminocarbonyl, phenylaminocarbonyl, alkanoyl, phenyl-C₁-C₆-alkanoyl, phenylcarbonyl, alkenoyl, alkynoyl or -R₆-NR'R'' where R₆ is C₁-C₆-alkylene, C₂-C₆-alkenylene or C₂-C₆-alkynylene and R' and R'' are each independently C₁-C₆-alkyl or alternatively the group -NR'R'' as a whole is 1-pyrrolidinyl, 1-piperidinyl, 4-morpholinyl, 4-C₁-C₆-alkyl-1-piperazinyl or 4-phenyl-1-piperazinyl, the term phenyl signifying a phenyl group which may be substituted as indicated above, or each R₂ is independently phenyl, nitrophenyl, cyanophenyl, trifluoromethyl, aminophenyl or C₁-C₆-alkanoylaminophenyl, wherein the phenyl group is unsubstituted, and R₃ is hydrogen, nitro, amino halogen, C₁-C₆-alkanoylamino, phenyl-C₁-C₆-alkanoylamino, phenylcarbonylamino, alkylamino, phenyl-C₁-C₆-alkylamino or C₁-C₆-alkyl, the term phenyl signifying a phenyl group which may be substituted as indicated above; or a pharmaceutically acceptable acid addition salt thereof.

2. A compound as defined in claim 1, where m is 1, n is 1 and p is zero.

3. A compound as defined in claim 2, where R is hydrogen, C₁-C₆-alkyl or C₁-C₆-alkoxy, R₁ is hydrogen, C₁-C₆-alkyl, C₂-C₆-alkenyl or formyl, R₂ is hydrogen, C₁-C₆-alkyl or phenyl-C₁-C₆-alkyl, the term phenyl signifying a phenyl group which may be substituted as indicated above; and R₃ is hydrogen, halogen, nitro or amino.

4. The compound as defined in claim 1, which is N-methyl-N-(4-pyridinyl)-1H-indol-1-amine or a pharmaceutically acceptable acid addition salt thereof.

5. The compound as defined in claim 1, which is N-(4-pyridinyl)-1H-indol-1-amine or a pharmaceutically acceptable acid addition salt thereof.

6. The compound as defined in claim 1, which is N-propyl-N-(4-pyridinyl)-1H-indol-1-amine, or a pharmaceutically acceptable acid addition salt thereof.

7. The compound as defined in claim 1, which is 3-ethyl-N-methyl-N-(4-pyridinyl)-1H-indol-1-amine, or a pharmaceutically acceptable acid addition salt thereof.

8. The compound as defined in claim 1, which is 3-ethenyl-N-methyl-N-(4-pyridinyl)-1H-indol-1-amine, or a pharmaceutically acceptable acid addition salt thereof.

9. A process for the preparation of a compound as defined in claim 1, which comprises
a) reacting a compound of the formula II 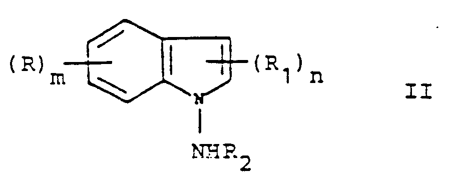 where m is 1 or 2 and n is 1 or 2, R is hydrogen, halogen, C₁-C₆-alkyl, C₁-C₆-alkoxy, nrtro, cyano, formyl, C₁-C₆-alkylthio or C₁-C₆-alkoxycarbonyl-C₁-C₆-alkylthio, R₁ is hydrogen, C₁-C₆-alkyl, halogen or cyano and R₂ is hydrogen or C₁-C₆-alkyl, with a compound of the formula III 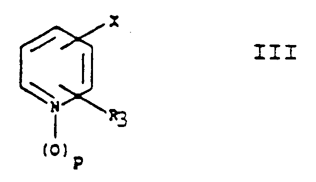 where X is chlorine or fluorine, R₃ is hydrogen, nitro, halogen or C₁-C₆-alkyl and p is 0 or 1, to afford a compound of the formula I, where R, R₁, R₂ and R₃ are as defined,
b) optionally treating a compound of the formula I as obtained in step a) where R₂ is hydrogen, with a strong base and reacting the obtained anion with a compound of the formula R₂Cl, where R₂ is C₁-C₆-alkyl, C₁-C₆-alkoxycarbonyl-C₁-C₆-alkyl, C₂-C₆-alkenyl, C₂-C₆-alkynyl, C₁-C₆-alkoxycarbonyl, phenyl-C₁-C₆-alkyl, phenyl-C₁-C₆-alkoxycarbonyl or phenyloxycarbonyl wherein the phenyl group may be substituted as indicated in claim 1, to afford a compound of the formula I, where m, n, p, R, R₁, R₃ are as defined in step a) and R₂ is as defined above,
c) optionally treating a compound of the formula I as obtained in step a) where R₂ is hydrogen, with a strong base and reacting the obtained anion with a compound of the formula 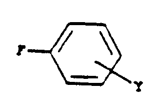 where Y is nitro, cyano or trifluoromethyl, to afford a compound of the formula I, where m, n, p, R, R₁ and R₃ are as defined in step a) and R₂ is nitrophenyl, cyanophenyl or trifluoromethylphenyl,
d) optionally reacting a compound of the formula I as obtained in step a), where R₂ is hydrogen, with a compound of the formula R₁₂NCO where R₁₂ is C₁-C₆-alkyl, phenyl-C₁-C₆-alkyl or phenyl wherein the phenyl group may be substituted as indicated in claim 1, to afford a compound of the formula I, where m, n, p, R, R₁ and R₃ are as defined in step a) and R₂ is C₁-C₆-alkylaminocarbonyl, phenyl-C₁-C₆-alkylaminocarbonyl or phenylaminocarbonyl wherein the phenyl group may be substituted as indicated in claim 1,
e) optionally reacting a compound of the formula I as obtained in step a) where R₂ is hydrogen, with a compound of the formula R₁₃COCl where R₁₃ is alkyl, phenyl-C₁-C₆-alkyl, phenyl, wherein the phenyl group may be substituted as indicated in claim 1, alkenyl or alkynyl to afford a compound of the formula I where m, n, p, R, R₁ and R₃ are as defined in step a) and R₂ is alkanoyl, phenyl-C₁-C₆-alkanoyl, phenylcarbonyl wherein the phenyl group may be substituted as indicated in claim 1, alkenoyl or alkynoyl,
f) optionally reacting a compound of the formula I as obtained in step a) where R₂ is hydrogen with a strong base and then reacting the product with a di-C₁-C₆-alkylsulfate to afford a compound of the formula I where m, n, p, R, R₁ and R₃ are as defined in step a) and R₂ is C₁-C₆-alkyl,
g) obtionally subjecting a compound of the formula I as obtained in step b) where R₂ is -CH₂C≡CH to a Mannich reaction with formaldehyd and a secondary amine of the formula HNR'R'', where R' and R'' are independently C₁-C₆-alkyl or R' and R'' taken together are 1-pyrrolidinyl, 1-piperidinyl, 1-morpholinyl, 4-C₁-C₆-alkyl-1-piperazinyl or 4-phenyl-l-piperazinyl wherein the phenyl group may be substituted as indicated in claim 1, to afford a compound of the formula I where m, n, p, R, R₁ and R₃ are as defined in step b) and R₂ is the group -CH₂C≡CCH₂NR'R'' where R' and R'' are as defined above,
h) optionally hydrogenating a compound of the formula I as obtained in step g) to afford a compound of the formula I where m, n, p, R, R₁ and R₃ are as defined in step a) and R₂ is the group -CH₂CH=CHCH₂NR'R'' or -(CH₂)₄NR'R'' where R' and R'' are as defined in step g),
i) optionally reacting a compound of the formula I where m, n, p, R and R₃ are as defined in step a), R₁ is hydrogen and R₂ is C₁-C₆-alkyl, C₁-C₆alkoxycarbonyl-C₁-C₆-alkyl, alkanoyl, C₁-C₆-alkoxycarbonyl, phenyl-C₁-C₆-alkyl, phenyl, phenyl-C₁-C₆-alkoxycarbonyl, phenyloxycarbonyl, phenyl-C₁-C₆-alkanoyl or phenylcarbonyl wherein the phenyl group may be substituted as indicated in claim 1, with a suitable chlorinating agent to afford the 3-chloro-derivative of a compound of the formula I where m, n, p, R, R₁ and R₃ are as defined above,
j) optionally reacting a compound of the formula I where m, n, p and R are as defined in step a), R₁ is hydrogen, R₂ is C₁-C₆-alkyl, C₁-C₆-alkoxycarbonyl, alkanoyl, alkenoyl, alkynoyl, phenyl-C₁-C₆-alkanoyl or phenylcarbonyl wherein the phenyl group may be substituted as indicated in claim 1, and R₃ is hydrogen, nitro, halogen or C₁-C₆-alkyl, either with phosphorus oxychloride and dimethylformaide to afford a compound of the formula I where R₁ is the formyl group at 3-position, or with sec. butyllithium and reacting the resulting lithio compound with N-formyl-N-methyl-aniline to afford a compound of the formula I where R₁ is the formyl group at 2-position,
k) optionally reacting a compound of the formula I where m, n, p and R are as defined in step a), R₁ is hydrogen, R₂ is C₁-C₆-alkyl, C₁-C₆-alkoxycarbonyl, alkanoyl, alkenoyl, alkynoyl, phenyl-C₁-C₆-alkanoyl or phenylcarbonyl wherein the phenyl group may be substituted as indicated in claim 1, and R₃ is hydrogen, nitro, halogen or C₁-C₆-alkyl, either with a C₁-C₆-alkanoyl-phenyl-C₁-C₆-alkanoyl- or phenylcarbonyl chloride of fomula R₁₂COCl wherein the phenyl group may be substituted as indicated in claim 1, in the presence of zinc chloride to afford a compound of the formula I where R₁ is C₁-C₆-alkanoyl, phenyl-C₁-C₆-alkanoyl or phenylcarbonyl, wherein the phenyl group may be substituted as defined in claim 1, or with sec-butyllithium and reacting the resultant lithio compound with R₁₂COCl to afford a compound of the formula I where the substitutent R₁ is in 2-position,
l) optionally reducing a compound of the formula I where m, n, p and R are as defined in step a), R₁ is C₁-C₆-alkanoyl, phenyl-C₁-C₆-alkanoyl or phenylcarbonyl, R₂ is C₁-C₆-alkyl, phenyl-C₁-C₆-alkyl or phenyl wherein the phenyl group may be substituted as indicated in claim 1, and R₃ is hydrogen, halogen or C₁-C₆-alkyl, either with NaBH₄, LiAlH₄ or borane complexes to afford a compound of the formula I where R₁ is the group -CH(OH)R₄ where R₄ is hydrogen, C₁-C₆-alkyl, phenyl-C₁-C₆-alkyl or phenyl, wherein the phenyl group may be substituted as indicated in claim 1, or with a Grignard reagent of the formula R₅MgBr to afford a compound of the formula I, where R₁ is the group 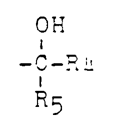 wherein R₅ is C₁-C₆-alkyl, phenyl-C₁-C₆-alkyl or phenyl,
wherein the phenyl group may be substituted as indicated in claim 1,
m) optionally hydrogenating a compound of the formula I, where m, n, p, R, R₁ and R₂ are as defined in formula I above, and R₃ is the nitro group, in the presence of a catalyst, to afford a compound of the formula I, where R₃ is the amino group,
n) optionally hydrolyzing a compound of the formula I, where m, n, p, R, R₁ are as defined in formula I above, R₃ is as defined in step a) and R₂ is the C₁-C₆-alkoxycarbonyl group, to afford a compound of the formula I where R₂ is hydrogen,
o) optionally reacting a compound of the formula I, where m, n, p, R, R₁ and R₂ are as defined in formula I above, and R₃ is the amino group, with phenyl formate, to afford a compound of the formula I, where R₃ is the formylamino group,
p) optionally reacting a compound of the formula I, where m, n, p, R, R₁ and R₂ are as defined in formula I above, and R₃ is the amino group, with an acyl chloride of the formula R₁₂COCl or acid anhydride of the formula R₁₂CO-O-COR₁₂, where R₁₂ is C₁-C₆-alkyl, phenyl-C₁-C₆-alkyl or phenyl, to afford a compound of the formula I where R₃ is C₁-C₆-alkanoylamino, phenyl-C₁-C₆-alkanoylamino or phenylcarbonylamino, wherein the phenyl group may be substituted as indicated in claim 1,
q) optionally reacting a compound of the formula I, where m, n, p, R, R₁ and R₂ are as defined in formula I above and R₃ is hydrogen with C₁-C₆-alkyl lithium to afford a compound of the formula I where R₃ is C₁-C₆-alkyl,
r) optionally reacting a compound of the formula I, where m, n, p and R are as defined in formula I above, R₂ is C₁-C₆-alkyl, phenyl-C₁-C₆-alkyl or phenyl, wherein the phenyl group may be substituted as indicated in claim 1, R₃ is hydrogen, halogen or C₁-C₆-alkyl and R₁ is the group -CH₂OH, with a strong alkali metal base and reacting the resultant alkoxide ion with a C₁-C₆-alkyl halide, phenyl-C₁-C₆-alkyl halide or phenyl halide of the formula R₅X wherein the phenyl group may be substituted as indicated in claim 1, to afford a compound of the formula I, where R₁ is the group -CH₂OR₅,
s) optionally subjecting a compound of the formula I, where m, n, p and R are as defined in step a), R₁ is -CHO, R₂ is C₁-C₆-alkyl, C₁-C₆-alkoxycarbonyl, alkanoyl, alkenoyl, alkynoyl, phenyl-C₁-C₆-alkanoyl or phenylcarbonyl, wherein the phenyl group may be substituted as indicated in claim 1, and R₃ is hydrogen, NO₂, halogen or C₁-C₆-alkyl, to a Wittig reaction with an ylide of the formula (C₆H₅)₃P=CR₂₀R₂₁, where R₂₀ and R₂₁ are each independently hydrogen, C₁-C₆-alkyl, phenyl or phenyl-C₁-C₆-alkyl, wherein the phenyl group may be substituted as indicated in claim 1, heteroaryl or heteroaryl-C₁-C₆-alkyl wherein the term heteroaryl has the meaning as indicated in claim 1, cyano, methoxy, C₁-C₆-alkoxycarbonyl or, taken together, form a C₃-C₇-cycloalkylidene, to afford a compound of the formula I where R₁ is the group -CH=CR₂₀R₂₁.
t) optionally catalytically hydrogenating a compound of the formula I as obtained in step s), to afford a compound of the formula I where R₁ is C₁-C₆-alkyl, phenyl-C₁-C₆-alkyl wherein the phenyl group may be substituted as indicated in claim 1, heteroaryl-C₁-C₆-alkyl wherein the term heteroaryl has the meaning as indicated in claim 1, cyano-C₁-C₆-alkyl, methoxy-C₁-C₆-alkyl, C₁-C₆-alkoxycarbonyl-C₁-C₆-alkyl or C₃-C₇-cycloalkyl-C₁-C₆-alkyl,
u) optionally catalytically hydrogenating a compound of the formula I where m, n, p, R, R₁ and R₃ are as defined in formula I above, and R₂ is nitrophenyl, to afford a compound of the formula I where R₂ is aninophenyl,
v) optionally reacting a compound of the formula I as obtained in step u) with phenyl formate to afford a compound of the formula I where R₂ is formylaminophenyl,
w) optionally reacting a compound of the formula I as obtained in step u) with an acyl chloride of the formula R₁₂COCl or acid anhydride of the formula R₁₂-CO-O-COR₁₂ where R₁₂ is C₁-C₆-alkyl, to afford a compound of the formula I where R₂ is C₁-C₆-alkanoylaminophenyl,
x) optionally reacting a compound of the formula I as obtained in step j) where R₁ is -CHO with hydroxylamine to afford the corresponding oxime and reacting this oxime with benzenesulfonyl chloride to afford a compound of the formula I where R₁ is the radical -CN,
y) optionally reacting a comuound of the formula I, where R₂ is C₁-C₆-alkoxycarbonyl-C₁-C₆-alkyl with ammonia to afford a compound of the formula I where R₂ is the group aminocarbonyl-C₁-C₆-alkyl.

10. A pharmaceutically composition which comprises a compound as defined in claim 1 as the active ingredient and a pharmaceutically acceptable carrier therefor.

11. Use of a compound as defined in claim 1 for the preparation of a medicament having memory enhancing, pain alleviating and/or depression alleviating activities.

## Claims (Claims for the following Contracting State(s): ES, GR)

1. A process for the preparation of a compound of formula I 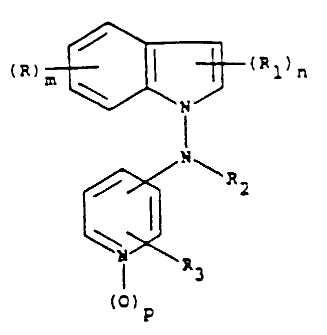 where m is 1 or 2; n is 1 or 2; P is 0̸ or 1; each R is independently hydrogen, halogen, C₁-C₆-alkyl, C₁-C₆-alkoxy, hydroxy, nitro, amino, C₁-C₆-alkylamino, C₁-C₆-alkylcarbonylamino, cyano, formyl, C₁-C₆-alkoxycarbonyl, C₁-C₆-alkylthio or C₁-C₆-alkoxycarbonyl-C₁-C₆-alkylthio; each R₁ is independently hydrogen, C₁-C₆-alkyl, C₂-C₆-alkenyl, formyl, C₁-C₆-alkylcarbonyl, phenyl-C₁-C₆-alkylcarbonyl phenylcarbonyl, halogen, phenyl-C₂-C₆-alkenyl, phenyl-C₁-C₆-alkyl heteroaryl-C₂-C₆-alkenyl, heteroaryl-C₁-C₆-alkyl, cyano-C₂-C₆-alkenyl, cyano-C₁-C₆-alkyl, methoxy-C₂-C₆-alkenyl, methoxy-C₁-C₆-alkyl, C₁-C₆-alkoxycarbonyl-C₂-C₆-alkenyl, C₁-C₆-alkoxycarbonyl-C₁-C₆-alkyl, C₃-C₇-cycloalkyl-C₂-C₆-alkenyl or C₃-C₇-cycloalkyl-C₁-C₆-alkyl, the term phenyl signifying a phenyl group which may have 1-3 substituents each of which being independently C₁-C₆-alkyl, C₁-C₆-alkoxy, halogen, CF₃, NO₂ or CN, and the term heteroaryl signifying a group
of the formula 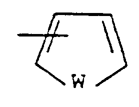 where W is O, S, NH or CH=N, cyano, -CH(OH)R₄, -C(OH)R₄R₅ or -CH₂OR₅, R₄ being hydrogen, C₁-C₆-alkyl, phenyl-C₁-C₆-alkyl or phenyl and R₅ being C₁-C₆-alkyl, phenyl-C₁-C₆-alkyl or phenyl; each R₂ is independently hydrogen, C₁-C₆-alkyl, C₂-C₆-alkenyl, C₂-C₆-alkynyl, C₁-C₆-alkoxycarbonyl-C₁-C₆-alkyl, C₁-C₆-alkylaminocarbonyl-C₁-C₆-alkyl, aminocarbonyl-C₁-C₆-alkyl, phenyl-C₁-C₆-alkyl, C₁-C₆-alkoxycarbonyl phenyl-C₁-C₆-alkoxycarbonyl, phenyloxycarbonyl, C₁-C₆-alkylaminocarbonyl, phenyl-C₁-C₆-alkylaminocarbonyl, phenylaminocarbonyl, alkanoyl, phenyl-C₁-C₆-alkanoyl, phenylcarbonyl, alkenoyl, alkynoyl or -R₆-NR'R'' where R₆ is C₁-C₆-alkylene, C₂-C₆-alkenylene or C₂-C₆-alkynylene and R' and R'' are each independently C₁-C₆-alkyl or alternatively the group -NR'R'' as a whole is 1-pyrrolidinyl, 1-piperidinyl, 4-morpholinyl, 4-C₁-C₆-alkyl-1-piperazinyl or 4-phenyl-1-piperazinyl, the term phenyl signifying a phenyl group which may be substituted as indicated above, or each R₂ is independently phenyl, nitrophenyl, cyanophenyl, trifluoromethyl, aminophenyl or C₁-C₆-alkanoylaminophenyl, wherein the phenyl group is unsubstituted, and R₃ is hydrogen, nitro, amino halogen, C₁-C₆-alkanoylamino, phenyl-C₁-C₆-alkanoylamino, phenylcarbonylamino, alkylamino, phenyl-C₁-C₆-alkylamino or C₁-C₆-alkyl, the term phenyl signifying a phenyl group which may be substituted as indicated above; or a pharmaceutically acceptable acid addition salt thereof, which comprises
a) reacting a compound of the formula II 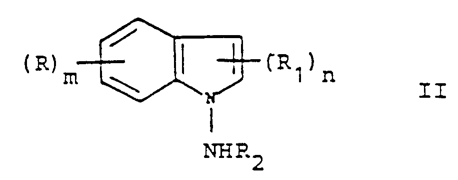 where m is 1 or 2 and n is 1 or 2, R is hydrogen, halogen, C₁-C₆-alkyl, C₁-C₆-alkoxy, nitro, cyano, formyl, C₁-C₆-alkylthio or C₁-C₆-alkoxycarbonyl-C₁-C₆-alkylthio, R₁ is hydrogen, C₁-C₆-alkyl, halogen or cyano and R₂ is hydrogen or C₁-C₆-alkyl, with a compound of the formula III 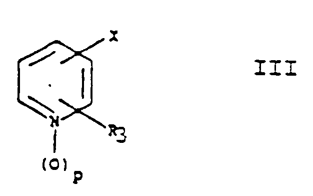 where X is chlorine or fluorine, R₃ is hydrogen, nitro, halogen or loweralkyl and p is 0 or 1, to afford a compound of the formula I, where R, R₁, R₂ and R₃ are as defined,
b) optionally treating a compound of the formula I as obtained in step a) where R₂ is hydrogen, with a strong base and reacting the obtained anion with a compound of the formula R₂Cl, where R₂ is C₁-C₆-alkyl, C₁-C₆-alkoxycarbonyl-C₁-C₆-alkyl, C₂-C₆-alkenyl, C₂-C₆-alkynyl, C₁-C₆-alkoxycarbonyl, phenyl-C₁-C₆-alkyl, phenyl-C₁-C₆-alkoxycarbonyl or phenyloxycarbonyl wherein the phenyl group may be substituted as indicated above, to afford a compound of the formula I, where m, n, p, R, R₁, R₃ are as defined in step a) and R₂ is as defined above,
c) optionally treating a compound of the formula I as obtained in step a) where R₂ is hydrogen, with a strong base and reacting the obtained anion with a compound of the formula 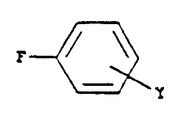 where Y is nitro, cyano or trifluoromethyl, to afford a compound of the formula I, where m, n, p, R, R₁ and R₃ are as defined in step a) and R₂ is nitrophenyl, cyanophenyl or trifluoromethylphenyl,
d) optionally reacting a compound of the formula I as obtained in step a), where R₂ is hydrogen, with a compound of the formula R₁₂NCO where R₁₂ is C₁-C₆-alkyl, phenyl-C₁-C₆-alkyl or phenyl wherein the phenyl group may be substituted as indicated above, to afford a compound of the formula I, where m, n, p, R, R₁ and R₃ are as defined in step a) and R₂ is C₁-C₆-alkylaminocarbonyl, phenyl-C₁-C₆-alkylaminocarbonyl or phenylaminocarbonyl wherein the phenyl group may be substituted as indicated above,
e) optionally reacting a compound of the formula I as obtained in step a) where R₂ is hydrogen, with a compound of the formula R₁₃COCl where R₁₃ is alkyl, phenyl-C₁-C₆-alkyl phenyl, wherein the phenyl group may be substituted as indicated above alkenyl or alkynyl to afford a compound of the formula I where m, n, p, R, R₁ and R₃ are as defined in step a) and R₂ is alkanoyl, phenyl-C₁-C₆-alkanoyl, phenylcarbonyl wherein the phenyl group may be substituted as indicated above, alkenoyl or alkynoyl,
f) optionally reacting a compound of the formula I as obtained in step a) where R₂ is hydrogen with a strong base and then reacting the product with a di-C₁-C₆-alkylsulfate to afford a compound of the formula I where m, n, p, R, R₁ and R₃ are as defined in step a) and R₂ is C₁-C₆-alkyl,
g) obtionally subjecting a compound of the formula I as obtained in step b) where R₂ is -CH₂C=CH to a Mannich reaction with formaldehyd and a secondary amine of the formula HNR'R'', where R' and R'' are independently C₁-C₆-alkyl or R' and R'' taken together are 1-pyrrolidinyl, 1-piperidinyl, 1-morpholinyl, 4-C₁-C₆-alkyl-1-piperazinyl or 4-phenyl-l-piperazinyl wherein the phenyl group may be substituted as indicated above, to afford a compound of the formula I where m, n, p, R, R₁ and R₃ are as defined in step b) and R₂ is the group - CH₂C≡CCH₂NR'R'' where R' and R'' are as defined above,
h) optionally hydrogenating a compound of the formula I as obtained in step g) to afford a compound of the formula I where m, n, p, R, R₁ and R₃ are as defined in step a) and R₂ is the group -CH₂CH=CHCH₂NR'R'' or -(CH₂)₄NR'R'' where R' and R'' are as defined in step g),
i) optionally reacting a compound of the formula I where m, n, p, R and R₃ are as defined in step a), R₁ is hydrogen and R₂ is C₁-C₆-alkyl, C₁-C₆alkoxycarbonyl-C₁-C₆-alkyl, alkanoyl, C₁-C₆-alkoxycarbonyl, phenyl-C₁-C₆-alkyl, phenyl, phenyl-C₁-C₆-alkoxycarbonyl, phenyloxycarbonyl, phenyl-C₁-C₆-alkanoyl or phenylcarbonyl wherein the phenyl group may be substituted as indicated above, with a suitable chlorinating agent to afford the 3-chloro-derivative of a compound of the formula I where m, n, p, R, R₁ and R₃ are as defined above,
j) optionally reacting a compound of the formula I where m, n, p and R are as defined in step a), R₁ is hydrogen, R₂ is C₁-C₆-alkyl, C₁-C₆-alkoxycarbonyl, alkanoyl, alkenoyl, alkynoyl, phenyl-C₁-C₆-alkanoyl or phenylcarbonyl wherein the phenyl group may be substituted as indicated above, and R₃ is hydrogen, nitro, halogen or C₁-C₆-alkyl, either with phosphorus oxychloride and dimethylformaide to afford a compound of the formula I where R₁ is the formyl group at 3-position, or with sec. butyllithium and reacting the resulting lithio compound with N-formyl-N-methyl-aniline to afford a compound of the formula I where R₁ is the formyl group at 2-position,
k) optionally reacting a compound of the formula I where m, n, p and R are as defined in step a), R₁ is hydrogen, R₂ is C₁-C₆-alkyl, C₁-C₆-alkoxycarbonyl, alkanoyl, alkenoyl, alkynoyl, phenyl-C₁-C₆-alkanoyl or phenylcarbonyl wherein the phenyl group may be substituted as indicated above, and R₃ is hydrogen, nitro, halogen or C₁-C₆-alkyl, either with a C₁-C₆-alkanoyl-phenyl-C₁-C₆-alkanoyl- or phenylcarbonyl chloride of fomula R₁₂COCl wherein the phenyl group may besubstituted as indicated above, in the presence of zinc chloride to afford a compound of the formula I where R₁ is C₁-C₆-alkanoyl, phenyl-C₁-C₆-alkanoyl or phenylcarbonyl, wherein the phenyl group may be substituted as defined above, or with sec-butyllithium and reacting the resultant lithio compound with R₁₂COCl to afford a compound of the formula I where the substitutent R₁ is in 2-position,
l) optionally reducing a compound of the formula I where m, n, p and R are as defined in step a), R₁ is C₁-C₆-alkanoyl, phenyl-C₁-C₆-alkanoyl or phenylcarbonyl, R₂ is C₁-C₆-alkyl, phenyl-C₁-C₆-alkyl or phenyl wherein the phenyl group may be substituted as indicated above, and R₃ is hydrogen, halogen or C₁-C₆-alkyl, either with NaBH₄, LiAlH₄ or borane complexes to afford a compound of the formula I where R₁ is the group -CH(OH)R₄ where R₄ is hydrogen, C₁-C₆-alkyl, phenyl-C₁-C₆-alkyl or phenyl, wherein the phenyl group may be substituted as indicated above, or witha Grignard reagent of the formula R₅MgBr to afford a compound of the formula I, where R₁ is the group 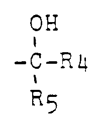 wherein R₅ is C₁-C₆-alkyl, phenyl-C₁-C₆-alkyl or phenyl, wherein the phenyl group may be substituted as indicated above,
m) optionally hydrogenating a compound of the formula I, where m, n, p, R, R₁ and R₂ are as defined in formula I above, and R₃ is the nitro group, in the presence of a catalyst, to afford a compound of the formula I, where R₃ is the amino group,
n) optionally hydrolyzing a compound of the formula I, where m, n, p, R, R₁ are as defined in formula I above, R₃ is as defined in step a) and R₂ is the C₁-C₆-alkoxycarbonyl group, to afford a compound of the formula I where R₂ is hydrogen,
o) optionally reacting a compound of the formula I, where m, n, p, R, R₁ and R₂ are as defined in formula I above, and R₃ is the amino group, with phenyl formate, to afford a compound of the formula I, where R₃ is the formylamino group,
p) optionally reacting a compound of the formula I, where m, n, p, R, R₁ and R₂ are as defined in formula I above, and R₃ is the amino group, with an acyl chloride of the formula R₁₂COCl or acid anhydride of the formula R₁₂CO-O-COR₁₂, where R₁₂ is C₁-C₆-alkyl, phenyl-C₁-C₆-alkyl or phenyl, to afford a compound of the formula I where R₃ is C₁-C₆-alkanoylamino, phenyl-C₁-C₆-alkanoylamino or phenylcarbonylamino, wherein the phenyl group may be substituted as indicated above,
q) optionally reacting a compound of the formula I, where m, n, p, R, R₁ and R₂ are as defined in formula I above and R₃ is hydrogen with C₁-C₆-alkyl lithium to afford a compound of the formula I where R₃ is C₁-C₆-alkyl,
r) optionally reacting a compound of the formula I, where m, n, p and R are as defined in formula I above, R₂ is C₁-C₆-alkyl, phenyl-C₁-C₆-alkyl or phenyl, wherein the phenyl group may be substituted as indicated above, R₃ is hydrogen, halogen or C₁-C₆-alkyl and R₁ is the group -CH₂OH, with a strong alkali metal base and reacting the resultant alkoxide ion with a C₁-C₆-alkyl halide, phenyl-C₁-C₆-alkyl halide or phenyl halide of the formula R₅X wherein the phenyl group may be substituted as indicated above, to afford a compound of the formula I, where R₁ is the group -CH₂OR₅,
s) optionally subjecting a compound of the formula I, where m, n, p and R are as defined in step a), R₁ is -CHO, R₂ is C₁-C₆-alkyl, C₁-C₆-alkoxycarbonyl, alkanoyl, alkenoyl, alkynoyl, phenyl-C₁-C₆-alkanoyl or phenylcarbonyl, wherein the phenyl group may be substituted as indicated above, and R₃ is hydrogen, NO₂, halogen or C₁-C₆-alkyl, to a Wittig reaction with an ylide of the formula (C₆H₅)₃P=CR₂₀R₂₁, where R₂₀ and R₂₁ are each independently hydrogen, C₁-C₆-alkyl, phenyl or phenyl-C₁-C₆-alkyl, wherein the phenyl group may be substituted as indicated above, heteroaryl or heteroaryl-C₁-C₆-alkyl wherein the term heteroaryl has the meaning as indicated above, cyano, methoxy, C₁-C₆-alkoxycarbonyl or, taken together, form a C₃-C₇-cycloalkylidene, to afford a compound of the formula I where R₁ is the group -CH=CR₂₀R₂₁.
t) optionally catalytically hydrogenating a compound of the formula I as obtained in step s), to afford a compound of the formula I where R₁ is C₁-C₆-alkyl, phenyl-C₁-C₆-alkyl wherein the phenyl group may be substituted as indicated above, heteroaryl-C₁-C₆-alkyl wherein the term heteroaryl has the meaning as indicated above, cyano-C₁-C₆-alkyl, methoxy-C₁-C₆-alkyl, C₁-C₆-alkoxycarbonyl-C₁-C₆-alkyl or C₃-C₇-cycloalkyl-C₁-C₆-alkyl,
u) optionally catalytically hydrogenating a compound of the formula I where m, n, p, R, R₁ and R₃ are as defined in formula I above, and R₂ is nitrophenyl, to afford a compound of the formula I where R₂ is aminophenyl,
v) optionally reacting a compound of the formula I as obtained in step u) with phenyl formate to afford a compound of the formula I where R₂ is formylaminophenyl,
w) optionally reacting a compound of the formula I as obtained in step u) with an acyl chloride of the formula R₁₂COCl or acid anhydride of the formula R₁₂-CO-O-COR₁₂ where R₁₂ is C₁-C₆-alkyl, to afford a compound of the formula I where R₂ is C₁-C₆-alkanoylaminophenyl,
x) optionally reacting a compound of the formula I as obtained in step j) where R₁ is -CHO with hydroxylamine to afford the corresponding oxime and reacting this oxime with benzenesulfonyl chloride to afford a compound of the formula I where R₁ is the radical -CN,
y) optionally reacting a compound of the formula I, where R₂ is C₁-C₆-alkoxycarbonyl-C₁-C₆-alkyl with ammonia to afford a compound of the formula I where R₂ is the group aminocarbonyl-C₁-C₆-alkyl.

2. A process as defined in claim 1 where m is 1, n is 1 and p is zero.

3. A process as defined in claim 2, where R is hydrogen, C₁-C₆-alkyl or C₁-C₆-alkoxy, R₁ is hydrogen, C₁-C₆-alkyl, C₂-C₆-alkenyl or formyl, R₂ is hydrogen, C₁-C₆-alkyl or phenyl-C₁-C₆-alkyl, wherein the phenyl group may be substituted as indicated in claim 1 and R₃ is hydrogen, halogen, nitro or amino.

4. A process as defined in claim 1, where the compound N-methyl-N-(4-pyridinyl)-1H-indol-1-amine or a pharmaceutically acceptable acid addition salt thereof is prepared.

5. A process as defined in claim 1, where the compound N-(4-pyridinyl)-1H-indol-1-amine or a pharmaceutically acceptable acid addition salt thereof is prepared.

6. A process as defined in claim 1, where the compound N-propyl-N-(4-pyridinyl)-1H-indol-1-amine, or a pharmaceutically acceptable acid addition salt thereof is prepared.

7. A process as defined in claim 1, where the compound 3-ethyl-N-methyl-N-(4-pyridinyl)-1H-indol-1-amine, or a pharmaceutically acceptable acid addition salt thereof is prepared.

8. A process as defined in claim 1, where the compound 3-ethenyl-N-methyl-N-(4-pyridinyl)-1H-indol-1-amine or a pharmaceutically acceptable addition salt thereof is prepared.

9. Use of a compound as defined in claim 1 for the preparation of a medicament having memory enhancing, pain alleviating and/or pain alleviating activities.

## Patentansprüche (Patentansprüche für folgende(n) Vertragsstaat(en): AT, BE, CH, DE, FR, GB, IT, LI, LU, NL, SE)

1. Eine Verbindung mit der Formel 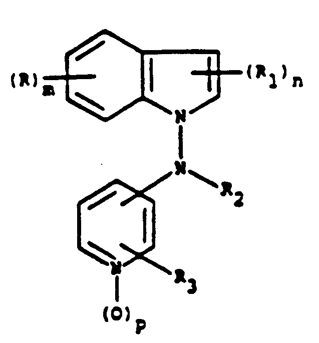 worin m den Wert 1 oder 2 aufweist; n den Wert 1 oder 2 hat; p den Wert 0 oder 1 aufweist; jeder Rest R unabhängig voneinander Wasserstoff, Halogen, C₁-C₆-Alkyl, C₁-C₆-Alkoxy, Hydroxy, Nitro, Amino, C₁-C₆-Alkylamino, C₁-C₆-Alkylcarbonylamino,Cyano,Formyl,C₁-C₆-Alkoxycarbonyl, C₁-C₆-Alkylthio oder C₁-C₆-Alkoxycarbonyl-C₁-C₆-alkylthio bedeutet; jeder Rest R₁ unabhängig voneinander Wasserstoff,
C₁-C₆-Alkyl, C₂-C₆-Alkenyl, Formyl, C₁-C₆-Alkylcarbonyl, Phenyl-C₁-C₆-alkylcarbonyl, Phenylcarbonyl, Halogen, Phenyl-C₂-C₆-alkenyl,Phenyl-C₁-C₆-alkyl, Heteroaryl-C₂-C₆-alkenyl, Heteroaryl-C₁-C₆-alkyl, Cyano-C₂-C₆-alkenyl, Cyano-C₁-C₆-alkyl, Methoxy-C₂-C₆-alkenyl, Methoxy-C₁-C₆-alkyl, C₁-C₆-Alkoxycarbonyl-C₂-C₆-alkenyl, C₁-C₆-Alkoxycarbonyl-C₁-C₆-alkyl,C₃-C₇-Cycloalkyl-C₂-C₆-alkenyl oder C₃-C₇-Cycloalkyl-C₁-C₆-alkyl darstellt, wobei der Ausdruck "Phenyl" eine Phenylgruppe bezeichnet, die 1 bis 3 Substituenten aufweisen kann, die jeweils unabhängig voneinander C₁-C₆-Alkyl, C₁-C₆-Alkoxy, Halogen, CF₃, NO₂ oder CN bedeuten, und worin der Ausdruck "Heteroaryl" eine Gruppe mit der Formel 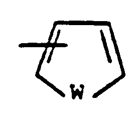 bezeichnet, worin W für O, S, NH oder CH=N steht, oder R₁ Cyano,-CH(OH)R₄,-C(OH)R₄R₅ oder -CH₂OR₅ darstellt, wobei R₄ Wasserstoff, C₁-C₆-Alkyl, Phenyl-C₁-C₆-alkyl oder Phenyl bedeutet und R₅ für C₁-C₆-Alkyl, Phenyl-C₁-C₆-alkyl oder Phenyl steht; worin jeder Rest R₂ unabhängig für Wasserstoff, C₁-C₆-Alkyl, C₂-C₆-Alkenyl, C₂-C₆-Alkinyl, C₁-C₆-Alkoxycarbonyl-C₁-C₆-alkyl,C₁-C₆-Alkylaminocarbonyl-C₁-C₆-alkyl,Aminocarbonyl-C₁-C₆-alkyl,Phenyl-C₁-C₆-alkyl, C₁-C₆-Alkoxycarbonyl, Phenyl-C₁-C₆-alkoxycarbonyl, Phenyloxycarbonyl, C₁-C₆-Alkylaminocarbonyl, Phenyl-C₁-C₆-alkylaminocarbonyl, Phenylaminocarbonyl, Alkanoyl, Phenyl-C₁-C₆-alkanoyl, Phenylcarbonyl, Alkenoyl, Alkinoyl oder -R₆-NR'R'' steht, worin R₆ C₁-C₆-Alkylen, C₂-C₆-Alkenylen oder C₂-C₆-Alkinylen darstellt und R' und R'' jeweils unabhängig voneinander C₁-C₆-Alkyl bedeuten oder in alternativer Weise die Gruppe -NR'R'' als Ganzes 1-Pyrrolidinyl, 1-Piperidinyl,4-Morpholinyl, 4-C₁-C₆-Alkyl-1-piperazinyl oder 4-Phenyl-1-piperazinyl darstellt, wobei der Ausdruck "Phenyl" eine Phenylgruppe bezeichnet, die wie oben angegeben substituiert sein kann, oder worin jeder Rest R₂ unabhängig voneinander Phenyl, Nitrophenyl, Cyanophenyl, Trifluormethyl, Aminophenyl oder C₁-C₆-Alkanoylaminophenyl bedeutet, worin die Phenylgruppe unsubstituiert ist, und R₃ für Wasserstoff, Nitro, Amino, Halogen, C₁-C₆-Alkanoylamino, Phenyl-C₁-C₆-alkanoylamino, Phenylcarbonylamino, Alkylamino, Phenyl-C₁-C₆-alkylamino oder C₁-C₆-Alkyl steht, wobei der Ausdruck "Phenyl" eine Phenylgruppe bezeichnet, die wie oben angegeben substituiert sein kann; oder ein pharmazeutisch annehmbares Säureadditionssalz hievon.

2. Verbindung nach Anspruch 1, worin m den Wert 1 hat, n den Wert 1 hat und p den Wert 0 aufweist.

3. Verbindung nach Anspruch 2, worin R Wasserstoff, C₁-C₆-Alkyl oder C₁-C₆-Alkoxy bedeutet, R₁ für Wasserstoff, C₁-C₆-Alkyl, C₂-C₆-Alkenyl oder Formyl steht, R₂ Wasserstoff, C₁-C₆-Alkyl oder Phenyl-C₁-C₆-alkyl darstellt, wobei der Ausdruck "Phenyl" eine Phenylgruppe bezeichnet, die wie oben angegeben substituiert sein kann; und R₃ Wasserstoff, Halogen, Nitro oder Amino bedeutet.

4. Verbindung nach Anspruch 1, nämlich N-Methyl-N-(4-pyridinyl)-1H-indol-1-amin oder ein pharmazeutisch annehmbares Säureadditionssalz hievon.

5. Verbindung nach Anspruch 1, nämlich N-(4-Pyridinyl)-1H-indol-1-amin oder ein pharmazeutisch annehmbares Säureadditionssalz hievon.

6. Verbindung nach Anspruch 1, nämlich N-Propyl-N-(4-pyridinyl)-1H-indol-1-amin oder ein pharmazeutisch annehmbares Säureadditionssalz hievon.

7. Verbindung nach Anspruch 1, nämlich 3-Ethyl-N-methyl-N-(4-pyridinyl)-1H-indol-1-amin oder ein pharmazeutisch annehmbares Säureadditionssalz hievon.

8. Verbindung nach Anspruch 1, nämlich 3-Ethenyl-N-methyl-N-(4-pyridinyl)-1H-indol-1-amin oder ein pharmazeutisch annehmbares Säureadditionssalz heivon.

9. Verfahren zur Herstellung einer Verbindung nach Anspruch 1, welches umfaßt:
a) Umsetzen einer Verbindung der Formel II 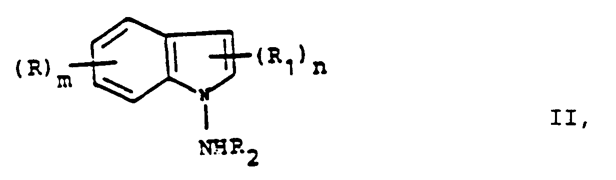 worin m den Wert 1 oder 2 aufweist und n den Wert 1 oder 2 hat, R für Wasserstoff, Halogen, C₁-C₆-Alkyl, C₁-C₆-Alkoxy, Nitro, Cyano, Formyl, C₁-C₆-Alkylthio oder C₁-C₆-Alkoxycarbonyl-C₁-C₆-alkylthio steht, R₁ Wasserstoff, C₁-C₆-Alkyl, Halogen oder Cyano bedeutet und R₂ Wasserstoff oder C₁-C₆-Alkyl darstellt, mit einer Verbindung der Formel III 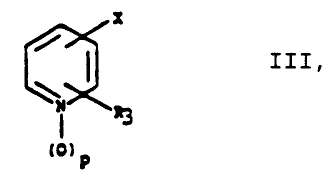 worin X Chlor oder Fluor ist, R₃ Wasserstoff, Nitro, Halogen oder C₁-C₆-Alkyl bedeutet und p den Wert 0 oder 1 aufweist, zur Ausbildung einer Verbindung der Formel I, worin R, R₁, R₂ und R₃ wie definiert sind,
b) gewünschtenfalls Behandeln einer Verbindung der Formel I, wie in Stufe a) erhalten, worin R₂ Wasserstoff bedeutet, mit einer starken Base und Umsetzen des erhaltenen Anions mit einer Verbindung der Formel R₂Cl, worin R₂ für C₁-C₆-Alkyl, C₁-C₆-Alkoxycarbonyl-C₁-C₆-alkyl, C₂-C₆-Alkenyl, C₂-C₆-Alkinyl, C₁-C₆-Alkoxycarbonyl, Phenyl-C₁-C₆-alkyl, Phenyl-C₁-C₆-alkoxycarbonyl oder Phenyloxycarbonyl steht, worin die Phenylgruppe wie in Anspruch 1 angegeben substituiert sein kann, zur Ausbildung einer Verbindung der Formel I, worin m, n, p, R, R₁, R₃ wie in Stufe a) definiert sind und R₂ wie oben definiert ist,
c) gewünschtenfalls Behandeln einer Verbindung der Formel I, wie in Stufe a) erhalten, worin R₂ für Wasserstoff steht, mit einer starken Base und Umsetzen des erhaltenen Anions mit einer Verbindung der Formel 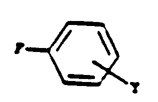 worin Y für Nitro, Cyano oder Trifluormethyl steht, zur Ausbildung einer Verbindung der Formel I, worin m, n, p, R, R₁ und R₃ wie in Stufe a) definiert sind und R₂ Nitrophenyl, Cyanophenyl oder Trifluormethylphenyl bedeutet,
d) gewünschtenfalls Umsetzen einer Verbindung der Formel I, wie in Stufe a) erhalten, worin R₂ Wasserstoff bedeutet, mit einer Verbindung der Formel R₁₂NCO, worin R₁₂ für C₁-C₆-Alkyl, Phenyl-C₁-C₆-alkyl oder Phenyl steht, worin die Phenylgruppe wie in Anspruch 1 angegeben substituiert sein kann, zur Ausbildung einer Verbindung der Formel I, worin m, n, p, R, R₁ und R₃ wie in Stufe a) definiert sind und R₂ für C₁-C₆-Alkylaminocarbonyl,Phenyl-C₁-C₆-alkylaminocarbonyl oder Phenylaminocarbonyl steht, worin die Phenylgruppe wie in Anspruch 1 angegeben substituiert sein kann,
e) gewünschtenfalls Umsetzen einer Verbindung der Formel I, wie in Stufe a) erhalten, worin R₂ für Wasserstoff steht, mit einer Verbindung der Formel R₁₃COCl, worin R₁₃ für Alkyl, Phenyl-C₁-C₆-alkyl, Phenyl, worin die Phenylgruppe wie in Anspruch 1 angegeben substituiert sein kann, für Alkenyl oder Alkinyl steht, zur Ausbildung der Verbindung der Formel I, worin m, n, p, R, R₁ und R₃ wie in Stufe a) definiert sind und R₂ Alkanoyl, Phenyl-C₁-C₆-alkanoyl, Phenylcarbonyl, worin die Phenylgruppe wie in Anspruch 1 angegeben substituiert sein kann, Alkenoyl oder Alkinoyl darstellt,
f) gewünschtenfalls Umsetzen einer Verbindung der Formel I, wie in Stufe a) erhalten, worin R₂ Wasserstoff bedeutet, mit einer starken Base und anschließendes Umsetzen des Produktes mit einem Di-C₁-C₆-alkylsulfat zur Ausbildung einer Verbindung der Formel I, worin m, n, p, R, R₁ und R₃ wie in Stufe a) definiert sind und R₂ C₁-C₆-Alkyl bedeutet,
g) gewünschtenfalls Ausführen einer Mannich-Reaktion an einer Verbindung der Formel I, wie in Stufe b) erhalten, worin R₂ für -CH₂=CH steht, mit Formaldehyd und einem sekundären Amin mit der Formel HHR'R'', worin R' und R'' unabhängig voneinander C₁-C₆-Alkyl bedeuten oder R' und R'' zusammen 1-Pyrrolidinyl, 1-Piperidinyl,1-Morpholinyl,4-C₁-C₆-Alkyl-1-piperazinyl oder 4-Phenyl-1-piperazinyl, worin die Phenylgruppe wie in Anspruch 1 angegeben substituiert sein kann, darstellen, zur Ausbildung einer Verbindung der Formel I, worin m, n, p, R, R₁ und R₃ wie in Stufe b) definiert sind und R₂ die Gruppe -CH₂C≡CCH₂NR'R'' bedeutet, worin R' und R'' wie vorstehend definiert sind,
h) gewünschtenfalls Hydrieren einer Verbindung der Formel I, wie in Stufe g) erhalten, zur Ausbildung einer Verbindung der Formel I, worin m, n, p, R, R₁ und R₃ wie in Stufe a) definiert sind und R₂ die Gruppe -CH₂CH=CHCH₂NR'R'' oder -(CH₂)₄NR'R'' bedeutet, worin R' und R'' wie in Stufe g) definiert sind,
i) gewünschtenfalls Umsetzen einer Verbindung der Formel I, worin m, n, p, R und R₃ wie in Stufe a) definiert sind,R₁ Wasserstoff darstellt und R₂ für C₁-C₆-Alkyl, C₁-C₆-Alkoxycarbonyl-C₁-C₆-alkyl,Alkanoyl, C₁-C₆-Alkoxycarbonyl,Phenyl-C₁-C₆-alkyl, Phenyl, Phenyl-C₁-C₆-alkoxycarbonyl, Phenyloxycarbonyl, Phenyl-C₁-C₆-alkanoyl oder Phenylcarbonyl steht, worin die Phenylgruppe wie in Anspruch 1 angegeben substituiert sein kann, mit einem geeigneten Chlorierungsmittel zur Ausbildung des 3-Chlorderivats einer Verbindung der Formel I, worin m, n, p, R, R₁ und R₃ wie vorstehend definiert sind,
j) gegebenenfalls Umsetzen einer Verbindung der Formel I, worin m, n, p und R wie in Stufe a) definiert sind, R₁ Wasserstoff darstellt, R₂ für C₁-C₆-Alkyl, C₁-C₆-Alkoxycarbonyl, Alkanoyl, Alkenoyl, Alkinoyl, Phenyl-C₁-C₆-alkanoyl oder Phenylcarbonyl steht, worin die Phenylgruppe wie in Anspruch 1 angegeben substituiert sein kann, und R₃ Wasserstoff, Nitro, Halogen oder C₁-C₆-Alkyl darstellt, entweder mit Phosphoroxychlorid und Dimethylformamid zur Ausbildung einer Verbindung der Formel I, worin R₁ die Formylgruppe in der 3-Stellung darstellt, oder mit sec.Butyllithium und Umsetzen der gebildeten Lithiumverbindung mit N-Formyl-N-methyl-anilin zur Ausbildung einer Verbindung der Formel I, worin R₁ die Formylgruppe in der 2-Stellung bezeichnet,
k) gewünschtenfalls Umsetzen einer Verbindung der Formel I, worin m, n, p und R wie in Stufe a) definiert sind, R₁ Wasserstoff darstellt, R₂ für C₁-C₆-Alkyl, C₁-C₆-Alkoxycarbonyl, Alkanoyl, Alkenoyl, Alkinoyl, Phenyl-C₁-C₆-alkanoyl oder Phenylcarbonyl steht, worin die Phenylgruppe wie in Anspruch 1 angegeben substituiert sein kann, und R₃ Wasserstoff, Nitro, Halogen oder C₁-C₆-Alkyl bedeutet, entweder mit einem C₁-C₆-Alkanoyl-, Phenyl-C₁-C₆-alkanoyl- oder Phenylcarbonylchlorid der Formel R₁₂COCl, worin die Phenylgruppe wie in Anspruch 1 angegeben substituiert sein kann, in Anwesenheit von Zinkchlorid zur Ausbildung einer Verbindung der Formel I, worin R₁ für C₁-C₆-Alkanoyl, Phenyl-C₁-C₆-alkanoyl oder Phenylcarbonyl steht, worin die Phenylgruppe wie in Anspruch 1 definiert substituiert sein kann, oder mit sec.Butyllithium und Umsetzen der gebildeten Lithiumverbindung mit R₁₂COCl zur Ausbildung einer Verbindung der Formel I, worin der Substituent R₁ in der 2-Stellung vorliegt,
l) gewünschtenfalls Reduzieren einer Verbindung der Formel I, worin m, n, p und R wie in Stufe a) definiert sind, R₁ für C₁-C₆-Alkanoyl, Phenyl-C₁-C₆-alkanoyl oder Phenylcarbonyl steht, R₂ die Bedeutung C₁-C₆-Alkyl, Phenyl-C₁-C₆-alkyl oder Phenyl, worin die Phenylgruppe wie in Anspruch 1 angegeben substituiert sein kann, aufweist und R₃ Wasserstoff, Halogen oder C₁-C₆-Alkyl darstellt, entweder mit NaBH₄, LiAlH₄ oder Borankomplexen zur Ausbildung einer Verbindung der Formel I, worin R₁ die Gruppe -CH(OH)R₄ darstellt, worin R₄ Wasserstoff, C₁-C₆-Alkyl, Phenyl-C₁-C₆-alkyl oder Phenyl bedeutet, worin die Phenylgruppe wie in Anspruch 1 angegeben substituiert sein kann, oder mit einem Grignard-Reagens mit der Formel R₅MgBr zur Ausbildung einer Verbindung der Formel I, worin R₁ die Gruppe 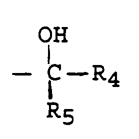 bedeutet, worin R₅ für C₁-C₆-Alkyl, Phenyl-C₁-C₆-alkyl oder Phenyl steht, worin die Phenylgruppe wie in Anspruch 1 angegeben substituiert sein kann,
m) gewünschtenfalls Hydrieren einer Verbindung der Formel I, worin m, n, p, R, R₁ und R₂ wie in der vorstehenden Formel I definiert sind und R₃ die Nitrogruppe darstellt, in Anwesenheit eines Katalysators zur Ausbildung einer Verbindung mit der Formel I, worin R₃ die Aminogruppe bezeichnet,
n) gewünschtenfalls Hydrolysieren einer Verbindung der Formel I, worin m, n, p, R, R₁ wie in der obigen Formel I definiert sind, R₃ wie in Stufe a) definiert ist und R₂ die C₁-C₆-Alkoxycarbonylgruppe darstellt, zur Ausbildung einer Verbindung der Formel I, worin R₂ Wasserstoff bedeutet,
o) gewünschtenfalls Umsetzen einer Verbindung der Formel I, worin m, n, p, R, R₁ und R₂ wie in der obigen Formel I definiert sind und R₃ die Aminogruppe bezeichnet, mit Phenylformiat zur Ausbildung einer Verbindung der Formel I, worin R₃ die Formylaminogruppe darstellt,
p) gewünschtenfalls Umsetzen einer Verbindung der Formel I, worin m, n, p, R, R₁ und R₂ wie in der obigen Formel I definiert sind und R₃ die Aminogruppe bezeichnet, mit einem Acylchlorid der Formel R₁₂COCl oder einem Säureanhydrid der Formel R₁₂CO-O-COR₁₂, worin R₁₂ für C₁-C₆-Alkyl, Phenyl-C₁-C₆-alkyl oder Phenyl steht, zur Ausbildung einer Verbindung der Formel I, worin R₃ für C₁-C₆-Alkanoylamino, Phenyl-C₁-C₆-alkanoylamino oder Phenylcarbonylamino steht, worin die Phenylgruppe wie in Anspruch 1 angegeben substituiert sein kann,
q) gewünschtenfalls Umsetzen einer Verbindung der Formel I, worin m, n, p, R, R₁ und R₂ wie in der obigen Formel I definiert sind und R₃ Wasserstoff bezeichnet, mit C₁-C₆-Alkyllithium zur Ausbildung einer Verbindung der Formel I, worin R₃ C₁-C₆-Alkyl bedeutet,
r) gewünschtenfalls Umsetzen einer Verbindung der Formel I, worin m, n, p und R wie in der obigen Formel I definiert sind, R₂ für C₁-C₆-Alkyl, Pheny-C₁-C₆-alkyl oder Phenyl steht, worin die Phenylgruppe wie in Anspruch 1 angegeben substituiert sein kann, R₃ Wasserstoff, Halogen oder C₁-C₆-Alkyl bedeutet und R₁ die Gruppe -CH₂OH darstellt, mit einer starken Alkalimetallbase und Umsetzen des gebildeten Alkoxidions mit einem C₁-C₆-Alkylhalogenid, Phenyl-C₁-C₆-alkylhalogenid oder Phenylhalogenid der Formel R₅X, worin die Phenylgruppe wie in Anspruch 1 angegeben substituiert sein kann, zur Ausbildung einer Verbindung der Formel I, worin R₁ die Gruppe -CH₂OR₅ bezeichnet,
s) gewünschtenfalls Vornahme einer Wittig-Reaktion an einer Verbindung der Formel I, worin m, n, p und R wie in Stufe a) definiert sind, R₁ für -CHO steht, R₂ für C₁-C₆-Alkyl, C₁-C₆-Alkoxycarbonyl, Alkanoyl, Alkenoyl, Alkinoyl, Phenyl-C₁-C₆-alkanoyl oder Phenylcarbonyl steht, worin die Phenylgruppe wie in Anspruch 1 angegeben substituiert sein kann, und R₃ Wasserstoff, NO₂, Halogen oder C₁-C₆-Alkyl darstellt, mit einem Ylid der Formel (C₆H₅)₃P=CR₂₀R₂₁, worin R₂₀ und R₂₁ jeweils unabhängig voneinander Wasserstoff, C₁-C₆-Alkyl, Phenyl oder Phenyl-C₁-C₆-alkyl, worin die Phenylgruppe wie in Anspruch 1 angegeben substituiert sein kann, Heteroaryl oder Heteroaryl-C₁-C₆-alkyl, worin der Ausdruck "Heteroaryl" die in Anspruch 1 angegebene Bedeutung aufweist, Cyano, Methoxy, C₁-C₆-Alkoxycarbonyl bedeuten oder zusammengenommen einen C₃-C₇-Cycloalkylidenrest ergeben, zur Ausbildung einer Verbindung der Formel I, worin R₁ die Gruppe -CH=CR₂₀R₂₁ darstellt,
t) gewünschtenfalls katalytisches Hydrieren einer Verbindung der Formel I, wie in Stufe s) erhalten, zur Ausbildung einer Verbindung der Formel I, worin R₁ für C₁-C₆-Alkyl, Phenyl-C₁-C₆-alkyl, worin die Phenylgruppe wie in Anspruch 1 angegeben substituiert sein kann, Heteroaryl-C₁-C₆-alkyl, worin der Ausdruck "Heteroaryl" die in Anspruch 1 angegebene Bedeutung aufweist,Cyano-C₁-C₆-alkyl,Methoxy-C₁-C₆-alkyl,C₁-C₆-Alkoxycarbonyl-C₁-C₆-alkyl oder C₃-C₇-Cycloalkyl-C₁-C₆-alkyl steht,
u) gewünschtenfalls katalytisches Hydrieren einer Verbindung der Formel I, worin m, n, p, R, R₁ und R₃ wie in der vorstehenden Formel I definiert sind und R₂ Nitrophenyl bedeutet, zur Ausbildung einer Verbindung der Formel I, worin R₂ Aminophenyl bedeutet,
v) gewünschtenfalls Umsetzen einer Verbindung der Formel I, wie in Stufe u) erhalten, mit Phenylformiat zur Ausbildung einer Verbindung der Formel I, worin R₂ Formylaminophenyl bezeichnet,
w) gewünschtenfalls Umsetzen einer Verbindung der Formel I, wie in Stufe u) erhalten, mit einem Acylchlorid der Formel R₁₂COCl oder einem Säureanhydrid der Formel R₁₂-CO-O-COR₁₂, worin R₁₂ für C₁-C₆-Alkyl steht, zur Ausbildung einer Verbindung der Formel I, worin R₂ die Bedeutung C₁-C₆-Alkanoylaminophenyl aufweist,
x) gewünschtenfalls Umsetzen einer Verbindung der Formel I, wie in Stufe j) erhalten, worin R₁ für -CHO steht, mit Hydroxylamin zur Ausbildung des entsprechenden Oxims und Umsetzen dieses Oxims mit Benzolsulfonylchlorid zur Ausbildung einer Verbindung der Formel I, worin R₁ den Rest -CN darstellt,
y) gewünschtenfalls Umsetzen einer Verbindung der Formel I, worin R₂ für C₁-C₆-Alkoxycarbonyl-C₁-C₆-alkyl steht, mit Ammoniak zur Ausbildung einer Verbindung der Formel I,worin R₂ die Gruppe Aminocarbonyl-C₁-C₆-alkyl darstellt.

10. Pharmazeutsiche Zusammensetzung, die ein Verbindung nach Anspruch 1 als aktiven Bestandteil und einen pharmazeutisch annehmbaren Träger hiefür umfaßt.

11. Verwendung einer Verbindung nach Anspruch 1 zur Herstellung eines Medikaments mit gedächtnisfordernder, schmerzlindernder und/oder Depressionen erleichternder Wirkung.

## Patentansprüche (Patentansprüche für folgende(n) Vertragsstaat(en): ES, GR)

1. Verfahren zur Herstellung einer Verbindung mit der Formel I 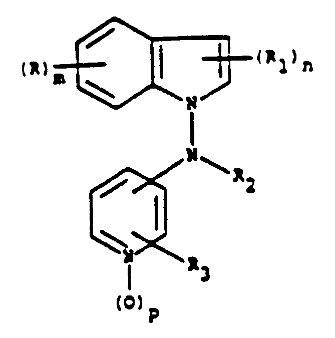 worin m den Wert 1 oder 2 aufweist; n den Wert 1 oder 2 hat; p den Wert 0 oder 1 aufweist; jeder Rest R unabhängig voneinander Wasserstoff, Halogen, C₁-C₆-Alkyl, C₁-C₆-Alkoxy, Hydroxy, Nitro, Amino, C₁-C₆-Alkylamino, C₁-C₆-Alkylcarbonylamino,Cyano,Formyl,C₁-C₆-Alkoxycarbonyl, C₁-C₆-Alkylthio oder C₁-C₆-Alkoxycarbonyl-C₁-C₆-alkylthio bedeutet; jeder Rest R₁ unabhängig voneinander Wasserstoff, C₁-C₆-Alkyl, C₂-C₆-Alkenyl, Formyl, C₁-C₆-Alkylcarbonyl, Phenyl-C₁-C₆-alkylcarbonyl, Phenylcarbonyl, Halogen, Phenyl-C₂-C₆-alkenyl,Phenyl-C₁-C₆-alkyl, Heteroaryl-C₂-C₆-alkenyl, Heteroaryl-C₁-C₆-alkyl, Cyano-C₂-C₆-alkenyl, Cyano-C₁-C₆-alkyl, Methoxy-C₂-C₆-alkenyl, Methoxy-C₁-C₆-alkyl, C₁-C₆-Alkoxycarbonyl-C₂-C₆-alkenyl, C₁-C₆-Alkoxycarbonyl-C₁-C₆-alkyl,C₃-C₇-Cycloalkyl-C₂-C₆-alkenyl oder C₃-C₇-Cycloalkyl-C₁-C₆-alkyl darstellt, wobei der Ausdruck "Phenyl" eine Phenylgruppe bezeichnet, die 1 bis 3 Substituenten aufweisen kann, die jeweils unabhängig voneinander C₁-C₆-Alkyl, C₁-C₆-Alkoxy, Halogen, CF₃, NO₂ oder CN bedeuten, und worin der Ausdruck "Heteroaryl" eine Gruppe mit der Formel 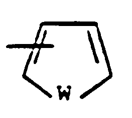 bezeichnet, worin W für O, S, NH oder CH=N steht, oder R₁ Cyano,-CH(OH)R₄,-C(OH)R₄R₅ oder -CH₂OR₅ darstellt,wobei R₄ Wasserstoff, C₁-C₆-Alkyl, Phenyl-C₁-C₆-alkyl oder Phenyl bedeutet und R₅ für C₁-C₆-Alkyl, Phenyl-C₁-C₆-alkyl oder Phenyl steht; worin jeder Rest R₂ unabhängig für Wasserstoff, C₁-C₆-Alkyl, C₂-C₆-Alkenyl, C₂-C₆-Alkinyl, C₁-C₆-Alkoxycarbonyl-C₁-C₆-alkyl, C₁-C₆-Alkylaminocarbonyl-C₁-C₆-alkyl,Aminocarbonyl-C₁-C₆-alkyl,Phenyl-C₁-C₆-alkyl, C₁-C₆-Alkoxycarbonyl, Phenyl-C₁-C₆-alkoxycarbonyl, Phenyloxycarbonyl, C₁-C₆-Alkylaminocarbonyl, Phenyl-C₁-C₆-alkylaminocarbonyl, Phenylaminocarbonyl, Alkanoyl, Phenyl-C₁-C₆-alkanoyl, Phenylcarbonyl, Alkenoyl, Alkinoyl oder -R₆-NR'R'' steht, worin R₆ C₁-C₆-Alkylen, C₂-C₆-Alkenylen oder C₂-C₆-Alkinylen darstellt und R' und R'' jeweils unabhängig voneinander C₁-C₆-Alkyl bedeuten oder in alternativer Weise die Gruppe -NR'R'' als Ganzes 1-Pyrrolidinyl, 1-Piperidinyl,4-Morpholinyl, 4-C₁-C₆-Alkyl-1-piperazinyl oder 4-Phenyl-1-piperazinyl darstellt, wobei der Ausdruck "Phenyl" eine Phenylgruppe bezeichnet, die wie oben angegeben substituiert sein kann, oder worin jeder Rest R₂ unabhängig voneinander Phenyl, Nitrophenyl, Cyanophenyl, Trifluormethyl, Aminophenyl oder C₁-C₆-Alkanoylaminophenyl bedeutet, worin die Phenylgruppe unsubstituiert ist, und R₃ für Wasserstoff, Nitro, Amino, Halogen, C₁-C₆-Alkanoylamino, Phenyl-C₁-C₆-alkanoylamino, Phenylcarbonylamino, Alkylamino, Phenyl-C₁-C₆-alkylamino oder C₁-C₆-Alkyl steht, wobei der Ausdruck "Phenyl" eine Phenylgruppe bezeichnet, die wie oben angegeben substituiert sein kann; oder eines pharmazeutisch annehmbaren Säureadditionssalzes hievon, welches umfaßt:
a) Umsetzen einer Verbindung der Formel II 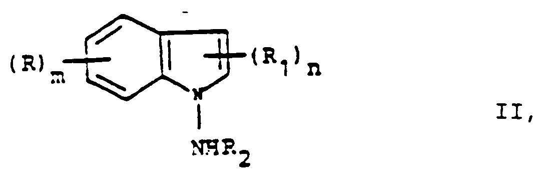 worin m den Wert 1 oder 2 aufweist und n den Wert 1 oder 2 hat, R für Wasserstoff, Halogen, C₁-C₆-Alkyl, C₁-C₆-Alkoxy, Nitro, Cyano, Formyl, C₁-C₆-Alkylthio oder C₁-C₆-Alkoxycarbonyl-C₁-C₆-alkylthio steht, R₁ Wasserstoff, C₁-C₆-Alkyl, Halogen oder Cyano bedeutet und R₂ Wasserstoff oder C₁-C₆-Alkyl darstellt, mit einer Verbindung der Formel III 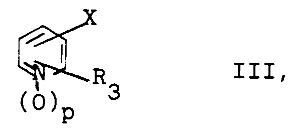 worin X Chlor oder Fluor ist, R₃ Wasserstoff, Nitro, Halogen oder C₁-C₆-Alkyl bedeutet und p den Wert 0 oder 1 aufweist, zur Ausbildung einer Verbindung der Formel I, worin R, R₁, R₂ und R₃ wie definiert sind,
b) gewünschtenfalls Behandeln einer Verbindung der Formel I, wie in Stufe a) erhalten, worin R₂ Wasserstoff bedeutet, mit einer starken Base und Umsetzen des erhaltenen Anions mit einer Verbindung der Formel R₂Cl, worin R₂ für C₁-C₆-Alkyl, C₁-C₆-Alkoxycarbonyl-C₁-C₆-alkyl, C₂-C₆-Alkenyl, C₂-C₆-Alkinyl, C₁-C₆-Alkoxycarbonyl, Phenyl-C₁-C₆-alkyl, Phenyl-C₁-C₆-alkoxycarbonyl oder Phenyloxycarbonyl steht, worin die Phenylgruppe wie oben angegeben substituiert sein kann, zur Ausbildung einer Verbindung der Formel I, worin m, n, p, R, R₁, R₃ wie in Stufe a) definiert sind und R₂ wie oben definiert ist,
c) gewünschtenfalls Behandeln einer Verbindung der Formel I, wie in Stufe a) erhalten, worin R₂ für Wasserstoff steht, mit einer starken Base und Umsetzen des erhaltenen Anions mit einer Verbindung der Formel 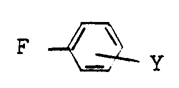 worin Y für Nitro, Cyano oder Trifluormethyl steht, zur Ausbildung einer Verbindung der Formel I, worin m, n, p, R, R₁ und R₃ wie in Stufe a) definiert sind und R₂ Nitrophenyl, Cyanophenyl oder Trifluormethylphenyl bedeutet,
d) gewünschtenfalls Umsetzen einer Verbindung der Formel I, wie in Stufe a) erhalten, worin R₂ Wasserstoff bedeutet, mit einer Verbindung der Formel R₁₂NCO, worin R₁₂ für C₁-C₆-Alkyl, Phenyl-C₁-C₆-alkyl oder Phenyl steht, worin die Phenylgruppe wie oben angegeben substituiert sein kann, zur Ausbildung einer Verbindung der Formel I, worin m, n, p, R, R₁ und R₃ wie in Stufe a) definiert sind und R₂ für C₁-C₆-Alkylaminocarbonyl,Phenyl-C₁-C₆-alkylaminocarbonyl oder Phenylaminocarbonyl steht, worin die Phenylgruppe wie oben angegeben substituiert sein kann,
e) gewünschtenfalls Umsetzen einer Verbindung der Formel I, wie in Stufe a) erhalten, worin R₂ für Wasserstoff steht, mit einer Verbindung der Formel R₁₃COCl, worin R₁₃ für Alkyl, Phenyl-C₁-C₆-alkyl, Phenyl, worin die Phenylgruppe wie oben angegeben substituiert sein kann, für Alkenyl oder Alkinyl steht, zur Ausbildung der Verbindung der Formel I, worin m, n, p, R, R₁ und R₃ wie in Stufe a) definiert sind und R₂ Alkanoyl, Phenyl-C₁-C₆-alkanoyl, Phenylcarbonyl, worin die Phenylgruppe wie oben angegeben substituiert sein kann, Alkenoyl oder Alkinoyl darstellt,
f) gewünschtenfalls Umsetzen einer Verbindung der Formel I, wie in Stufe a) erhalten, worin R₂ Wasserstoff bedeutet, mit einer starken Base und anschließendes Umsetzen des Produktes mit einem Di-C₁-C₆-alkylsulfat zur Ausbildung einer Verbindung der Formel I, worin m, n, p, R, R₁ und R₃ wie in Stufe a) definiert sind und R₂ C₁-C₆-Alkyl bedeutet,
g) gewünschtenfalls Ausführen einer Mannich-Reaktion an einer Verbindung der Formel I, wie in Stufe b) erhalten, worin R₂ für -CH₂=CH steht, mit Formaldehyd und einem sekundären Amin mit der Formel HNR'R'', worin R' und R'' unabhängig voneinander C₁-C₆-Alkyl bedeuten oder R' und R'' zusammen 1-Pyrrolidinyl, 1-Piperidinyl,1-Morpholinyl,4-C₁-C₆-Alkyl-1-piperazinyl oder 4-Phenyl-1-piperazinyl, worin die Phenylgruppe wie oben angegeben substituiert sein kann, darstellen, zur Ausbildung einer Verbindung der Formel I, worin m, n, p, R, R₁ und R₃ wie in Stufe b) definiert sind und R₂ die Gruppe -CH₂C≡CCH₂NR'R'' bedeutet,worin R' und R'' wie vorstehend definiert sind,
h) gewünschtenfalls Hydrieren einer Verbindung der Formel I, wie in Stufe g) erhalten, zur Ausbildung einer Verbindung der Formel I, worin m, n, p, R, R₁ und R₃ wie in Stufe a) definiert sind und R₂ die Gruppe -CH₂CH=CHCH₂NR'R'' oder -(CH₂)₄NR'R'' bedeutet, worin R' und R'' wie in Stufe g) definiert sind,
i) gewünschtenfalls Umsetzen einer Verbindung der Formel I, worin m, n, p, R und R₃ wie in Stufe a) definiert sind,R₁ Wasserstoff darstellt und R₂ für C₁-C₆-Alkyl, C₁-C₆-Alkoxycarbonyl-C₁-C₆-alkyl,Alkanoyl, C₁-C₆-Alkoxycarbonyl,Phenyl-C₁-C₆-alkyl, Phenyl, Phenyl-C₁-C₆-alkoxycarbonyl, Phenyloxycarbonyl, Phenyl-C₁-C₆-alkanoyl oder Phenylcarbonyl steht, worin die Phenylgruppe wie oben angegeben substituiert sein kann, mit einem geeigneten Chlorierungsmittel zur Ausbildung des 3-Chlorderivats einer Verbindung der Formel I, worin m, n, p, R, R₁ und R₃ wie vorstehend definiert sind,
j) gegebenenfalls Umsetzen einer Verbindung der Formel I, worin m, n, p und R wie in Stufe a) definiert sind, R₁ Wasserstoff darstellt, R₂ für C₁-C₆-Alkyl, C₁-C₆-Alkoxycarbonyl, Alkanoyl, Alkenoyl, Alkinoyl, Phenyl-C₁-C₆-alkanoyl oder Phenylcarbonyl steht, worin die Phenylgruppe wie oben angegeben substituiert sein kann, und R₃ Wasserstoff, Nitro, Halogen oder C₁-C₆-Alkyl darstellt, entweder mit Phosphoroxychlorid und Dimethylformamid zur Ausbildung einer Verbindung der Formel I, worin R₁ die Formylgruppe in der 3-Stellung darstellt, oder mit sec.Butyllithium und Umsetzen der gebildeten Lithiumverbindung mit N-Formyl-N-methyl-anilin zur Ausbildung einer Verbindung der Formel I, worin R₁ die Formylgruppe in der 2-Stellung bezeichnet,
k) gewünschtenfalls Umsetzen einer Verbindung der Formel I, worin m, n, p und R wie in Stufe a) definiert sind, R₁ Wasserstoff darstellt, R₂ für C₁-C₆-Alkyl, C₁-C₆-Alkoxycarbonyl, Alkanoyl, Alkenoyl, Alkinoyl, Phenyl-C₁-C₆-alkanoyl oder Phenylcarbonyl steht, worin die Phenylgruppe wie oben angegeben substituiert sein kann, und R₃ Wasserstoff, Nitro, Halogen oder C₁-C₆-Alkyl bedeutet, entweder mit einem C₁-C₆-Alkanoyl-, Phenyl-C₁-C₆-alkanoyl- oder Phenylcarbonylchlorid der Formel R₁₂COCl, worin die Phenylgruppe wie oben angegeben substituiert sein kann, in Anwesenheit von Zinkchlorid zur Ausbildung einer Verbindung der Formel I, worin R₁ für C₁-C₆-Alkanoyl, Phenyl-C₁-C₆-alkanoyl oder Phenylcarbonyl steht, worin die Phenylgruppe wie oben definiert substituiert sein kann, oder mit sec.Butyllithium und Umsetzen der gebildeten Lithiumverbindung mit R₁₂COCl zur Ausbildung einer Verbindung der Formel I, worin der Substituent R₁ in der 2-Stellung vorliegt,
l) gewünschtenfalls Reduzieren einer Verbindung der Formel I, worin m, n, p und R wie in Stufe a) definiert sind, R₁ für C₁-C₆-Alkanoyl, Phenyl-C₁-C₆-alkanoyl oder Phenylcarbonyl steht, R₂ die Bedeutung C₁-C₆-Alkyl, Phenyl-C₁-C₆-alkyl oder Phenyl, worin die Phenylgruppe wie oben angegeben substituiert sein kann, aufweist und R₃ Wasserstoff, Halogen oder C₁-C₆-Alkyl darstellt, entweder mit NaBH₄, LiAlH₄ oder Borankomplexen zur Ausbildung einer Verbindung der Formel I, worin R₁ die Gruppe -CH(OH)R₄ darstellt, worin R₄ Wasserstoff, C₁-C₆-Alkyl, Phenyl-C₁-C₆-alkyl oder Phenyl bedeutet, worin die Phenylgruppe wie oben angegeben substituiert sein kann, oder mit einem Grignard-Reagens mit der Formel R₅MgBr zur Ausbildung einer Verbindung der Formel I, worin R₁ die Gruppe 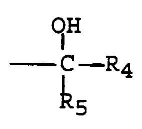 bedeutet, worin R₅ für C₁-C₆-Alkyl, Phenyl-C₁-C₆-alkyl oder Phenyl steht, worin die Phenylgruppe wie in oben angegeben substituiert sein kann,
m) gewünschtenfalls Hydrieren einer Verbindung der Formel I, worin m, n, p, R, R₁ und R₂ wie in der vorstehenden Formel I definiert sind und R₃ die Nitrogruppe darstellt, in Anwesenheit eines Katalysators zur Ausbildung einer Verbindung mit der Formel I, worin R₃ die Aminogruppe bezeichnet,
n) gewünschtenfalls Hydrolysieren einer Verbindung der Formel I, worin m, n, p, R, R₁ wie in der obigen Formel I definiert sind, R₃ wie in Stufe a) definiert ist und R₂ die C₁-C₆-Alkoxycarbonylgruppe darstellt, zur Ausbildung einer Verbindung der Formel I, worin R₂ Wasserstoff bedeutet,
o) gewünschtenfalls Umsetzen einer Verbindung der Formel I, worin m, n, p, R, R₁ und R₂ wie in der obigen Formel I definiert sind und R₃ die Aminogruppe bezeichnet, mit Phenylformiat zur Ausbildung einer Verbindung der Formel I, worin R₃ die Formylaminogruppe darstellt,
p) gewünschtenfalls Umsetzen einer Verbindung der Formel I, worin m, n, p, R, R₁ und R₂ wie in der obigen Formel I definiert sind und R₃ die Aminogruppe bezeichnet, mit einem Acylchlorid der Formel R₁₂COCl oder einem Säureanhydrid der Formel R₁₂CO-O-COR₁₂, worin R₁₂ für C₁-C₆-Alkyl, Phenyl-C₁-C₆-alkyl oder Phenyl steht, zur Ausbildung einer Verbindung der Formel I, worin R₃ für C₁-C₆-Alkanoylamino, Phenyl-C₁-C₆-alkanoylamino oder Phenylcarbonylamino steht, worin die Phenylgruppe wie oben angegeben substituiert sein kann,
q) gewünschtenfalls Umsetzen einer Verbindung der Formel I, worin m, n, p, R, R₁ und R₂ wie in der obigen Formel I definiert sind und R₃ Wasserstoff bezeichnet, mit C₁-C₆-Alkyllithium zur Ausbildung einer Verbindung der Formel I, worin R₃ C₁-C₆-Alkyl bedeutet,
r) gewünschtenfalls Umsetzen einer Verbindung der Formel I, worin m, n, p und R wie in der obigen Formel I definiert sind, R₂ für C₁-C₆-Alkyl, Pheny-C₁-C₆-alkyl oder Phenyl steht, worin die Phenylgruppe wie oben angegeben substituiert sein kann, R₃ Wasserstoff, Halogen oder C₁-C₆-Alkyl bedeutet und R₁ die Gruppe -CH₂OH darstellt, mit einer starken Alkalimetallbase und Umsetzen des gebildeten Alkoxidions mit einem C₁-C₆-Alkylhalogenid, Phenyl-C₁-C₆-alkylhalogenid oder Phenylhalogenid der Formel R₅X, worin die Phenylgruppe wie oben angegeben substituiert sein kann, zur Ausbildung einer Verbindung der Formel I, worin R₁ die Gruppe -CH₂OR₅ bezeichnet,
s) gewünschtenfalls Vornahme einer Wittig-Reaktion an einer Verbindung der Formel I, worin m, n, p und R wie in Stufe a) definiert sind, R₁ für -CHO steht, R₂ für C₁-C₆-Alkyl,C₁-C₆-Alkoxycarbonyl, Alkanoyl, Alkenoyl,Alkinoyl,Phenyl-C₁-C₆-alkanoyl oder Phenylcarbonyl steht, worin die Phenylgruppe wie oben angegeben substituiert sein kann, und R₃ Wasserstoff, NO₂, Halogen oder C₁-C₆-Alkyl darstellt, mit einem Ylid der Formel (C₆H₅)₃P=CR₂₀R₂₁,worin R₂₀ und R₂₁ jeweils unabhängig voneinander Wasserstoff, C₁-C₆-Alkyl, Phenyl oder Phenyl-C₁-C₆-alkyl, worin die Phenylgruppe wie oben angegeben substituiert sein kann, Heteroaryl oder Heteroaryl-C₁-C₆-alkyl, worin der Ausdruck "Heteroaryl" die in Anspruch 1 angegebene Bedeutung aufweist, Cyano, Methoxy, C₁-C₆-Alkoxycarbonyl bedeuten oder zusammengenommen einen C₃-C₇-Cycloalkylidenrest ergeben, zur Ausbildung einer Verbindung der Formel I, worin R₁ die Gruppe -CH=CR₂₀R₂₁ darstellt,
t) gewünschtenfalls katalytisches Hydrieren einer Verbindung der Formel I, wie in Stufe s) erhalten, zur Ausbildung einer Verbindung der Formel I, worin R₁ für C₁-C₆-Alkyl, Phenyl-C₁-C₆-alkyl, worin die Phenylgruppe wie oben angegeben substituiert sein kann, Heteroaryl-C₁-C₆-alkyl, worin der Ausdruck "Heteroryl" die oben angegebene Bedeutung aufweist,Cyano-C₁-C₆-alkyl,Methoxy-C₁-C₆-alkyl,C₁-C₆-Alkoxycarbonyl-C₁-C₆-alkyl oder C₃-C₇-Cycloalkyl-C₁-C₆-alkyl steht,
u) gewünschtenfalls katalytisches Hydrieren einer Verbindung der Formel I, worin m, n, p, R, R₁ und R₃ wie in der vorstehenden Formel I definiert sind und R₂ Nitrophenyl bedeutet, zur Ausbildung einer Verbindung der Formel I, worin R₂ Aminophenyl bedeutet,
v) gewünschtenfalls Umsetzen einer Verbindung der Formel I, wie in Stufe u) erhalten, mit Phenylformiat zur Ausbildung einer Verbindung der Formel I, worin R₂ Formylaminophenyl bezeichnet,
w) gewünschtenfalls Umsetzen einer Verbindung der Formel I, wie in Stufe u) erhalten, mit einem Acylchlorid der Formel R₁₂COCl oder einem Säureanhydrid der Formel R₁₂-CO-O-COR₁₂, worin R₁₂ für C₁-C₆-Alkyl steht, zur Ausbildung einer Verbindung der Formel I, worin R₂ die Bedeutung C₁-C₆-Alkanoylaminophenyl aufweist,
x) gewünschtenfalls Umsetzen einer Verbindung der Formel I, wie in Stufe j) erhalten, worin R₁ für -CHO steht, mit Hydroxylamin zur Ausbildung des entsprechenden Oxims und Umsetzen dieses Oxims mit Benzolsulfonylchlorid zur Ausbildung einer Verbindung der Formel I, worin R₁ den Rest -CN darstellt,
y) gewünschtenfalls Umsetzen einer Verbindung der Formel I, worin R₂ für C₁-C₆-Alkoxycarbonyl-C₁-C₆-alkyl steht, mit Ammoniak zur Ausbildung einer Verbindung der Formel I,worin R₂ die Gruppe Aminocarbonyl-C₁-C₆-alkyl darstellt.

2. Verfahren nach Anspruch worin m den Wert 1 hat, n den Wert 1 hat und p den Wert 0 aufweist.

3. Verfahren nach Anspruch 2, worin R Wasserstoff, C₁-C₆-Alkyl oder C₁-C₆-Alkoxy bedeutet, R₁ für Wasserstoff, C₁-C₆-Alkyl, C₂-C₆-Alkenyl oder Formyl steht, R₂ Wasserstoff, C₁-C₆-Alkyl oder Phenyl-C₁-C₆-alkyl darstellt, wobei der Ausdruck "Phenyl" eine Phenylgruppe bezeichnet, die wie in Anspruch 1 angegeben substituiert sein kann; und R₃ Wasserstoff, Halogen, Nitro oder Amino bedeutet.

4. Verfahren nach Anspruch 1, worin die Verbindung N-Methyl-N-(4-pyridinyl)-1H-indol-1-amin oder ein pharmazeutisch annehmbares Säureadditionssalz hievon hergestellt wird.

5. Verfahren nach Anspruch 1, worin die Verbindung N-(4-Pyridinyl)-1H-indol-1-amin oder ein pharmazeutisch annehmbares Säureadditionssalz hievon hergestellt wird.

6. Verfahren nach Anspruch 1, worin die Verbindung N-Propyl-N-(4-pyridinyl)-1H-indol-1-amin oder ein pharmazeutisch annehmbares Säureadditionssalz hievon hergestellt wird.

7. Verfahren nach Anspruch 1, worin die Verbindung 3-Ethyl-N-methyl-N-(4-pyridinyl)-1H-indol-1-amin oder ein pharmazeutisch annehmbares Säureadditionssalz hievon hergestellt wird.

8. Verfahren nach Anspruch 1, worin die Verbindung 3-Ethenyl-N-methyl-N-(4-pyridinyl)-1H-indol-1-amin oder ein pharmazeutisch annehmbares Säureadditionssalz hievon hergestellt wird.

9. Verwendung einer Verbindung nach Anspruch 1 zur Herstellung eines Medikaments mit gedächtnisfördernder, schmerzlindernder und/oder Depressionen erleichternder Wirkung.

## Revendications (Revendications pour l'(les) Etat(s) contractant(s) suivant(s): AT, BE, CH, DE, FR, GB, IT, LI, LU, NL, SE)

1. Composé de formule 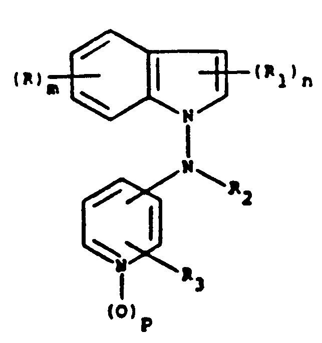 dans laquelle m est 1 ou 2; n est 1 ou 2; p est 0 ou 1; chaque radical R est indépendamment un atome d'hydrogène ou d'halogène, ou un groupe alkyle en C₁-C₆, alcoxy en C₁-C₆, hydroxy, nitro, amino, alkyl(C₁-C₆)-amino, alkyl(C₁-C₆)-carbonylamino, cyano, formyle, alcoxy(C₁-C₆)-carbonyle, alkyl(C₁-C₆)-thio ou alcoxy(C₁-C₆)-carbonyl-alkyl(C₁-C₆)-thio; chaque radical R₁ est indépendamment un atome d'hydrogène ou d'halogène, ou un groupe alkyle en C₁-C₆, alcényle en C₂-C₆, formyle, alkyl(C₁-C₆)-carbonyle, phényl-alkyl(C₁-C₆)-carbonyle, phénylcarbonyle, phényl-alcényle(C₂-C₆), phényl-alkyle(C₁-C₆), hétéroaryl-alcényle(C₂-C₆), hétéroaryl-alkyle(C₁-C₆), cyano-alcényle(C₂-C₆), cyano-alkyle(C₁-C₆), méthoxy-alcényle(C₂-C₆), méthoxy-alkyle(C₁-C₆), alcoxy(C₁-C₆)-carbonyl-alcényle(C₂-C₆), alcoxy(C₁-C₆)-carbonyl-alkyle(C₁-C₆), cycloalkyl(C₃-C₇)-alcényle(C₂-C₆) ou cycloalkyl(C₃-C₇)-alkyle(C₁-C₆), le terme "phényle" signifiant un groupe phényle qui peut comporter 1-3 substituants, chacun d'eux étant indépendamment un atome d'halogène ou un radical alkyle en C₁-C₆, alcoxy en C₁-C₆, CF₃, NO₂ ou CN, et le terme "hétéroaryle" signifiant un groupe de formule 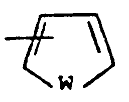 dans laquelle W est O, S, NH ou CH=N, cyano, -CH(OH)R₄, -C(OH)R₄R₅ ou -CH₂OR₅, R₄ étant un atome d'hydrogène ou un radical alkyle en C₁-C₆, phényl-alkyle(C₁-C₆) ou phényle, et R₅ étant un radical alkyle en C₁-C₆, phényl-alkyle(C₁-C₆) ou phényle; chaque radical R₂ est indépendamment un atome d'hydrogène ou un groupe alkyle en C₁-C₆, alcényle en C₂-C₆, alcynyle en C₂-C₆, alcoxy(C₁-C₆)-carbonyl-alkyle(C₁-C₆), alkyl(C₁-C₆)amino-carbonyl-alkyle(C₁-C₆), aminocarbonyl-alkyle(C₁-C₆), phényl-alkyle-(C₁-C₆), alcoxy(C₁-C₆)-carbonyle, phényl-alcoxy(C₁-C₆)-carbonyle, phényloxycarbonyle, alkyl(C₁-C₆)amino-carbonyle, phényl-alkyl(C₁-C₆)amino-carbonyle, phénylaminocarbonyle, alcanoyle, phényl-alcanoyle(C₁-C₆), phénylcarbonyle, alcénoyle, alcynoyle ou -R₆-NR'R'', R₆ étant un groupe alkylène en C₁-C₆, alcénylène en C₂-C₆ ou alcynylène en C₂-C₆, et R' et R'' étant chacun indépendamment un groupe alkyle en C₁-C₆, ou bien le groupe -NR'R'' dans son ensemble étant un radical 1-pyrrolidinyle, 1-pipéridinyle, 4-morpholinyle, 4-alkyl(C₁-C₆)-1-pipérazinyle ou 4-phényl-1-pipérazinyle, le terme "phényle" signifiant un groupe phényle qui peut étre substitué comme indiqué plus haut, ou chaque radical R₂ est indépendamment un groupe phényle, nitrophényle, cyanophényle, trifluorométhyle, aminophényle ou alcanoyl(C₁-C₆)aminophényle, le radical phényle n'étant pas substitué, et R₃ est un atome d'hydrogène ou d'halogène, ou un groupe nitro, amino, alcanoyl(C₁-C₆)-amino, phényl-alcanoyl(C₁-C₆)-amino, phénylcarbonylamino, alkylamino, phényl-alkyl(C₁-C₆)-amino ou alkyle en C₁-C₆, le terme "phényle" signifiant un groupe phényle qui peut être substitué comme indiqué plus haut; ou sel d'addition avec un acide pharmaceutiquement acceptable de celui-ci.

2. Composé selon la revendication 1, dans lequel m est 1, n est 1 et p est zéro.

3. Composé selon la revendication 2, dans lequel R est un atome d'hydrogène ou un groupe alkyle en C₁-C₆ ou alcoxy en C₁-C₆, R₁ est un atome d'hydrogène ou un groupe alkyle en C₁-C₆, alcényle en C₂-C₆ ou formyle, R₂ est un atome d'hydrogène ou un groupe alkyle en C₁-C₆ ou phényl-alkyle(C₁-C₆), le terme "phényle" signifiant un groupe phényle qui peut être substitué comme indiqué plus haut, et R₃ est un atome d'hydrogène ou d'halogène ou le groupe nitro ou amino.

4. Composé selon la revendication 1, qui est la N-méthyl-N-(4-pyridinyl)-1H-indol-1-amine ou un sel pharmaceutiquement acceptable de celle-ci.

5. Composé selon la revendication 1, qui est la N-(4-pyridinyl)-1H-indol-1-amine ou un sel pharmaceutiquement acceptable de celle-ci.

6. Composé selon la revendication 1, qui est la N-propyl-N-(4-pyridinyl)-1H-indol-1-amine ou un sel pharmaceutiquement acceptable de celle-ci.

7. Composé selon la revendication 1, qui est la 3-éthyl-N-méthyl-N-(4-pyridinyl)-1H-indol-1-amine ou un sel pharmaceutiquement acceptable de celle-ci.

8. Composé selon la revendication 1, qui est la 3-éthényl-N-méthyl-N-(4-pyridinyl)-1H-indol-1-amine ou un sel pharmaceutiquement acceptable de celle-ci.

9. Procédé pour la préparation d'un composé selon la revendication 1, comprenant
a) la mise en réaction d'un composé de formule II 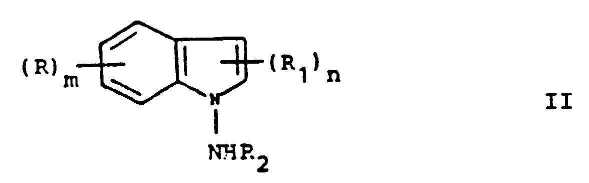 dans laquelle m est 1 ou 2 et n est 1 ou 2, R est un atome d'hydrogène ou d'halogène, ou un groupe alkyle en C₁-C₆, alcoxy en C₁-C₆, nitro, cyano, formyle, alkyl(C₁-C₆)-thio ou alcoxy(C₁-C₆)-carbonyl-alkyl(C₁-C₆)-thio, R₁ est un atome d'hydrogène ou d'halogène, ou un groupe alkyle en C₁-C₆ ou cyano, et R₂ est un atome d'hydrogène ou un groupe alkyle en C₁-C₆,
avec un composé de formule III 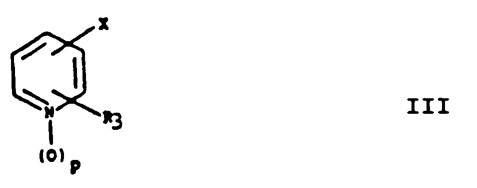 dans laquelle X est un atome de chlore ou de fluor, R₃ est un atome d'hydrogène ou d'halogène, ou un groupe nitro ou alkyle en C₁-C₆, et p est 0 ou 1,
pour l'obtention d'un composé de formule I dans lequel R, R₁, R₂ et R₃ sont tels que définis,
b) éventuellement le traitement par une base forte d'un composé de formule I, tel qu'obtenu dans l'étape a), dans lequel R₂ est un atome d'hydrogéne, et la mise en réaction de l'anion obtenu avec un composé de formule R₂Cl, dans laquelle R₂ est un groupe alkyle en C₁-C₆, alcoxy(C₁-C₆)-carbonyl-alkyle(C₁-C₆), alcényle en C₂-C₆, alcynyle en C₂-C₆, alcoxy(C₁-C₆)-carbonyle, phényl-alkyle(C₁-C₆), phényl-alcoxy(C₁-C₆)-carbonyle ou phényloxycarbonyle, le radical phényle pouvant être substitué comme indiqué dans la revendication 1, pour l'obtention d'un composé de formule I dans lequel m, n, p, R, R₁, R₃ sont tels que définis dans l'étape a) et R₂ est tel que défini ci-dessus,
c) éventuellement le traitement par une base forte d'un composé de formule I tel qu'obtenu dans l'étape a), dans lequel R₂ est un atome d'hydrogène, et la mise en réaction de l'anion obtenu avec un composé de formule 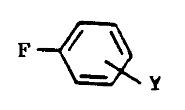 dans laquelle Y est le groupe nitro ou trifluorométhyle, pour l'obtention d'un composé de formule I dans lequel m, n, p, R, R₁ et R₃ sont tels que définis dans l'étape a) et R₂ est le groupe nitrophényle, cyanophényle ou trifluorométhylphényle,
d) éventuellement la mise en réaction d'un composé de formule I, tel qu'obtenu dans l'étape a), dans lequel R₂ est un atome d'hydrogène, avec un composé de formule R₁₂NCO, dans laquelle R₁₂ est un groupe alkyle en C₁-C₆, phénylalkyle(C₁-C₆) ou phényle, le radical phényle pouvant être substitué comme indiqué dans la revendication 1, pour l'obtention d'un composé de formule I dans lequel m, n, p, R, R₁ et R₃ sont tels que définis dans l'étape a), et R₂ est un groupe alkyl(C₁-C₆)amino-carbonyle, phényl-alkyl(C₁-C₆)amino-carbonyle ou phénylaminocarbonyle, le radical phényle pouvant être substitué comme indiqué dans la revendication 1,
e) éventuellement la mise en réaction d'un composé de formule I, tel qu'obtenu dans l'étape a), dans lequel R₂ est un atome d'hydrogène, avec un composé de formule R₁₃COCl, dans laquelle R₁₃ est un groupe alkyle, phényl-alkyle(C₁-C₆), phényle, le radical phényle pouvant être substitué comme indiqué dans la revendication 1, alcényle ou alcynyle, pour l'obtention d'un composé de formule I dans lequel m, n, p, R, R₁ et R₃ sont tels que définis dans l'étape a), et R₂ est un groupe alcanoyle, phényl-alcanoyle(C₁-C₆), phénylcarbonyle, le radical phényle pouvant être substitué comme indiqué dans la revendication 1, alcénoyle ou alcynoyle,
f) éventuellement la mise en réaction avec une base forte d'un composé de formule I, tel qu'obtenu dans l'étape a), dans lequel R₂ est un atome d'hydrogène, et ensuite la mise en réaction du produit avec un sulfate de dialkyle en C₁-C₆, pour l'obtention d'un composé de formule I dans lequel m, n, p, R, R₁ et R₃ sont tels que définis dans l'étape a), et R₂ est un groupe alkyle en C₁-C₆,
g) éventuellement la soumission à une réaction de Mannich d'un composé de formule I, tel qu'obtenu dans l'étape b), dans lequel R₂ est -CH₂C≡CH, avec du formaldéhyde et une amine secondaire de formule HNR'R'', dans laquelle R' et R'' sont indépendamment un groupe alkyle en C₁-C₆, ou R' et R'' forment ensemble un groupe 1-pyrrolidinyle, 1-pipéridinyle, 1-morpholinyle, 4-alkyl(C₁-C₆)-1-pipérazinyle ou 4-phényl-1-pipérazinyle, dans lequel le radical phényle peut être substitué comme indiqué dans la revendication 1, pour l'obtention d'un compose de formule I dans lequel m, n, p, R, R₁ et R₃ sont tels que définis dans l'étape b), et R₂ est le groupe -CH₂C≡CCH₂NR'R'', dans lequel R' et R'' sont tels que définis ci-dessus,
h) éventuellement l'hydrogénation d'un composé de formule I, tel qu'obtenu dans l'étape g), pour l'obtention d'un compose de formule I dans lequel m, n, p, R, R₁ et R₃ sont tels que définis dans l'étape a), et R₂ est le groupe -CH₂CH=CHCH₂NR'R'' ou -(CH₂)₄NR'R'', R' et R'' étant tels que définis dans l'étape g).
i) éventuellement la mise en réaction d'un composé de formule I, dans lequel m, n, p, R et R₃ sont tels que définis dans l'étape a), R₁ est un atome d'hydrogène et R₂ est un groupe alkyle en C₁-C₆, alcoxy(C₁-C₆)-carbonyl-alkyle(C₁-C₆), alcanoyle, alcoxy(C₁-C₆)-carbonyle, phényl-alkyle(C₁-C₆), phényle, phényl-alcoxy(C₁-C₆)-carbonyle, phényloxycarbonyle, phényl-alcanoyle(C₁-C₆) ou phénylcarbonyle, le radical phényle pouvant être substitué comme indiqué dans la revendication 1, avec un agent de chloration approprié, pour l'obtention du dérivé 3-chloro d'un composé de formule I dans lequel m, n, p, R, R₁ et R₃ sont tels que définis plus haut,
j) éventuellement la mise en réaction d'un composé de formule I dans lequel m, n, p et R sont tels que définis dans l'étape a), R₁ est un atome d'hydrogène, R₂ est un groupe alkyle en C₁-C₆, alcoxy(C₁-C₆)-carbonyle, alcanoyle, alcénoyle, alcynoyle, phényl-alcanoyle(C₁-C₆) ou phénylcarbonyle, le radical phényle pouvant être substitué comme indiqué dans la revendication 1, et R₃ est un atome d'hydrogène ou d'halogène, ou un groupe nitro ou alkyle en C₁-C₆, soit avec de l'oxychlorure de phosphore et du diméthyl-formamide, pour l'obtention d'un composé de formule I dans lequel R est le groupe formyle en position 3, soit avec du sec-butyl-lithium et la mise en réaction du composé lithié résultant avec de la N-formyl-N-méthyl-aniline, pour l'obtention d'un composé de formule I dans lequel R₁ est le groupe formyle en position 2,
k) éventuellement la mise en réaction d'un composé de formule I dans lequel m, n, p et R sont tels que définis dans l'étape a), R₁ est un atome d'hydrogène, R₂ est un groupe alkyle en C₁-C₆, alcoxy(C₁-C₆)-carbonyle, alcanoyle, alcénoyle, alcynoyle, phényl-alcanoyle(C₁-C₆) ou phénylcarbonyle, le radical phényle pouvant être substitué comme indiqué dans la revendication 1, et R₃ est un atome d'hydrogène ou d'halogène, ou un groupe nitro ou alkyle en C₁-C₆, soit avec un chlorure d'alcanoyle en C₁-C₆, de phényl-alcanoyle en C₁-C₆ ou de phénylcarbonyle de formule R₁₂COCl, le radical phényle pouvant être substitué comme indiqué dans la revendication 1, en présence de chlorure de zinc, pour l'obtention d'un composé de formule I dans lequel R₁ est un groupe alcanoyle en C₁-C₆, phényl-alcanoyle(C₁-C₆) ou phénylcarbonyle, le radical phényle pouvant être substitué comme indiqué dans la revendication 1, soit avec du sec-butyl-lithium et la mise en réaction du composé lithié résultant avec R₁₂COCl, pour l'obtention d'un composé de formule I dans lequel le substituant R₁ est en position 2,
l) éventuellement la réduction d'un composé de formule I dans lequel m, n, p et R sont tels que définis dans l'étape a), R₁ est un groupe alcanoyle en C₁-C₆, phényl-alcanoyle(C₁-C₆) ou phénylcarbonyle, R₂ est un groupe alkyle en C₁-C₆, phényl-alkyle(C₁-C₆) ou phényle, le radical phényle pouvant être substitué comme indiqué dans la revendication 1, et R₃ est un atome d'hydrogène ou d'halogène, ou un groupe alkyle en C₁-C₆, soit avec NaBH₄, LiAlH₄ ou des complexes de borane, pour l'obtention d'un composé de formule I dans lequel R₁ est le groupe -CH(OH)R₄ dans lequel R₄ est un atome d'hydrogène ou un groupe alkyle en C₁-C₆, phényl-alkyle(C₁-C₆) ou phényle, le radical phényle pouvant être substitué comme indiqué dans la revendication 1, soit avec un réactif de Grignard de formule R₅MgBr, pour l'obtention d'un composé de formule I dans lequel R₁ est un groupe 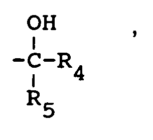 dans lequel R₅ est alkyle en C₁-C₆, phényl-alkyle(C₁-C₆) ou phényle, le radical phényle pouvant être substitué comme indiqué dans la revendication 1,
m) éventuellement l'hydrogénation d'un composé de formule I dans lequel m, n, p, R, R₁ et R₂ sont tels que définis dans la formule I ci-dessus, et R₃ est le groupe nitro, en présence d'un catalyseur, pour l'obtention d'un composé de formule I dans lequel R₃ est le groupe amino,
n) éventuellement l'hydrolyse d'un composé de formule I dans lequel m, n, p, R, R₁ sont tels que définis dans la formule I ci-dessus, R₃ est tel que défini dans l'étape a) et R₂ est un groupe alcoxy(C₁-C₆)-carbonyle, pour l'obtention d'un composé de formule I dans lequel R₂ est un atome d'hydrogène,
o) éventuellement la mise en réaction d'un composé de formule I dans lequel m, n, p, R, R₁ et R₂ sont tels que définis dans la formule I donnée plus haut, et R₃ est le groupe amino, avec du formiate de phényle, pour l'obtention d'un composé de formule I dans lequel R₃ est le groupe formylamino,
p) éventuellement la mise en réaction d'un composé de formule I, dans lequel m, n, p, R, R₁ et R₂ sont tels que définis dans la formule I donnée plus haut, et R₃ est le groupe amino, avec un chlorure d'acyle de formule R₁₂COCl ou un anhydride d'acide de formule R₁₂CO-O-COR₁₂, R₁₂ étant un groupe alkyle en C₁-C₆, phényl-alkyle(C₁-C₆) ou phényle, pour l'obtention d'un composé de formule I dans lequel R₃ est un groupe alcanoyl(C₁-C₆)-amino, phénylalcanoyl(C₁-C₆)-amino ou phénylcarbonylamino, le radical phényle pouvant être substitué comme indiqué dans la revendication 1,
q) éventuellement la mise en réaction d'un composé de formule I dans lequel m, n, p, R, R₁ et R₂ sont tels que définis dans la formule I donnée plus haut, et R₃ est un atome d'hydrogène, avec un alkyl(C₁-C₆)-lithium, pour l'obtention d'un composé de formule I dans lequel R₃ est un groupe alkyle en C₁-C₆,
r) éventuellement la mise en réaction d'un composé de formule I dans lequel m, n, p et R sont tels que définis dans la formule I donnée plus haut, R₂ est un groupe alkyle en C₁-C₆, phényl-alkyle(C₁-C₆) ou phényle, le radical phényle pouvant être substitué comme indiqué dans la revendication 1, R₃ est un atome d'hydrogène ou d'halogène, ou un groupe alkyle en C₁-C₆, et R₁ est le groupe -CH₂OH, avec une base alcaline forte, et la mise en réaction de l'ion alcoolate résultant avec un halogénure d'alkyle en C₁-C₆, un halogénure de phényl-alkyle(C₁-C₆) ou un halogénure de phényle de formule R₅X, le radical phényle pouvant être substitué comme indiqué dans la revendication 1, pour l'obtention d'un composé de formule I dans lequel R₁ est le groupe -CH₂OR₅,
s) éventuellement la soumission d'un composé de formule I dans lequel m, n, p et R sont tels que définis dans l'étape a), R₁ est -CHO, R₂ est un groupe alkyle en C₁-C₆, alcoxy(C₁-C₆)-carbonyle, alcanoyle, alcénoyle, alcynoyle, phényl-alcanoyle(C₁-C₆) ou phénylcarbonyle, le radical phényle pouvant être substitué comme indiqué dans la revendication 1, et R₃ est un atome d'hydrogène ou d'halogène, ou un groupe NO₂ ou alkyle en C₁-C₆, à une réaction de Wittig avec un ylure de formule (C₆H₅)₃P=CR₂₀R₂₁, R₂₀ et R₂₁ étant chacun indépendamment un atome d'hydrogène ou un groupe alkyle en C₁-C₆, phényle ou phényl-alkyle(C₁-C₆), le radical phényle pouvant être substitué comme indiqué dans la revendication 1, un groupe hétéroaryle ou hétéroaryl-alkyle(C₁-C₆), le terme "hétéroaryle" ayant la signification donnée dans la revendication 1, un groupe cyano, méthoxy, alcoxy(C₁-C₆)-carbonyle, ou forment ensemble un groupe cycloalkylidène en C₃-C₇, pour l'obtention d'un composé de formule I dans lequel R₁ est le groupe -CH=CR₂₀R₂₁,
t) éventuellement l'hydrogénation catalytique d'un composé de formule I, tel qu'obtenu dans l'étape s), pour l'obtention d'un composé de formule I dans lequel R₁ est un groupe alkyle en C₁-C₆, phényl-alkyle(C₁-C₆) dans lequel le radical phényle peut être substitué comme indiqué dans la revendication 1, un groupe hétéroaryl-alkyle(C₁-C₆), le terme "hétéroaryle" ayant la signification donnée dans la revendication 1, un groupe cyano-alkyle(C₁-C₆), méthoxy-alkyle(C₁-C₆), alcoxy(C₁-C₆)-carbonyl-alkyle(C₁-C₆) ou cycloalkyl(C₃-C₇)-alkyle(C₁-C₆),
u) éventuellement l'hydrogénation catalytique d'un composé de formule I dans lequel m, n, p, R, R₁ et R₃ sont tels que définis dans la formule I donnée plus haut, et R₂ est le groupe nitrophényle, pour l'obtention d'un composé de formule I dans lequel R₂ est le groupe aminophényle,
v) éventuellement la mise en réaction d'un composé de formule I, tel qu'obtenu dans l'étape u), avec du formiate de méthyle, pour l'obtention d'un composé de formule I dans lequel R₂ est le groupe formylaminophényle,
w) éventuellement la mise en réaction d'un composé de formule I, tel qu'obtenu dans l'étape u), avec un chlorure d'acyle de formule R₁₂COCl ou un anhydride d'acide de formule R₁₂-CO-O-COR₁₂, R₁₂ étant un groupe alkyle en C₁-C₆, pour l'obtention d'un composé de formule I dans lequel R₂ est un groupe alcanoyl(C₁-C₆)amino-phényle,
x) éventuellement la mise en réaction d'un composé de formule I, tel qu'obtenu dans l'étape j), dans lequel R₁ est -CHO, avec de l'hydroxylamine, pour l'obtention de l'oxime correspondante, et la mise en réaction de cette oxime avec du chlorure de benzènesulfonyle, pour l'obtention d'un composé de formule I dans lequel R₁ est le radical -CN,
y) éventuellement la mise en réaction d'un composé de formule I dans lequel R₂ est un groupe alcoxy(C₁-C₆)-carbonyl-alkyle(C₁-C₆), avec de l'ammoniac, pour l'obtention d'un composé de formule I dans lequel R₂ est un groupe amino-carbonyl-alkyle(C₁-C₆).

10. Composition pharmaceutique comprenant comme composant actif un composé tel que défini dans la revendication 1, et un véhicule pharmaceutiquement acceptable pour celui-ci.

11. Utilisation d'un composé tel que défini dans la revendication 1, pour la fabrication d'un médicament ayant des activités d'amélioration de la mémoire, de soulagement de la douleur et/ou d'atténuation de la dépression.

## Revendications (Revendications pour l'(les) Etat(s) contractant(s) suivant(s): ES, GR)

1. Procédé pour la préparation d'un composé de formule 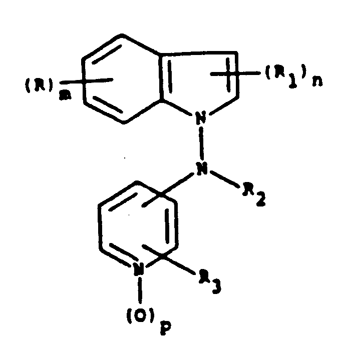 dans laquelle m est 1 ou 2; n est 1 ou 2; p est 0 ou 1; chaque radical R est indépendamment un atome d'hydrogène ou d'halogène, ou un groupe alkyle en C₁-C₆, alcoxy en C₁-C₆, hydroxy, nitro, amino, alkyl(C₁-C₆)-amino, alkyl(C₁-C₆)-carbonylamino, cyano, formyle, alcoxy(C₁-C₆)-carbonyle, alkyl(C₁-C₆)-thio ou alcoxy(C₁-C₆)-carbonyl-alkyl(C₁-C₆)-thio; chaque radical R₁ est indépendamment un atome d'hydrogène ou d'halogène, ou un groupe alkyle en C₁-C₆, alcényle en C₂-C₆, formyle, alkyl(C₁-C₆)-carbonyle, phényl-alkyl(C₁-C₆)-carbonyle, phénylcarbonyle, phényl-alcényle(C₂-C₆), phényl-alkyle(C₁-C₆), hétéroaryl-alcényle(C₂-C₆), hétéroaryl-alkyle(C₁-C₆), cyano-alcényle(C₂-C₆), cyano-alkyle(C₁-C₆), méthoxy-alcényle(C₂-C₆), méthoxy-alkyle(C₁-C₆), alcoxy(C₁-C₆)-carbonyl-alcényle(C₂-C₆), alcoxy(C₁-C₆)-carbonyl-alkyle(C₁-C₆), cycloalkyl(C₃-C₇)-alcényle(C₂-C₆) ou cycloalkyl(C₃-C₇)-alkyle(C₁-C₆), le terme "phényle" signifiant un groupe phényle qui peut comporter 1-3 substituants, chacun d'eux étant indépendamment un atome d'halogène ou un radical alkyle en C₁-C₆, alcoxy en C₁-C₆, CF₃, NO₂ ou CN, et le terme "hétéroaryle" signifiant un groupe de formule 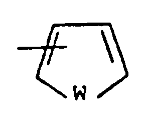 dans laquelle W est O, S, NH ou CH=N, cyano, -CH(OH)R₄, -C(OH)R₄R₅ ou -CH₂OR₅, R₄ étant un atome d'hydrogène ou un radical alkyle en C₁-C₆, phényl-alkyle(C₁-C₆) ou phényle, et R₅ étant un radical alkyle en C₁-C₆, phényl-alkyle(C₁-C₆) ou phényle; chaque radical R₂ est indépendamment un atome d'hydrogène ou un groupe alkyle en C₁-C₆, alcényle en C₂-C₆, alcynyle en C₂-C₆, alcoxy(C₁-C₆)-carbonyl-alkyle(C₁-C₆), alkyl(C₁-C₆)amino-carbonyl-alkyle(C₁-C₆), aminocarbonyl-alkyle(C₁-C₆), phényl-alkyle-(C₁-C₆), alcoxy(C₁-C₆)-carbonyle, phényl-alcoxy(C₁-C₆)-carbonyle, phényloxycarbonyle, alkyl(C₁-C₆)amino-carbonyle, phényl-alkyl(C₁-C₆)amino-carbonyle, phénylaminocarbonyle, alcanoyle, phényl-alcanoyle(C₁-C₆), phénylcarbonyle, alcénoyle, alcynoyle ou -R₆-NR'R'', R₆ étant un groupe alkylène en C₁-C₆, alcénylène en C₂-C₆ ou alcynylbne en C₂-C₆, et R' et R'' étant chacun indépendamment un groupe alkyle en C₁-C₆, ou bien le groupe -NR'R'' dans son ensemble étant un radical 1-pyrrolidinyle, 1-pipéridinyle, 4-morpholinyle, 4-alkyl(C₁-C₆)-1-pipérazinyle ou 4-phényl-1-pipérazinyle, le terme "phényle" signifiant un groupe phényle qui peut être substitué comme indiqué plus haut, ou chaque radical R₂ est indépendamment un groupe phényle, nitrophényle, cyanophényle, trifluorométhyle, aminophényle ou alcanoyl(C₁-C₆)aminophényle, le radical phényle n'étant pas substitué, et R₃ est un atome d'hydrogène ou d'halogène, ou un groupe nitro, amino, alcanoyl(C₁-C₆)-amino, phényl-alcanoyl(C₁-C₆)-amino, phénylcarbonylamino, alkylamino, phényl-alkyl(C₁-C₆)-amino ou alkyle en C₁-C₆, le terme "phényle" signifiant un groupe phényle qui peut être substitué comme indiqué plus haut; ou d'un sel d'addition avec un acide pharmaceutiquement acceptable de celui-ci, qui comprend
a) la mise en réaction d'un composé de formule II 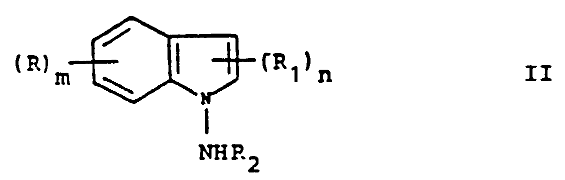 dans laquelle m est 1 ou 2 et n est 1 ou 2, R est un atome d'hydrogène ou d'halogène, ou un groupe alkyle en C₁-C₆, alcoxy en C₁-C₆, nitro, cyano, formyle, alkyl(C₁-C₆)-thio ou alcoxy(C₁-C₆)-carbonyl-alkyl(C₁-C₆)-thio, R₁ est un atome d'hydrogène ou d'halogène, ou un groupe alkyle en C₁-C₆ ou cyano, et R₂ est un atome d'hydrogène ou un groupe alkyle en C₁-C₆,
avec un composé de formule III 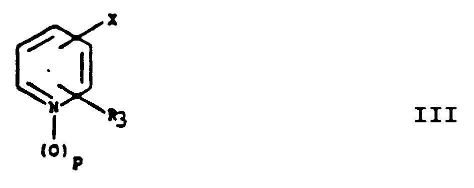 dans laquelle X est un atome de chlore ou de fluor, R₃ est un atome d'hydrogène ou d'halogène, ou un groupe nitro ou alkyle inférieur, et p est 0 ou 1,
pour l'obtention d'un composé de formule I dans lequel R, R₁, R₂ et R₃ sont tels que définis,
b) éventuellement le traitement par une base forte d'un composé de formule I, tel qu'obtenu dans l'étape a), dans lequel R₂ est un atome d'hydrogène, et la mise en réaction de l'anion obtenu avec un composé de formule R₂Cl, dans laquelle R₂ est un groupe alkyle en C₁-C₆, alcoxy(C₁-C₆)-carbonyl-alkyle(C₁-C₆), alcényle en C₂-C₆, alcynyle en C₂-C₆, alcoxy(C₁-C₆)-carbonyle, phényl-alkyle(C₁-C₆), phényl-alcoxy(C₁-C₆)-carbonyle ou phényloxycarbonyle, le radical phényle pouvant être substitué comme indiqué plus haut, pour l'obtention d'un composé de formule I dans lequel m, n, p, R, R₁, R₃ sont tels que définis dans l'étape a) et R₂ est tel que défini ci-dessus,
c) éventuellement le traitement par une base forte d'un composé de formule I tel qu'obtenu dans l'étape a), dans lequel R₂ est un atome d'hydrogène, et la mise en réaction de l'anion obtenu avec un composé de formule 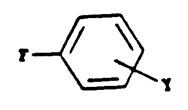 dans laquelle Y est le groupe nitro ou trifluorométhyle, pour l'obtention d'un composé de formule I dans lequel m, n, p, R, R₁ et R₃ sont tels que définis dans l'étape a) et R₂ est le groupe nitrophényle, cyanophényle ou trifluorométhyl-phényle,
d) éventuellement la mise en réaction d'un composé de formule I, tel qu'obtenu dans l'étape a), dans lequel R₂ est un atome d'hydrogène, avec un composé de formule R₁₂NCO, dans laquelle R₁₂ est un groupe alkyle en C₁-C₆, phénylalkyle(C₁-C₆) ou phényle, le radical phényle pouvant être substitué comme indiqué plus haut, pour l'obtention d'un composé de formule I dans lequel m, n, p, R, R₁ et R₃ sont tels que définis dans l'étape a), et R₂ est un groupe alkyl(C₁-C₆)-amino-carbonyle, phényl-alkyl(C₁-C₆)amino-carbonyle ou phénylaminocarbonyle, le radical phényle pouvant être substitué comme indiqué plus haut,
e) éventuellement la mise en réaction d'un composé de formule I, tel qu'obtenu dans l'étape a), dans lequel R₂ est un atome d'hydrogène, avec un composé de formule R₁₃COCl, dans laquelle R₁₃ est un groupe alkyle, phényl-alkyle(C₁-C₆), phényle, le radical phényle pouvant être substitué comme indiqué plus haut, alcényle ou alcynyle, pour l'obtention d'un composé de formule I dans lequel m, n, p, R, R₁ et R₃ sont tels que définis dans l'étape a), et R₂ est un groupe alcanoyle, phényl-alcanoyle(C₁-C₆), phénylcarbonyle, le radical phényle pouvant être substitué comme indiqué plus haut, alcénoyle ou alcynoyle,
f) éventuellement la mise en réaction avec une base forte d'un composé de formule I, tel qu'obtenu dans l'étape a), dans lequel R₂ est un atome d'hydrogène, et ensuite la mise en réaction du produit avec un sulfate de dialkyle en C₁-C₆, pour l'obtention d'un composé de formule I dans lequel m, n, p, R, R₁ et R₃ sont tels que définis dans l'étape a), et R₂ est un groupe alkyle en C₁-C₆,
g) éventuellement la soumission à une réaction de Mannich d'un composé de formule I, tel qu'obtenu dans l'étape b), dans lequel R₂ est -CH₂C≡CH, avec du formaldéhyde et une àmine secondaire de formule HNR'R'', dans laquelle R' et R'' sont indépendamment un groupe alkyle en C₁-C₆, ou R' et R'' forment ensemble un groupe 1-pyrrolidinyle, 1-pipéridinyle, 1-morpholinyle, 4-alkyl(C₁-C₆)-1-pipérazinyle ou 4-phényl-1-pipérazinyle, dans lequel le radical phényle peut être substitué comme indiqué plus haut, pour l'obtention d'un composé de formule I dans lequel m, n, p, R, R₁ et R₃ sont tels que définis dans l'étape b), et R₂ est le groupe -CH₂C≡CCH₂NR'R'', dans lequel R' et R'' sont tels que définis ci-dessus,
h) éventuellement l'hydrogénation d'un composé de formule I, tel qu'obtenu dans l'étape g), pour l'obtention d'un composé de formule I dans lequel m, n, p, R, R₁ et R₃ sont tels que définis dans l'étape a), et R₂ est le groupe -CH₂CH=CHCH₂NR'R'' ou -(CH₂)₄NR'R'', R' et R'' étant tels que définis dans l'étape g).
i) éventuellement la mise en réaction d'un composé de formule I, dans lequel m, n, p, R et R₃ sont tels que définis dans l'étape a), R₁ est un atome d'hydrogène et R₂ est un groupe alkyle en C₁-C₆, alcoxy(C₁-C₆)-carbonyl-alkyle(C₁-C₆), alcanoyle, alcoxy(C₁-C₆)-carbonyle, phényl-alkyle(C₁-C₆), phényle, phényl-alcoxy(C₁-C₆)-carbonyle, phényloxycarbonyle, phényl-alcanoyle(C₁-C₆) ou phénylcarbonyle, le radical phényle pouvant être substitué comme indiqué plus haut, avec un agent de chloration approprié, pour l'obtention du dérivé 3-chloro d'un composé de for-mule I dans lequel m, n, p, R, R₁ et R₃ sont tels que définis plus haut,
j) éventuellement la mise en réaction d'un composé de formule I dans lequel m, n, p et R sont tels que définis dans l'étape a), R₁ est un atome d'hydrogène, R₂ est un groupe alkyle en C₁-C₆, alcoxy(C₁-C₆)-carbonyle, alcanoyle, alcénoyle, alcynoyle, phényl-alcanoyle(C₁-C₆) ou phénylcarbonyle, le radical phényle pouvant être substitué comme indiqué plus haut, et R₃ est un atome d'hydrogbne ou d'halogène, ou un groupe nitro ou alkyle en C₁-C₆, soit avec de l'oxychlorure de phosphore et du diméthylformamide, pour l'obtention d'un composé de formule I dans lequel R est le groupe formyle en position 3, soit avec du sec-butyl-lithium et la mise en réaction du composé lithié résultant avec de la N-formyl-N-méthyl-aniline, pour l'obtention d'un composé de formule I dans lequel R₁ est le groupe formyle en position 2,
k) éventuellement la mise en réaction d'un composé de formule I dans lequel m, n, p et R sont tels que définis dans l'étape a), R₁ est un atome d'hydrogène, R₂ est un groupe alkyle en C₁-C₆, alcoxy(C₁-C₆)-carbonyle, alcanoyle, alcénoyle, alcynoyle, phényl-alcanoyle(C₁-C₆) ou phénylcarbonyle, le radical phényle pouvant être substitué comme indiqué plus haut, et R₃ est un atome d'hydrogène ou d'halogène, ou un groupe nitro ou alkyle en C₁-C₆, soit avec un chlorure d'alcanoyle en C₁-C₆, de phényl-alcanoyle en C₁-C₆ ou de phénylcarbonyle de formule R₁₂COCl, le radical phényle pouvant être substitué comme indiqué plus haut, en présence de chlorure de zinc, pour l'obtention d'un composé de formule I dans lequel R₁ est un groupe alcanoyle en C₁-C₆, phényl-alcanoyle(C₁-C₆) ou phénylcarbonyle, le radical phényle pouvant être substitué comme indiqué plus haut, soit avec du sec-butyl-lithium et la mise en réaction du composé lithié résultant avec R₁₂COCl, pour l'obtention d'un composé de formule I dans lequel le substituant R₁ est en position 2,
l) éventuellement la réduction d'un composé de formule I dans lequel m, n, p et R sont tels que définis dans l'étape a), R₁ est un groupe alcanoyle en C₁-C₆, phényl-alcanoyle(C₁-C₆) ou phénylcarbonyle, R₂ est un groupe alkyle en C₁-C₆, phényl-alkyle(C₁-C₆) ou phényle, le radical phényle pouvant être substitué comme indiqué dans plus haut, et R₃ est un atome d'hydrogène ou d'halogène, ou un groupe alkyle en C₁-C₆, soit avec NaBH₄, LiAlH₄ ou des complexes de borane, pour l'obtention d'un composé de formule I dans lequel R₁ est le groupe -CH(OH)R₄ dans lequel R₄ est un atome d'hydrogène ou un groupe alkyle en C₁-C₆, phényl-alkyle(C₁-C₆) ou phényle, le radical phényle pouvant être substitué comme indiqué plus haut, soit avec un réactif de Grignard de formule R₅MgBr, pour l'obtention d'un composé de formule I dans lequel R₁ est un groupe 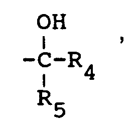 dans lequel R₅ est alkyle en C₁-C₆, phényl-alkyle(C₁-C₆) ou phényle, le radical phényle pouvant être substitué comme indiqué plus haut,
m) éventuellement l'hydrogénation d'un composé de formule I dans lequel m, n, p, R, R₁ et R₂ sont tels que définis dans la formule I ci-dessus, et R₃ est le groupe nitro, en présence d'un catalyseur, pour l'obtention d'un composé de formule I dans lequel R₃ est le groupe amino,
n) éventuellement l'hydrolyse d'un composé de formule I dans lequel m, n, p, R, R₁ sont tels que définis dans la formule I ci-dessus, R₃ est tel que défini dans l'étape a) et R₂ est un groupe alcoxy(C₁-C₆)-carbonyle, pour l'obtention d'un composé de formule I dans lequel R₂ est un atome d'hydrogène,
o) éventuellement la mise en réaction d'un composé de formule I dans lequel m, n, p, R, R₁ et R₂ sont tels que définis dans la formule I donnée plus haut, et R₃ est le groupe amino, avec du formiate de phényle, pour l'obtention d'un composé de formule I dans lequel R₃ est le groupe formylamino.
p) éventuellement la mise en réaction d'un composé de formule I, dans lequel m, n, p, R, R₁ et R₂ sont tels que définis dans la formule I donnée plus haut, et R₃ est le groupe amino, avec un chlorure d'acyle de formule R₁₂COCl ou un anhydride d'acide de formule R₁₂CO-O-COR₁₂, R₁₂ étant un groupe alkyle en C₁-C₆, phényl-alkyle(C₁-C₆) ou phényle, pour l'obtention d'un composé de formule I dans lequel R₃ est un groupe alcanoyl(C₁-C₆)-amino, phénylalcanoyl(C₁-C₆)-amino ou phénylcarbonylamino, le radical phényle pouvant être substitué comme indiqué plus haut,
q) éventuellement la mise en réaction d'un composé de formule I dans lequel m, n, p, R, R₁ et R₂ sont tels que définis dans la formule I donnée plus haut, et R₃ est un atome d'hydrogène, avec un alkyl(C₁-C₆)-lithium, pour l'obtention d'un composé de formule I dans lequel R₃ est un groupe alkyle en C₁-C₆,
r) éventuellement la mise en réaction d'un composé de formule I dans lequel m, n, p et R sont tels que définis dans la formule I donnée plus haut, R₂ est un groupe alkyle en C₁-C₆, phényl-alkyle(C₁-C₆) ou phényle, le radical phényle pouvant être substitué comme indiqué plus haut, R₃ est un atome d'hydrogène ou d'halogène, ou un groupe alkyle en C₁-C₆, et R₁ est le groupe -CH₂OH, avec une base alcaline forte, et la mise en réaction de l'ion alcoolate résultant avec un halogénure d'alkyle en C₁-C₆, un halogénure de phényl-alkyle(C₁-C₆) ou un halogénure de phényle de formule R₅X, le radical phényle pouvant être substitué comme indiqué plus haut, pour l'obtention d'un composé de formule I dans lequel R₁ est le groupe -CH₂OR₅,
s) éventuellement la soumission d'un composé de formule I dans lequel m, n, p et R sont tels que définis dans l'étape a), R₁ est -CHO, R₂ est un groupe alkyle en C₁-C₆, alcoxy(C₁-C₆)-carbonyle, alcanoyle, alcénoyle, alcynoyle, phényl-alcanoyle(C₁-C₆) ou phénylcarbonyle, le radical phényle pouvant être substitué comme indiqué plus haut, et R₃ est un atome d'hydrogène ou d'halogène, ou un groupe NO₂ ou alkyle en C₁-C₆, à une réaction de Wittig avec un ylure de formule (C₆H₅)₃P=CR₂₀R₂₁, R₂₀ et R₂₁ étant chacun indépendamment un atome d'hydrogène ou un groupe alkyle en C₁-C₆, phényle ou phényl-alkyle(C₁-C₆), le radical phényle pouvant être substitué comme indiqué plus haut, un groupe hétéroaryle ou hétéroaryl-alkyle(C₁-C₆), le terme "hétéroaryle" ayant la signification donnée plus haut, un groupe cyano, méthoxy, alcoxy(C₁-C₆)-carbonyle, ou forment ensemble un groupe cycloalkylidène en C₃-C₇, pour l'obtention d'un composé de formule I dans lequel R₁ est le groupe -CH=CR₂₀R₂₁,
t) éventuellement l'hydrogénation catalytique d'un composé de formule I, tel qu'obtenu dans l'étape s), pour l'obtention d'un composé de formule I dans lequel R₁ est un groupe alkyle en C₁-C₆, phényl-alkyle(C₁-C₆) dans lequel le radical phényle peut être substitué comme indiqué plus haut, un groupe hétéroaryl-alkyle(C₁-C₆), le terme "hétéroaryle" ayant la signification donnée plus haut, un groupe cyano-alkyle(C₁-C₆), méthoxy-alkyle(C₁-C₆), alcoxy(C₁-C₆)-carbonyl-alkyle(C₁-C₆) ou cycloalkyl(C₃-C₇)-alkyle(C₁-C₆),
u) éventuellement l'hydrogénation catalytique d'un composé de formule I dans lequel m, n, p, R, R₁ et R₃ sont tels que définis dans la formule I donnée plus haut, et R₂ est le groupe nitrophényle, pour l'obtention d'un composé de formule I dans lequel R₂ est le groupe aminophényle,
v) éventuellement la mise en réaction d'un composé de formule I, tel qu'obtenu dans l'étape u), avec du formiate de méthyle, pour l'obtention d'un composé de formule I dans lequel R₂ est le groupe formylaminophényle,
w) éventuellement la mise en réaction d'un composé de formule I, tel qu'obtenu dans l'étape u), avec un chlorure d'acyle de formule R₁₂COCl ou un anhydride d'acide de formule R₁₂-CO-O-COR₁₂, R₁₂ étant un groupe alkyle en C₁-C₆, pour l'obtention d'un composé de formule I dans lequel R₂ est un groupe alcanoyl(C₁-C₆)amino-phényle,
x) éventuellement la mise en réaction d'un composé de formule I, tel qu'obtenu dans l'étape j), dans lequel R₁ est -CHO, avec de l'hydroxylamine, pour l'obtention de l'oxime correspondante, et la mise en réaction de cette oxime avec du chlorure de benzènesulfonyle, pour l'obtention d'un composé de formule I dans lequel R₁ est le radical -CN,
y) éventuellement la mise en réaction d'un composé de formule I dans lequel R₂ est un groupe alcoxy(C₁-C₆)-carbonyl-alkyle(C₁-C₆), avec de l'ammoniac, pour l'obtention d'un composé de formule I dans lequel R₂ est un groupe amino-carbonyl-alkyle(C₁-C₆).

2. Procédé selon la revendication 2, dans lequel m est 1, n est 1 et p est zéro.

3. Procédé selon la revendication 2, dans lequel R est un atome d'hydrogène ou un groupe alkyle en C₁-C₆ ou alcoxy en C₁-C₆, R₁ est un atome d'hydrogène ou un groupe alkyle en C₁-C₆, alcényle en C₂-C₆ ou formyle, R₂ est un atome d'hydrogène ou un groupe alkyle en C₁-C₆ ou phényl-alkyle(C₁-C₆), le terme "phényle" signifiant un groupe phényle qui peut être substitué comme indiqué plus haut, et R₃ est un atome d'hydrogène ou d'halogène ou le groupe nitro ou amino.

4. Procédé selon la revendication 1, dans lequel on prépare le composé N-méthyl-N-(4-pyridinyl)-1H-indol-1-amine ou un sel pharmaceutiquement acceptable de celui-ci.

5. Procédé selon la revendication 1, dans lequel on prépare le composé N-(4-pyridinyl)-1H-indol-1-amine ou un sel pharmaceutiquement acceptable de celui-ci.

6. Procédé selon la revendication 1, dans lequel on prépare le composé N-propyl-N-(4-pyridinyl)-1H-indol-1-amine ou un sel pharmaceutiquement acceptable de celui-ci.

7. Procédé selon la revendication 1, dans lequel on prépare le composé 3-éthyl-N-méthyl-N-(4-pyridinyl)-1H-indol-1-amine ou un sel pharmaceutiquement acceptable de celui-ci.

8. Procédé selon la revendication 1, dans lequel on prépare le composé 3-éthényl-N-méthyl-N-(4-pyridinyl)-1H-indol-1-amine ou un sel pharmaceutiquement acceptable de celui-ci.

9. Utilisation d'un composé tel que défini dans la revendication 1, pour la fabrication d'un médicament ayant des activités d'amélioration de la mémoire, de soulagement de la douleur et/ou d'atténuation de la dépression.
